# EUROPEAN PATENT APPLICATION

(11) **EP 1 772 454 A1**
(43) Date of publication of application: **11.04.2007**
(21) Application number: 05766456.7
(22) Date of filing: 22.07.2005
(51) Int. Cl.: C07D 471/04, C07D 487/04, A61K 31/437, A61K 31/519, A61P 1/00, A61P 1/10, A61P 1/12, A61P 3/14, A61P 11/00, A61P 25/16, A61P 25/24, A61P 25/28, A61P 43/00

(54) **NITROGENOUS FUSED BICYCLIC COMPOUND**

(30) Priority: 23.07.2004 JP 2004216500; 23.07.2004 JP 2004216501
(71) Applicant: TANABE SEIYAKU CO., LTD., Chuo-ku, Osaka-shi, Osaka 541-8505 (JP)
(72) Inventor: TAKAMURO, Iwao TANABE SEIYAKU CO., LTD., Osaka 5418505 (JP); KAWANAMI, Saburo TANABE SEIYAKU CO., LTD., Osaka 5418505 (JP); TSUBOI, Yasunori TANABE SEIYAKU CO., LTD., Osaka54185 05 (JP); HIMIYAMA, Toshiyuki TANABE SEIYAKU CO., LTD., Osaka 5418505 (JP); HASEGAWA, Yuko, Lawrenceville, NJ 08648-1268 (US); MOCHIDA, Hideki TANABE SEIYAKU CO., LTD., Osaka5418505 (JP); NOGI, Kouji TANABE SEIYAKU CO., LTD., Osaka 5418505 (JP)
(74) Representative: HOFFMANN EITLE
(86) International application number: PCT/JP2005/013459
(87) International publication number: WO 2006/009245

(57) **Abstract**

A novel nitrogenous fused bicyclic compound represented by the following general formula [1] or a pharmacologically acceptable salt of the compound. They have excellent SK channel blocking activity and are useful as a medicine. [I] (In the formula, R⁰ represents hydrogen, halogeno, etc.; R¹ represents a group represented by the formula (a) or (b); A represents a group represented by the formula (X) or (Y); D¹, D² and D³ each represents N or CH; R² represents halogeno or optionally halogenated lower alkyl, etc.; R³ represents hydrogen or lower alkyl; and Q represents lower alkylene.)

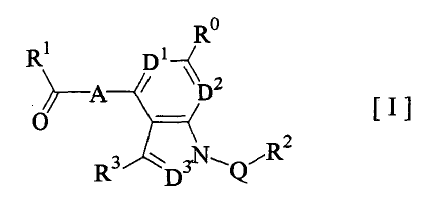

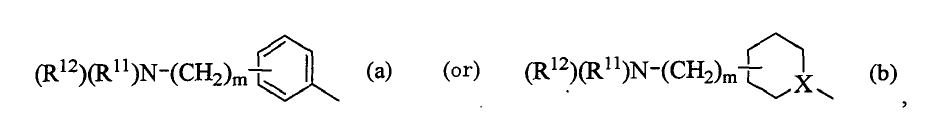

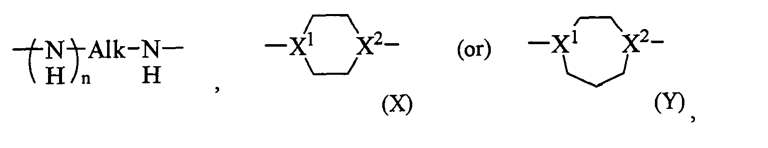

## Description

### TECHNICAL FIELD

This invention relates to a novel nitrogen-containing hetero-bicyclic compound, which has an excellent small-conductance potassium channel (SK) blocking activity and is useful as a medicament.

### BACKGROUND ART

Ca²⁺-activated potassium (K) channels consist of at least three subtypes: Big- (BK), Intermediate- (IK) and Small-conductance K channel. These channels are activated by increase in intracellular Ca²⁺ level. Although BK and IK channels are sensitive to changes in membrane voltage and increase in intracellular Ca²⁺ level, SK channels are characterized in that they are not significantly sensitive to the change in membrane voltage. Besides, SK channels are characterized in that they have a low conductance of 6 to 20 pS to single channel and a higher sensitivity to apamin.

SK channels are present in various cells such as liver cells or blood cells as well as in excitable cells such as nerve cells and muscle cells, and they may be responsible for cell functions including chemokine release, muscle contraction and secretion.

Apamin is a well-known selective SK channel blocker, and it has been reported that this agent activates gastrointestinal peristaltic function (S. A. Waterman and M. Costa, J. Physiology 477, 459-468, 1994*;* N. Spencer et al., J Physiology 517, 889-898, 1999), improves cognitive/acquisition deficits (S. Ikonen et al., Eur J. Pharmacol. 347, 13-21, 1998*;* C. Ghelardini et al., Br. J. Pharmacol. 123, 1079-1084, 1998) and decreases immobility time in mouse forced swimming test (N. Galeotti et al., Br. J. Pharmacol. 126, 1653-1659, 1999). Moreover, it has been reported that a specific receptor for apamin exists in skeletal muscle cells and administration of this agent alleviates the symptoms in patients with myotonic muscle dystrophy (J. F. Renaud et al., Nature 319, 678-680, 1986*;* M I. Behrens et al., Muscle & Nerve 17, 1264-1270, 1994)*.* Furthermore, it has been reported that mice conditionally over-expressing one of the SK subtype receptors, SK3, showed abnormal respiratory responses to hypoxia under a low partial pressure of oxygen *(*C. T. Bond et al., Science 289, 1942-1946; 2000*).*

As a compound showing a SK channel-blocking activity, bis(benzimidazol) compounds such as 1,1'-(α,α'-p-xylene)-3,3'-(α, α'-m-xylene)-bis(benzimidazolium) (WO00/01676), cyclophan compounds such as 7,18-diaza-3,4(1,4)-dibenzena-1,6(1,4)-diquinolina-cyclooctadecaphan 3 trifluoroacetate hydrate (WO97/48705), cross-linked bisquinoline compounds such as 1,4-bis-(2-methyl-quinolin-4-yl)-[1,4]-diazepane (US Patent No.5,866,562), compounds having a cyclohexane-1,1'(2'H)-spiroisoquinoline moiety (WO02/79189) and a bis-benzimidazole compound such as 2-[4-(5-amino-1H-benzimidazol-2-yl)-pyridin-3-yl]-benzimidazol-5-ylamine (WO03/094861) have been known.

### DISCLOSURE OF INVENTION

### PROBLEM TO BE SOLVED BY INVENTION

The object of the present invention is to provide a novel nitrogen-containing hetero-bicyclic compound useful as a medicament having an excellent SK channel blocking activity.

### MEASURES TO SOLVE PROBLEM

The present invention relates to a nitrogen-containing hetero-bicyclic compound of the following formula [I]: 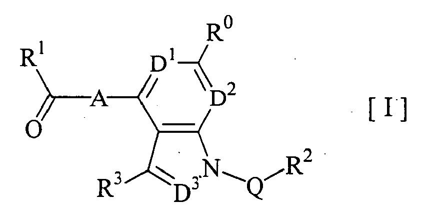 wherein R⁰ is a hydrogen atom, a halogen atom, a cyano group, a lower alkoxy group, a lower alkyl group, an amino group (said amino group being optionally substituted by a lower alkyl group(s)) or a heteroaryl group, R¹ is a group of the following formula: 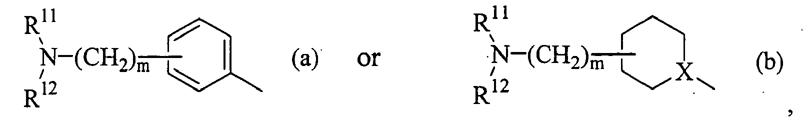 R¹¹ and R¹² are the same or different and a hydrogen atom, a lower alkyl group, a hydroxy-lower alkyl group, a cyclo-lower alkyl group, a lower alkanoyl group, a lower alkenoyl group, a lower alkoxy-lower alkyl group, a cyclo-lower alkyl-carbonyl group, an amino-lower alkyl group (said amino moiety being optionally substituted by a lower alkyl group(s)), a lower alkoxy-carbonyl group, a nitrogen-containing heterocyclic group-substituted lower alkyl group or a nitrogen-containing heterocyclic group optionally substituted by a lower alkyl group, or both groups combine each other at their termini together with an adjacent nitrogen atom to form a nitrogen-containing heterocyclic group (said heterocyclic group being optionally substituted by a lower alkyl group), X is N or CH, m is an integer of 0 or 1, A is a group of the following formula: 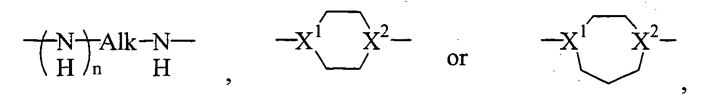 Alk is a lower alkylene group, one of X¹ and X² is N or CH and another is N, n is an integer of 0 or 1,
D¹, D² and D³ are N or CH,
R² is an aryl (or heteroaryl) group optionally substituted by one or two groups selected from a halogen atom, a lower alkyl group optionally substituted by a halogen atom, a lower alkoxy group optionally substituted by a halogen atom, a lower alkanoyl group and an amino group optionally substituted by one or two lower alkyl groups,
R³ is a hydrogen atom or a lower alkyl group and
Q is a lower alkylene group
or a pharmaceutically acceptable salt thereof.

### EFFECT OF INVENTION

A compound [I] of the present invention or a pharmaceutically acceptable salt thereof shows a significant antagonizing activity against apamin, which is known as a selective SK channel blocker, in a competitive binding assay. Therefore, the compound [I] or a pharmaceutically acceptable salt thereof is useful as a SK channel blocker which is applicable to treatment and/or prophylaxis of SK channel-related diseases such as gastrointestinal motility disorders (e.g., constipation, irritable bowel syndrome, , post-operative ileus, gastoroesophageal reflux disease), central nervous system disorders (e.g., memory and learning disorders, emotional disorder, Arzheimer's disease, depression, Perkinson's disease), myotonic muscular dystrophy or sleep apnea.

### BEST MODE TO CARRY OUT INVENTION

Concrete examples of the compound [I] of the present invention include a compound of the following formula [IA]: 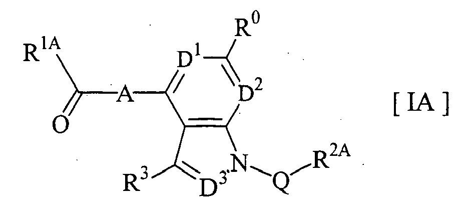 wherein R⁰ is a hydrogen atom, a halogen atom, a cyano group, a lower alkoxy group, a lower alkyl group, an amino group optionally substituted by a lower alkyl group(s) or a heteroaryl group, R^{1A} is a group of the following formula: 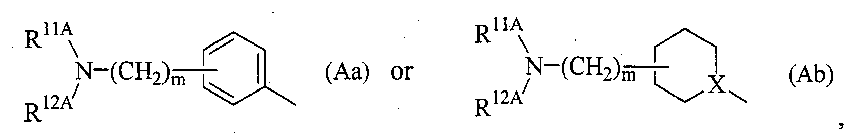 R^{11A} and R^{12A} are the same or different and a hydrogen atom, a lower alkyl group, a hydroxy-lower alkyl group, a cyclo-lower alkyl group, a lower alkanoyl group, a lower alkenoyl group, a lower alkoxy-lower alkyl group, a cyclo-lower alkyl-carbonyl group, an amino-lower alkyl group (said amino moiety being optionally substituted by a lower alkyl group(s)), a lower alkoxy-carbonyl group or a nitrogen-containing heterocyclic group-substituted lower alkyl group or both groups combine each other at their termini together with an adjacent nitrogen atom to form a nitrogen-containing heterocyclic group (said heterocyclic group being optionally substituted by a lower alkyl group), X is N or CH, m is an integer of 0 or 1, A is a group of the following formula: 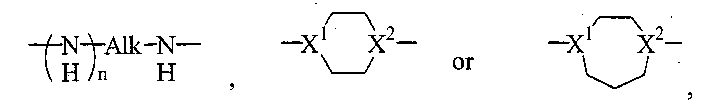 Alk is a lower alkylene group, one of X¹ and X² is N or CH and another is N, n is an integer of 0 or 1,
D¹, D² and D³ are N or CH,
R^{2A} is an aryl group optionally substituted by a lower alkoxy group or a heteroaryl group optionally substituted by a group(s) selected from a lower alkyl group and a lower alkoxy group,
R³ is a hydrogen atom or a lower alkyl group,
Q is a lower alkylene group;
provided that both R⁰ and R³ are not simultaneously a hydrogen atom when all of D¹, D²
and D³ are N and A is a group of the following formula: 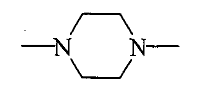 or a pharmaceutically acceptable salt thereof.

More concretely, examples of the above-mentioned compound [IA] include:
(1) a compound in which R^{1A} is a group of the formula (Aa) and one of R^{11A} and R^{12A} is a hydrogen atom or an amino-lower alkyl group (said amino moiety being optionally substituted by a lower alkyl group(s)) and another is a lower alkyl group, a lower alkanoyl group, a cyclo-lower alkyl-carbonyl group or a lower alkenoyl group; and
(2) a compound in which R^{1A} is a group of the formula (Ab) and one of R^{11A} and R^{12A} is a hydrogen atom, a lower alkyl group, an amino-lower alkyl group (said amino moiety may be substituted by a lower alkyl group(s)) or a nitrogen-containing heterocyclic group-substituted lower alkyl group and another is a lower alkyl group, a cyclo-lower alkyl group, a hydroxy-lower alkyl group, a lower alkoxy-lower alkyl group, a lower alkanoyl group or a cyclo-lower alkyl-carbonyl group or both of them combine each other at their termini together with an adjacent nitrogen atom to form a nitrogen-containing heterocyclic group (said heterocyclic group being optionally substituted by a lower alkyl group(s)).

In the above compound [IA] of the present invention, examples of the heterocyclic group in R^{11A} and R^{12A} may be a saturated or unsaturated 5- to 8-membered nitrogen-containing heteromonocyclic group (said heterocyclic group may further contain an oxygen atom(s) as a heteroatom(s) other than the nitrogen atom). Such heteromonocyclic group may be a pyrrolidinyl group, a piperidyl group, a piperazinyl group, a morpholinyl group, a thiomorpholinyl group, an azepinyl group or an azeocinyl group.

Examples of the aryl group in R^{2A} include a phenyl group and examples of the heteroaryl group include a 5- or 6-membered heteroaryl group containing one to three heteroatom(s) selected from a sulfur atom, an oxygen atom and a nitrogen atom. Such heteroaryl group may be a thienyl group, a pyridyl group or a thiazolyl group.

Among the above-mentioned compound [IA] of the present invention, preferred examples include a compound in which R^{2A} is a lower alkoxy-phenyl group such as an ethoxyphenyl group, a lower alkyl-pyridyl group such as a n-propylpyridyl group, a lower alkoxy-pyridyl group such as an ethoxypyridyl group or a lower alkyl-thiazolyl group such as a n-propylthiazolyl group.

Among the above-mentioned compound [IA], examples of the more preferred compound may be a compound in which Q is a methylene group.

Among the above-mentioned compound [IA], examples of the particularly preferred compound include:
(1) a compound of the following formula [IA-a]: 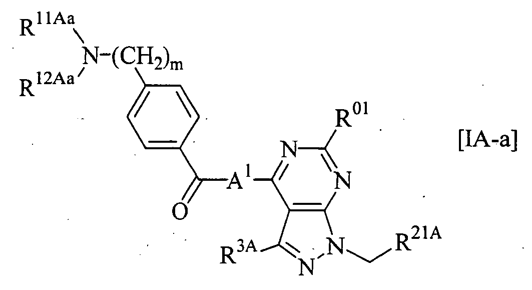 wherein R⁰¹ is a hydrogen atom, a halogen atom, a cyano group, a lower alkyl group, a lower alkoxy group, an amino group (said amino group being optionally substituted by a lower alkyl group(s)) or a thienyl group, one of R^{11Aa} and R^{12Aa} is a hydrogen atom or an amino-lower alkyl group (said amino moiety being optionally substituted by a lower alkyl group(s)) and another is a lower alkyl group, a lower alkanoyl group, a lower alkenoyl group or a cyclo-lower alkyl-carbonyl group, R^{21A} is a lower alkoxy-phenyl group, a lower alkyl-pyridyl group, a lower alkoxy-pyridyl group or a lower alkyl-thiazolyl group, R^{3A} is a hydrogen atom or a lower alkyl group, A¹ is a group of the following formula: 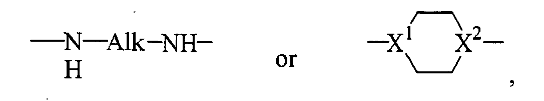 an the other symbols are the same as defined above,
   provided that both R⁰¹ and R^{3A} are not simultaneously a hydrogen atom when A¹ is a group of the following formula: 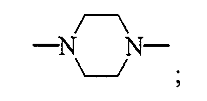
(2) a compound of the following formula [IA-b]: 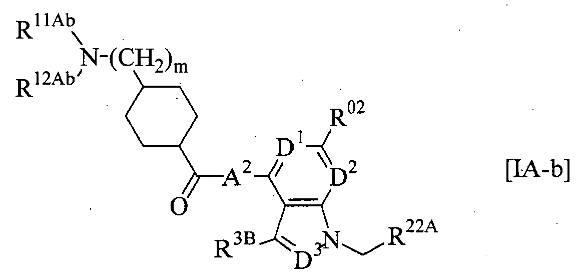 wherein R⁰² is a hydrogen atom, a halogen atom, a cyano group, a lower alkyl group, a lower alkoxy group, an amino group (said amino group being optionally substituted by a lower alkyl group(s)) or a thienyl group, one of R^{11Ab} and R^{12Ab} is a lower alkyl group and another is a lower alkoxy-lower alkyl group or both of them combine each other at their termini together with an adjacent nitrogen atom to form a saturated or unsaturated 5- to 8-membered hetero-monocyclic group (said hetero-monocyclic group being optionally substituted by a lower alkyl group(s) and may further contain an oxygen atom(s) as a heteroatom(s)), R^{22A} is a lower alkoxy-phenyl group, a lower alkoxy-pyridyl group or a lower alkyl-thiazolyl group, R^{3B} is a hydrogen atom or a lower alkyl group, A² is a group of the following formula: 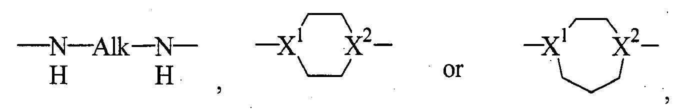 and the other symbols are the same as defined above,
   provided that both R⁰² and R^{3B} are not simultaneously a hydrogen atom when all of D¹, D² and D³ are N and A² is a group of the following formula: 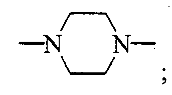 and
(3) a compound of the following formula [IA-c]: 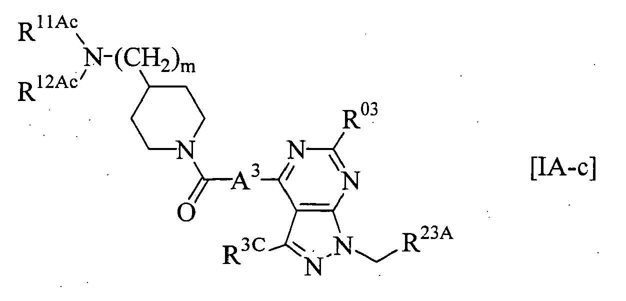
wherein R⁰³ is a hydrogen atom, a halogen atom, a cyano group, a lower alkyl group, a lower alkoxy group, an amino group (said amino group being optionally substituted by a lower alkyl group(s)) or a thienyl group, one of R^{11Ac} and R^{12Ac} is a hydrogen atom, a lower alkyl group, an amino-lower alkyl group (said amino moiety being optionally substituted by a lower alkyl group(s)) or a nitrogen-containing heterocyclic group-substituted lower alkyl group and another is a lower alkyl group, a cyclo-lower alkyl group, a hydroxy-lower alkyl group, a lower alkoxy-lower alkyl group, a lower alkanoyl group or a cyclo-lower alky-carbonyl group or both of them combine each other at their termini together with an adjacent nitrogen atom to form a saturated or unsaturated 5- to 8-membered heteromonocyclic group (said heteromonocyclic group may be substituted by a lower alkyl group(s) and may further contain an oxygen atom(s) as a heteroatom(s) other than the nitrogen atom), R^{23A} is a lower alkoxy-phenyl group, a lower alkyl-pyridyl group, a lower alkoxy-pyridyl group or a lower alkyl-thiazolyl group, R^{3C} is a hydrogen atom or a lower alkyl group, A³ is a group of the following formula: 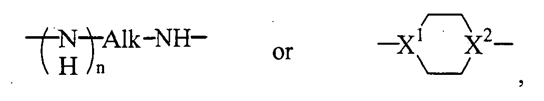 and the other symbols are the same as defined above,
provided that both R⁰³ and R^{3C} are not simultaneously a hydrogen atom when A³ is a group of the following formula: 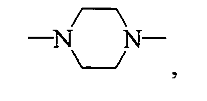 or a pharmaceutically acceptable salt thereof.

Among the compound [IA-c], examples of the further preferred compound may be a compound in which one of R^{11Ac} and R^{12Ac} is a lower alkyl group and another is a lower alkyl group, a lower alkoxy-lower alkyl group or a hydroxy-lower alkyl group.

Concrete examples of the particularly preferred compound [I] mentioned above include:
7-(3-ethoxybenzyl)-4-[4-[[trans-4-[[N-(2-methoxyethyl)-N-(tert-butyl)amino]methyl]cyclohexyl]carbonyl]piperazin-1-yl]-7H-pyrrolo[2,3-d]pyrimidine;
1-(3-ethoxybenzyl)-4-[4-[[trans-4-[[N-(2-methoxyethyl)-N-(tert-butyl)amino]methyl]cyclohexyl]carbonyl]piperazin-1-yl]-1H-pyrazolo[3,4-b]pyridine;
6-chloro-4-[4-[(trans-4-piperidin-1-ylcyclohexyl)carbonyl]piperazin-1-yl]-1-[(2-propyl-1,3-thiazol-4-yl)methyl]-1H-pyrazolo[3,4-d]pyrimidine;
1-(3-ethoxybenzyl)-4-[1-[(trans-4-piperidin-1-ylcyclohexyl)carbonyl]piperidin-4-yl]-1H-pyrazolo[3,4-d]pyrimidine;
6-ethoxy-1-[(6-ethoxypyridin-2-yl)methyl]-4-[4-[(trans-4-piperidin-1-ylcyclohexyl)carbonyl]piperazin-1-yl]-1H-pyrazolo[3,4-d]pyrimidine;
1-(3-ethoxybenzyl)-4-[4-[[trans-4-[[N-(2-methoxyethyl)-N-(tert-butyl)amino]methyl]cyclohexyl]carbonyl]piperazin-1-yl]-1H-pyrazolo[4,3-c]pyridine;
6-chloro-1-[(2-propyl-1,3-thiazol-4-yl)methyl]-4-[4-[(trans-4-[[N-(2-methoxyethyl)-N-(tert-butyl)amino]methyl]cyclohexyl]carbonyl]piperazin-1-yl]-1H-pyrazolo[3,4-d]pyrimidine;
6-ethoxy-1-[(6-ethoxypyridin-2-yl)methyl]-4-[4-[[trans-4-[[N-(2-methoxyethyl)-N-(tert-butyl)amino]methyl]cyclohexyl]carbonyl]piperazin-1-yl]-1H-pyrazolo[3,4-d]pyrimidine;
1-(3-ethoxybenzyl)-4-[4-[[4-[[N-ethyl-N-(tert-butyl)amino]methyl]piperidin-1-yl]carbonyl]piperidin-1-yl]-1H-pyrazolo[3,4-d]pyrimidine;
1-[(6-propylpyridin-2-yl)methyl]-4-[4-[[4-[[N-(2-methoxyethyl)-N-(tert-butyl)amino]methyl]piperidin-1-yl]carbonyl]piperidin-1-yl]-1H-pyrazolo[3,4-d]pyrimidine;
1-[(6-propylpyridin-2-yl)methyl]-4-[4-([4-[[N-ethyl-N-(tert-butyl)amino]methyl]piperidin-1-yl]carbonyl]piperidin-1-yl]-1H-pyrazolo[3,4-d]pyrimidine;
1-[(6-ethoxypyridin-2-yl)methyl]-4-[4-[[4-[[N-(2-methoxyethyl)-N-(tert-butyl)amino]methyl]piperidin-1-yl]carbonyl]piperidin-1-yl]-1H-pyrazolo[3,4-d]pyrimidine;
1-[(6-ethoxypyridin-2-yl)methyl]-4-[4-[[4-[[N,N-(diisopropyl)amino]methyl]piperidin-1-yl]carbonyl]piperidin-1-yl]-1H-pyrazolo[3,4-d]pyrimidine;
1-[(6-ethoxypyridin-2-yl)methyl]-4-[4-[[4-[[N-(2-hydroxyethyl)-N-(tert-butyl)amino]methyl]piperidin-1-yl]carbonyl]piperidin-1-yl]-1H-pyrazolo[3,4-d]pyrimidine;
1-[(6-ethoxypyridin-2-yl)methyl]-4-[4-[[4-[[N-(2-hydroxyethyl)-N-isopropylamino]methyl]piperidin-1-yl]carbonyl]piperidin-1-yl]-1H-pyrazolo[3,4-d]pyrimidine;
1-[(2-propyl-1,3-thiazol-4-yl)methyl]-4-[4-[[4-[[N-(2-methoxyethyl)-N-(tert-butyl)amino]methyl]piperidin-1-yl]carbonyl]piperidin-1-yl]-1H-pyrazolo[3,4-d]pyrimidme; or a pharmaceutically acceptable salt thereof.

Another concrete examples of the compound [I] of the present invention include a compound of the following formula [IB]: 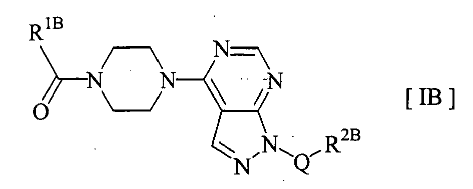 wherein R^{1B} is a group of the following formula (Ba) or (Bb): 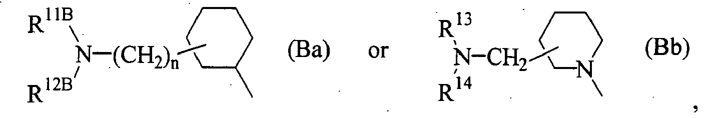 R^{11B} and R^{12B} are the same or different and a hydrogen atom, a lower alkyl group, a hydroxy-lower alkyl group, a lower alkoxy-lower alkyl group, a lower alkanoyl group, a lower alkoxy-carbonyl group, a lower alkenoyl group, an amino-lower alkyl group (amino moiety of said group being optionally substituted by a lower alkyl group) or a cyclo-lower alkyl-carbonyl group, or both of them combine each other at their termini together with an adjacent nitrogen atom to form a nitrogen-containing heterocyclic group, R¹³ and R¹⁴ are the same or different and a hydrogen atom, a lower alkyl group, a hydroxy-lower alkyl group, a lower alkoxy-lower alkyl group, a lower alkanoyl group, a lower alkenoyl group, a cyclo-lower alkyl-carbonyl group, an amino-lower alkyl group (amino moiety of said group being optionally substituted by a lower alkyl group), a nitrogen-containing heterocyclic group optionally substituted by a lower alkyl group or a lower alkyl group substituted by a nitrogen-containing heterocyclic group, n is an integer of 0 or 1,
Q is a lower alkylene group,
R^{2B} is a group of the following formula: 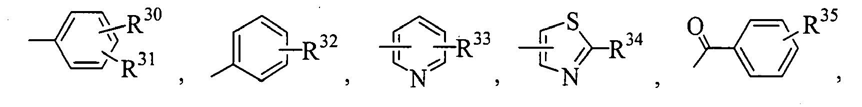 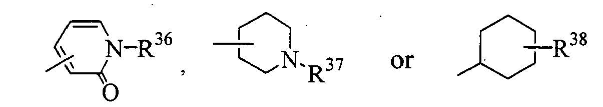 R³⁰ is a halogen atom, R³¹ is a lower alkyl group optionally substituted by a halogen atom, R³², R³³, R³⁴ and R³⁵ are a lower alkyl group, a lower alkoxy group, a halogeno-lower alkoxy group, a lower alkanoyl group or an amino group (said amino group being optionally substituted by a lower alkyl group), R³⁶, R³⁷ and R³⁸ are a lower alkyl group, a lower alkoxy group or a lower alkanoyl group,
provided that:
(i) R¹², R³³ and R³⁴ are not a lower alkyl group or a lower alkoxy group, when R^{1B} is a group of the formula (Ba), and
(ii) R^{2B} is not a group of the formula: 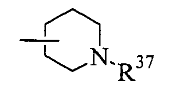
when n is an integer of 0;
or a pharmaceutically acceptable salt thereof.

More concrete examples of the above-mentioned compound [IB] include:
a compound in which compound in which R^{1B} is a substituted cyclohexyl group of the formula (Ba), one of R¹¹ and R¹² is a C₁₋₆ alkyl group, a hydroxy-C₁₋₆ alkyl group, a C₁₋₆ alkoxy-C₁₋₆ alkyl group, a di(C₁₋₆ alkyl)amino-C₁₋₆ alkyl group or a C₁₋₆ alkyl group substituted by a nitrogen-containing heterocyclic group and another is a C₁₋₆ alkyl group, a C₂₋₇ alkanoyl group or a C₃₋₈ cycloalkyl-carbonyl group, or both of them combine each other at their termini together with an adjacent nitrogen atom to form a nitrogen-containing heterocyclic group, Q is a C₁₋₆ alkylene group, R^{2B} is a group of the following formula: 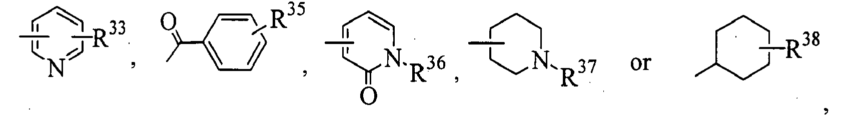 R³³ is a halogeno-C₁₋₆ alkoxy group, a C₂₋₇ alkanoyl group or a di(C₁₋₆ alkyl)amino group, R³⁵ is a C₁₋₆ alkoxy group, R³⁶ is a C₁₋₆ alkyl group, R³⁷ is a C₂₋₇ alkanoyl group and R³⁸ is a C₁₋₆ alkoxy group; or
a compound in which R^{1B} is a substituted piperidyl group of the formula (Bb), one of R¹³ and R¹⁴ is a C₁₋₆ alkyl group, a hydroxy-C₁₋₆ alkyl group, a C₁₋₆ alkoxy-C₁₋₆ alkyl group, a di(C₁₋₆ alkyl)amino-C₁₋₆ alkyl group or a C₁₋₆ alkyl group substituted by a nitrogen-containing heterocyclic group and another is a C₁₋₆ alkyl group, a C₁₋₆ alkyl-substituted nitrogen-containing heterocyclic group, a C₂₋₇ alkanoyl group or a C₃₋₈ cycloalkyl-carbonyl group, Q is a C₁₋₆ alkylene group, R^{2B} is a group of the following formula: 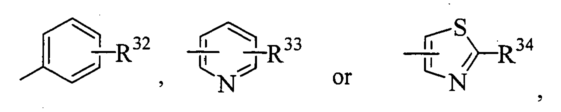 R³² is a C₁₋₆ alkoxy group, R³³ is a C₁₋₆ alkyl group or a C₁₋₆ alkoxy group and R³⁴ is a C₁₋₆ alkyl group.

With regard to the above-mentioned compound [IB] of the present invention, the nitrogen-containing heterocyclic group in R^{11B}, R^{12B}, R¹³ or R¹⁴ is a saturated or unsaturated nitrogen-containing 5- or 6-membered heterocyclic group such as a pyrrolidinyl group or a piperidyl group.

Among the above-mentioned compound [IB] of the present invention, preferred examples include a compound in which Q is methylene group.

Among the compound [IB], more preferred examples include a compound of the following formula [IB-a]: 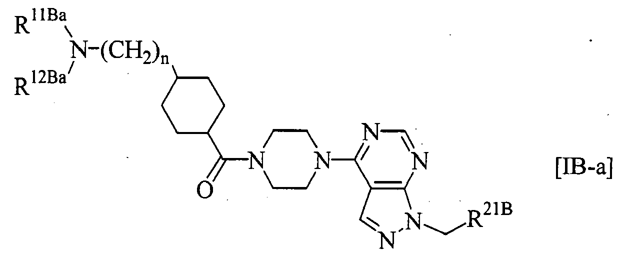 in which one of R^{11Ba} and R^{12Ba} is a lower alkyl and another is a hydroxy-lower alkyl group or a lower alkoxy-lower alkyl group, or both of them combine each other at their termini together with an adjacent nitrogen atom to form a 5- or 6-membered nitrogen-containing heterocyclic group, R^{21B} is a group of the following formula: 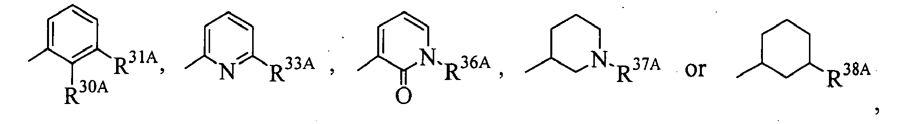 R^{30A} is a halogen atom, R^{31A} is a halogeno-lower alkyl group, R^{33A} is an amino group optionally substituted by a lower alkyl group, or a lower alkanoyl group, R^{36A} is a lower alkyl group, R^{37A} is a lower alkanoyl group, R^{38A} is a lower alkoxy group and n is an integer of 0 or 1;
provided that R^{21B} is not a group of the formula: 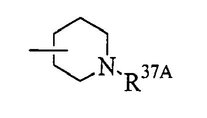 when n is an integer of 0;
or a pharmaceutically acceptable salt thereof.

Other preferred examples of the compound include a compound of the following formula [IB-b]: 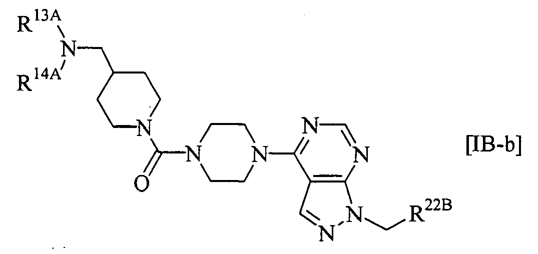 in which one of R^{13A} and R^{14A} is a lower alkyl group, an amino-lower alkyl group (the amino moiety of said group being optionally substituted by a lower alkyl group), a nitrogen-containing 5- or 6-membered heterocyclic group optionally substituted by a lower alkyl group or a lower alkyl group substituted by a nitrogen-containing 5- or 6-membered heterocyclic group and another is a lower alkyl group, a hydroxy-lower alkyl group, a lower alkoxy-lower alkyl group, a lower alkanoyl group or a cyclo-lower alkyl-carbonyl group, R^{22B} is a group of the following formula: 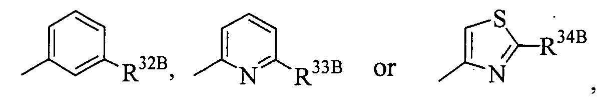 R^{32B} is a lower alkoxy group, R^{33B} is a lower alkyl group or a lower alkoxy group and R^{34B} is a lower alkyl group or a pharmaceutically acceptable salt thereof.

Among the above-mentioned compound [IB], the further preferred examples include:
(1) a compound [IB-a] in which one of R^{11Ba} and R^{12Ba} is a C₁₋₄ alkyl group such as a tert-butyl group and another is a hydroxy-C₁₋₄ alkyl group such as a hydroxyethyl group or a C₁₋₄ alkoxy-C₁₋₄ alkyl group such as a methoxyethyl group, or both of them combine each other at their termini together with an adjacent nitrogen atom to form a nitrogen-containing 6-membered heterocyclic group such as a piperidyl group, R^{30A} is fluorine atom, R^{31A} is a trifluoro-C₁₋₄ alkyl group such as a trifluoromethyl group, R^{33A} is a di(C₁₋₄ alkyl)amino group such as a dimethylamino group or a C₂₋₅ alkanoyl group such as an acetyl group or a propionyl group, R ^{36A} is a C₁₋₄ alkyl group such as a methyl group, an ethyl group or a propyl group, R^{37A} is a C₂₋₅ alkanoyl group such as an acetyl group or a propionyl group, and R^{38A} is a C₁₋₄ alkoxy group such as a methoxy group, an ethoxy group or a propyloxy group; and
(2) a compound [IB-b] in which:
   i) one of R^{13A} and R^{14A} is a C₁₋₄ alkyl group such as a methyl group, an ethyl group, a propyl group or a tert-butyl group or a C₁₋₄ alkyl-substituted nitrogen-containing 6-membered heterocyclic group such as a methylpiperidyl group and another is a C₁₋₄ alkyl group such as an isopropyl group or a tert-butyl group, a hydroxy-C₁₋₄ alkyl group such as a hydroxyethyl group or a C₁₋₄ alkoxy-C₁₋₄ alkyl group such as a methoxyethyl group, R^{32B} is a C₁₋₄ alkoxy group such as a methoxy group, an ethoxy group or a propyloxy group, R^{33B} is a C₁₋₄ alkyl group such as a methyl group, an ethyl group, a propyl group or a tert-butyl group or a C₁₋₄ alkoxy group such as a methoxy group or an ethoxy group, R^{34B} is a C₁₋₄ alkyl group such as a methyl group, an ethyl group, a propyl group or a tert-butyl group; or
   ii) one of R^{13A} and R^{14A} is a di(C₁₋₄ alkyl)amino-C₁₋₄ alkyl group such as a dimethylaminoethyl group or a diisopropylaminoethyl group or a nitrogen-containing 5-membered heterocyclic group-substituted C₁₋₄ alkyl group such as a pyrrolidinylethyl group and another is a C₂₋₅ alkanoyl group such as an acetyl group or a C₃₋₆ cycloalkyl-carbonyl group such as a cyclopropylcarbonyl group, R^{32B} is a C₁₋₄ alkoxy group such as a methoxy group, an ethoxy group or a propyloxy group, R^{33B} is a C₁₋₄ alkyl group such as a methyl group, an ethyl group, a propyl group or a tert-butyl group or a C₁₋₄ alkoxy group such as a methoxy group, an ethoxy group or a propyloxy group and R^{34B} is a C₁₋₄ alkyl group such as a methyl group, an ethyl group, a propyl group or a tert-butyl group.

Particularly preferred examples of the above-mentioned compound [IB] include:
4-[4-[(trans-4-piperidin-1-ylcyclohexyl)carbonyl]piperazin-1-yl]-1-[(6-propionylp yridin-2-yl)methyl]-1H-pyrazolo[3,4-d]pyrimidine;
4-[4-[[trans-4-[[N-(2-methoxyethyl)-N-(tert-butyl)amino]methyl]cyclohexyl]carbo nyl]piperazin-1-yl]-1-(1-propionylpiperidin-3-yl)-1H-pyrazolo[3,4-d]pyrimidine;
1-[(3-methoxycyclohexyl)methyl]-4-[4-[[trans-4-[[N-(2-methoxyethyl)-N-(tert-but yl)amino]methyl]cyclohexyl]carbonyl]piperazin-1-yl]-1H-pyrazolo[3,4-d]pyrimidine;
1-(2-oxo-1-propyl-1,2-dihydropyridin-3-yl)-4-[4-[[trans-4-[[N-tert-butyl-(2-metho xyethyl)amino]methyl]cyclohexyl]carbonyl]piperazin-1-yl]-1H-pyrazolo[3,4-d]pyrimidine;
1-(2-oxo-1-propyl-1,2-dihydropyridin-3-yl)-4-[4-[(trans-4-piperidin-1-ylcyclohexy 1)carbonyl]piperazin-1-yl]-1H-pyrazolo [3,4-d]pyrimidine;
1-[2-fluoro-3-(trifluoromethyl)benzyl]-4-[4-[(trans-4-piperidin-1-ylcyclohexyl)car bonyl]piperazin-1-yl]-1H-pyrazolo[3,4-d]pyrimidine;
1-(3-ethoxybenzyl)-4-[4-[[4-[[N-[2-(diisopropylamino)ethyl]-N-pivaloylamino]me thyl]piperidin-1-yl]carbonyl]piperazin-1-yl]-1H-pyrazolo[3,4-d]pyrimidine;
1-[[(2-propyl-1,3-thiazol-4-yl)methyl]-4-[4-[[4-[[N-[2-(diisopropylamino)ethyl]-N -pivaloylamino]methyl]piperidin-1-yl]carbonyl]piperazin-1-yl]-1H-pyrazolo[3,4-d]pyrimidi ne;
1-[(6-ethoxypyridin-2-yl)methyl]-4-[4-[[4-[[N-(cyclopropylcarbonyl)-N-[2-(dimet hylamino)ethyl]amino]methyl]piperidin-1-yl]carbonyl]piperazin-1-yl]-1H-pyrazolo[3,4-d]p yrimidine;
1-[(6-ethoxypyridin-2-yl)methyl]-4-[4-[[4-[[N-(cyclopropylcarbonyl)-N-[2-(diisop ropylamino)ethyl]amino]methyl]piperidin-1-yl]carbonyl]piperazin-1-yl]-1H-pyrazolo[3,4-d ]pyrimidine;
1-[(6-ethoxypyridin-2-yl)methyl]-4-[4-[[4-[[N-[2-(diisopropylamino)ethyl-N-pival oylamino]methyl]piperidin-1-yl]carbonyl]piperazin-1-yl]-1H-pyrazolo[3,4-d]pyrimidine;
1-(3-ethoxybenzyl)-4-[4-[[4-[[N-(2-methoxyethyl)-N-(tert-butyl)amino]methyl]pip eridin-1-yl]carbonyl]piperazin-1-yl]-1H-pyrazolo[3,4-d]pyrimidine;
1-(3-ethoxybenzyl)-4-[4-[[4-[[N,N-diisopropylamino)methyl]piperidin-1-yl]carbo nyl]piperazin-1-yl]-1H-pyrazolo[3,4-d]pyrimidine;
1-(3-ethoxybenzyl)-4-[4-[[4-[[N-ethyl-N-(tert-butyl)amino]methyl]piperidin-1-yl]c arbonyl]piperazin-1-yl]-1H-pyrazolo[3,4-d]pyrimidine;
1-[(2-propyl-1,3-thiazol-4-yl)methyl]-4-[4-[[4-[[N-ethyl-N-(tert-butyl)amino]meth yl]piperidin-1-yl]carbonyl]piperazin-1-yl]-1H-pyrazolo[3,4-d]pyrimidine;
1-[(2-propyl-1,3-thiazol-4-yl)methyl]-4-[4-[[4-[(N,N-diisopropylamino)methyl]pip eridin-1-yl]carbonyl]piperazin-1-yl]-1H-pyrazolo[3,4-d]pyrimidine;
1-[(6-ethoxypyridin-2-yl)methyl]-4-[4-[[4-[[N-(2-methoxyethyl)-N-(tert-butyl)ami no]methyl]piperidin-1-yl]carbonyl]piperazin-1-yl]-1H-pyrazolo[3,4-d]pyrimidine;
1-[(6-ethoxypyridin-2-yl)methyl]-4-[4-[[4-[(N,N-diisopropylamino)methyl]piperid in-1-yl]carbonyl]piperazin-1-yl]-1H-pyrazolo[3,4-d]pyrimidine;
1-[(6-ethoxypyridin-2-yl)methyl]-4-[4-[[4-[[N-ethyl-N-(tert-butyl)amino]methyl]p iperidin-1-yl]carbonyl]piperazin-1-yl]-1H-pyrazolo[3,4-d]pyrimidine;
1-[(6-ethoxypyridin-2-yl)methyl]-4-[4-[[4-[[N-(2-hydroxyethyl)-N-(tert-butyl)ami no]methyl]piperidin-1-yl]carbonyl]piperazin-1-yl]-1H-pyrazolo [3,4-d]pyrimidine;
1-[(6-ethoxypyridin-2-yl)methyl]-4-[4-[[4-[[N-(2-hydroxyethyl)-N-isopropylamin o]methyl]piperidin-1-yl]carbonyl]piperazin-1-yl]-1H-pyrazolo[3,4-d]pyrimidine;
1-[(6-propylpyridin-2-yl)methyl]-4-[4-[[4-[[N-(2-methoxyethyl)-N-(tert-butyl)ami no]methyl]piperidin-1-yl]carbonyl]piperazin-1-yl]-1H-pyrazolo[3,4-d]pyrimidine;
1-[(6-propylpyridin-2-yl)methyl]-4-[4-[[4-[(N,N-diisopropylamino)methyl]piperid in-1-yl]carbonyl]piperazin-1-yl]-1H-pyrazolo[3,4-d]pyrimidine;
1-[(6-propylpyridin-2-yl)methyl]-4-[4-[[4-[[N-ethyl-N-(tert-butyl)amino]methyl]pi peridin-1-yl]carbonyl]piperazin-1-yl]-1H-pyrazolo [3,4-d]pyrimidine;
1- [(6-propylpyridin-2-yl)methyl]-4-[4-[[4-[[N-(2-hydroxyethyl)-N-(isopropylamin o)methyl]piperidin-1-yl]carbonyl]piperazin-1-yl]-1H-pyrazolo[3,4-d]pyrimidine; or a pharmaceutically acceptable salt thereof.

When the compound [I] of the present invention has an asymmetric carbon atom(s) at the substituent(s) in R¹, it may exist in the form of a stereoisomer thereof (diastereoisomers, optical isomers) owing to said asymmetric carbon atom(s) thereof, and the present invention also includes these stereoisomers and a mixture thereof.

A compound [I] of the present invention or a pharmaceutically acceptable salt thereof shows a significant antagonizing activity against apamin, which is known as a selective SK channel blocker, in a competitive binding assay. Therefore, the compound [I] or a pharmaceutically acceptable salt thereof is useful as a SK channel blocker which is applicable to treatment and/or prophylaxis of SK channel-related diseases such as gastrointestinal motility disorders (e.g., constipation, irritable bowel syndrome, post-operative ileus, gastoroesophageal reflux disease), central nervous system disorders (e.g., memory and learning disorders, emotional disorders, Arzheimer's disease, depression, Perkinson's disease), myotonic muscular dystrophy or sleep apnea.

Moreover, the compound of the present invention shows a low toxicity and are safe as a medicament.

The compound [I] of the present invention can be clinically used either in the free form or in the form of a pharmaceutically acceptable salt thereof. The pharmaceutically acceptable salt of the compound [I] includes a salt with an inorganic acid salt such as hydrochloride, sulfate, phosphate or hydrobromide, or an organic acid salt such as an acetate, an oxalate, a citrate, a methanesulfonate, a benzenesulfonate, a tosylate or a maleate. Besides, when the compound [I] of the present invention has a carboxyl group(s) and the like in its molecule, examples of the pharmaceutically acceptable salt include, salts with a base such as an alkali metal (e.g., sodium salt, potassium salt). or an alkali earth metal (e.g., calcium salt).

The compound [I], a salt thereof, or its intermediate or a salt thereof includes either intramolecular salt or an additive thereof, and solvates or hydrates thereof.

The present compound [I] or a pharmaceutically acceptable salt thereof can be either orally or parenterally, and can be formulated into a conventional pharmaceutical preparation such as tablets, granules, fine granules, capsules, powders, injections or inhalants.

The dose of the compound [I] of the present invention or a pharmaceutically acceptable salt thereof may vary in accordance with the administration routes, and the ages, weights and conditions of the patients. For example, when administered as an injection preparation, it is usually in the range of about 0.0001 to 1 mg/kg/day, preferably in the range of about 0.001 to 0.1 mg/kg/day. When administered as an oral preparation, it is usually in the range of about 0.001 to 100 mg/kg/day, preferably in the range of 0.01 to 10 mg/kg/day.

The compound [I] of the present invention may be prepared by, for example, the following manners, but should not be construed to be limited thereto.

### Process A:

Among the compound [I] of the present invention, a compound of the following formula [I-a] : 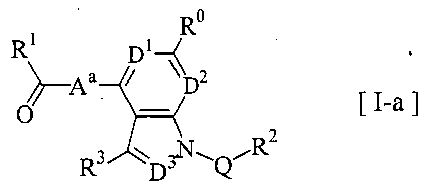 wherein the group represented by -C(=O)-A^{a}- is a group of the following formula: 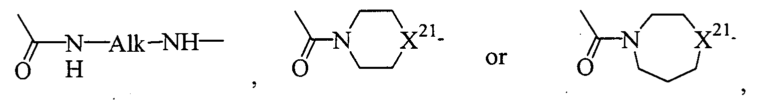 X²¹ is N or CH and other symbols are the same as defined above, can be prepared by reacting a compound of the following formula [IIa]: 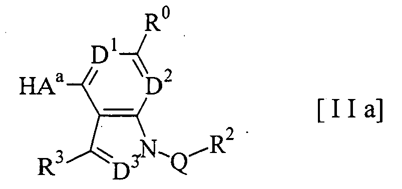 wherein the symbols are the same as defined above or a salt thereof with a compound of the following formula [IIIa]:

R¹-COOR⁴ [IIIa]

wherein R⁴ is a hydrogen atom, a lower alkyl group or a benzyl group and other symbol is the same as defined above.

When R⁴ is a hydrogen atom, the above-mentioned reaction can be carried out in a solvent in the presence of a condensing agent and in the presence or absence of an activating agent and a base. Examples of the solvent include any solvent which does not disturb the reaction, such as methylene chloride, chloroform, dimethylformaide, dimethylacetamide, tetrahydrofuran, dioxane, toluene, benzene, 1,2-dichloroethane, 1-methyl-2-pyrrolidinone, 1,2-dimethoxyethane and the like.
The condensing agent includes, for example, dicyclohexylcarbodiimide (DCC), 1-ethyl-3-(3-dimethylaminopropyl)carbodiimide · hydrochloride (WSC · HCl), diphenylphosphoryl azide (DPPA), carbonyldiimidazole (CDI), diethylcyanophosphonate (DEPC), diisopropylcarbodiimide (DIPCI), benzotriazol-1-yloxy-trispyrrolidinophosphonium hexafluorophosphate (PyBOP), carbonylditriazole, N-cyclohexylcarbodiimide-N'-propyloxymethylpolystyrene (PS-carbodiimide), N-ethoxycarbonyl-2-ethoxy-1,2-dihydroquinoline (EEDQ), 2-(7-azabenzotriazol-1-yl)-1,1,3,3-tetramethyl-uronium hexafluorophosphate (HATU), 2-(1H-benzotriazol-1-yl-1,1,3,3-tetramethyl-uronium hexafluorophosphate (HBTU), bromotrispyrrolidinophosphonium hexafluorophosphate (PyBroP), 2-(1H-benzotriazol-1-yl)-1,1,3,3-tetramethyluronium tetrafluoroborate (TBTU), chloro-1,1,3,3-tetramethyluronium hexachloroantimonate (ACTU) and the like. Examples of the activating agent include 1-hydroxybenzotriazole (HOBt), 1-hydroxysuccinimide (HOSu), dimethylaminopyridine (DMAP), 1-hydroxy-7-aza- benzotriazole (HOAt), hydroxyphthalimide (HOPht), pentafluorophenol (Pfp-OH), 1-hydroxybenzotriazole-6-sulfonamidomethylpolystyrene (PS-HOBt) and the like. The base includes, for example, pyridine, triethylamine, diisopropylethylamine, 4-methylmorpholine, 1,8-diazabicyclo[5,4,0]-7-undecene (DBU), potassium carbonate, sodium carbonate, cesium carbonate, sodium hydrogencarbonate and the like.

In the above-mentioned process, the compound [IIa] can be used in an amount of 0.3 to 10 moles, preferably 0.5 to 2 moles per mole of the compound [IIIa]. The condensing agent can be used in an amount of 1 to 10 moles, preferably 1.5 to 4 moles per one mole of the compound [IIa] or [IIIa]. The base can be used in an amount of 1 to 10 moles, preferably 2 to 4 moles per one mole of the compound [IIa] or [IIIa]. The activating agent can be used in an amount of 1 to 10 moles, preferably 1.5 to 4 moles per one mole of the compound [IIa] or [IIIa]. The reaction can be carried out at -20 to 80 °C, preferably 0 to 30 °C.

Meanwhile, when R⁴ in the compound [IIIa] is hydrogen atom, the reaction process A to prepare the compound [I-a] can be carried out by converting the compound to a reactive derivative at the carboxyl group (e.g., an acid halide, a mixed acid anhydride) and the like, and reacting the reactive derivative with the compound [IIa] in the presence of the base mentioned above in the solvent or without solvent.

When R⁴ in the compound [IIIa] is a lower alkyl group or a benzyl group, the reaction process A can be also carried out by converting the compound to a corresponding carboxylic acid compound by a conventional manner such as hydrolysis, acidolysis using hydrochloric acid, formic acid, trifluoroacetic acid and the like or hydrogenation, and then reacting the carboxylic acid compound with the compound [IIa] by the above-mentioned manner.

Furthermore, when R⁴ in the compound [IIIa] is a lower alkyl group or a benzyl group, the reaction process A can be also carried out by directly reacting the ester compound [IIa] with the compound [IIIa] in the presence of a base in a solvent or without solvent. The solvent includes any solvent which does not disturb the reaction, such as methylene chloride, chloroform, dimethylformamide, dimethylacetamide, tetrahydrofuran, dioxane, toluene, benzene, 1 ;2-dichloroethane, 1-methyl-2-pyrrolidinone, methanol, ethanol, isopropanol and the like. The base includes, for example, triethylamine, diisopropylethylamine, 4-methylmorpholine, 1,8-diazabicyclo [5,4,0]-undecene (DBU), dimethylaminopyridine (DMAP) and the like.

In the above-mentioned process, the compound [IIIa] can be used in an amount of 0.3 to 10 moles, preferably 0.5 to 2 moles per mole of the compound [IIa]. The base can be used in an amount of 1 to 10 moles, preferably 1 to 4 moles per one mole of the compound [IIa] or [IIIa]. The reaction can be carried out at 25 to 150 °C, preferably 60 to 120 °C.

### Process B:

Among the above-mentioned compounds [I], a compound of the following formula [I-b] : 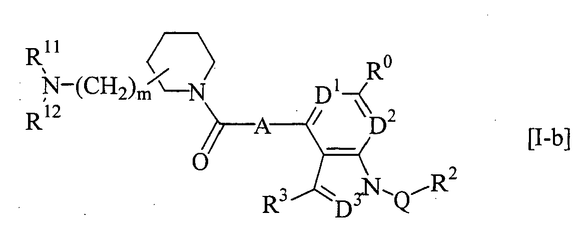 wherein the symbols are the same as defined above can be prepared by reacting a compound of the following formula [IIb]: 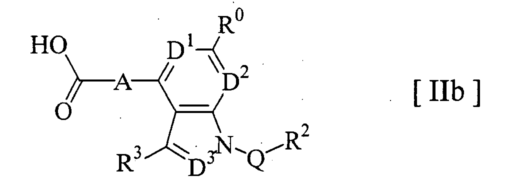 wherein the symbols are the same as defined above, its reactive derivative or a salt thereof with a piperidine compound of the following formula [IIIb] : 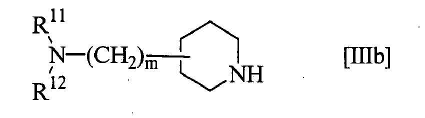 wherein the symbols are the same as defined above or a salt thereof.

In case of preparing the compound [I-b] from the compound [IIb] or a salt thereof and the compound [IIIb], this reaction can be carried out in a solvent in the presence of a condensing agent and in the presence or absence of an activating agent or a base. Such solvent, condensing agent, activating agent and base can be selected from those exemplified in the above-mentioned Process A. The salt of the compound [IIb] may be hydrochloride and the like.

In case of using a reactive derivative of the compound [IIb], such as a corresponding acid halide, such reaction can be carried out in a solvent in the presence of a base which is exemplified in the above Process A.

In each of the above-mentioned processes, the compound [IIIb] can be used in an amount of 0.8 to 3 moles, preferably 1 to 1.5 moles per mole of the compound [IIb] or its reactive derivative. The base can be used in an amount of 1 to 4 moles, preferably 2 to 3 moles per one mole of the compound [IIb] or [IIIb].

The reaction can be carried out at - 20 to 150 °C, preferably 0 to 60 °C.

### Process C:

The compound [I-a] mentioned above can be also prepared by reacting a compound of the following formula [IIc]: 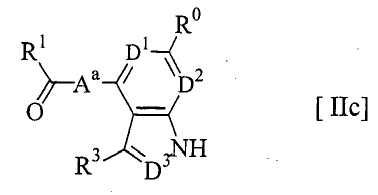 wherein the symbols are the same as defined above with a compound of the following formula [IIIc]:

R²-Q-X³ [IIIc]

wherein X³ is a reactive residue and other symbol is the same as defined above.

The reaction of the compound [IIc] with the compound [IIIc] can be carried out in an appropriate solvent in the presence of a dehydrating agent or a base. Examples of the solvent include any solvent which does not disturb the reaction, such as methylene chloride, chloroform, 1,2-dichloroethane, N,N-dimethylformaide, N,N-dimethylacetamide, 1-methyl-2-pyrrolidinone, tetrahydrofuran, 1,2-dimethoxy- ethane, 1,4-dioxane, toluene, benzene and the like. In case that X³ is a hydroxyl group, the dehydrating agent may be a combination of a lower alkyl azodicarboxylate such as diisopropyl azodicarboxylate and a tri-substituted phosphine such triphenylphosphine or phosphorane. Besides, in case that X³ is a removing group such as a halogen atom, a lower alkylsulphonyloxy group or an arylsulphonyloxy group, examples of the base include an alkali metal hydroxide such as lithium hydroxide, an alkali metal hydride, an alkali metal carbonate, an alkali metal alkoxide, lithium diisopropylamide (LDA) and the like.

In the above-mentioned processes, the compound [IIIc] can be used in an amount of 1 to 5 moles, preferably 1 to 2 moles per mole of the compound [IIc]. The dehydrating agent can be used in an amount of 1 to 5 moles, preferably 1 to 2 moles per one mole of the compound [IIc] or [IIIc]. The reaction can be carried out at -20 to 100 °C, preferably 0 to 30 °C.

### Process D:

Among the compounds [I] of the present invention, a compound of the following formula [I-d] : 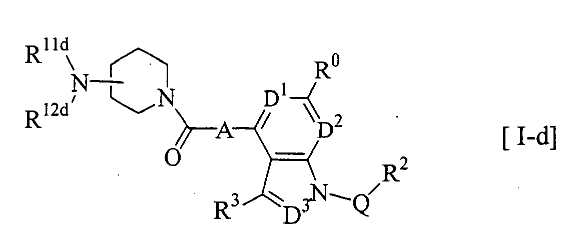 wherein R^{11d} and R^{12d} are the same or different and a hydrogen atom, a lower alkyl group, a hydroxy-lower alkyl group, a cyclo-lower alkyl group, a lower alkoxy-lower alkyl group, an amino-lower alkyl group (said amino moiety may be substituted by a lower alkyl group(s)), a lower alkoxy-carbonyl group or a nitrogen-containing heterocyclic group-substituted lower alkyl group or both of them combine each other at their termini together with an adjacent nitrogen atom to form a nitrogen-containing heterocyclic group (said heterocyclic group may be substituted by a lower alkyl group) and other symbols are the same as defined above, can be prepared by reacting a compound of the following formula [IId]: 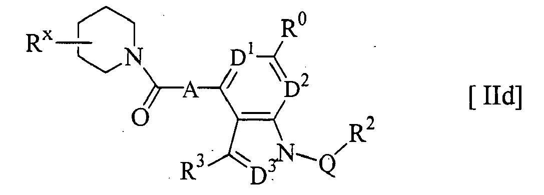 wherein R^{x} is an oxo group and other symbols are the same as defined above with an amine compound of the following formula [IIId]:

(R^{11d})(R^{12d})NH [IIId]

wherein the symbols are the same as defined above.

The present reaction can be carried out in an appropriate solvent in the presence of a reducing agent or under a condition of catalytic hydrogenation.

In case of using a reducing agent, examples of the solvent include any solvent which does not disturb the reaction, such as methanol, ethanol, propanol, isopropanol, tert-buthanol, 2-methoxyethanol, methylene chloride, chloroform, 1,2-dichloroethane, N,N-dimethylformaide, N,N-dimethylacetamide, 1-methylpyrrolidinone, tetrahydrofuran, 1,2-dimethoxyethane, 1,4-dioxane, toluene, benzene, acetic acid, sulfuric acid, hydrochloric acid, trifluoroacetic acid and the like. Examples of the reducing agent include macroporous triethylammoniummethylpolystyrene cyanoborohydride (MP-Cyanoborohydride), sodium borohydride, sodium triacetoxyborohydride, sodium cyanoborohydride and the like. The compound [IIId] can be used in an amount of 1 to 10 moles, preferably 1 to 2 moles per mole of the compound [IId]. The reducing agent can be used in an amount of 1 to 10 moles, preferably 1 to 4 moles per one mole of the compound [IId] or [IIId]. The reaction can be carried out at -20 to 100 °C, preferably 0 to 40°C.

In case of catalytic hydrogenation, examples of the solvent include any solvent which does not disturb the reaction, such as methanol, ethanol, propanol, isopropanol, tert-buthanol, 2-methoxyethanol, methylene chloride, chloroform, 1,2-dichloroethane, N,N-dimethylformaide, N,N-dimethylacetamide, 1-methylpyrrolidinone, tetrahydrofuran, 1,2-dimethoxyethane, 1,4-dioxane, toluene, benzene, acetic acid, sulfuric acid, hydrochloric acid and the like. Examples of the catalyst include palladium-carbon, palladium-black, platinum oxide and the like. The compound [IIId] can be used in an amount of 1 to 10 moles, preferably 1 to 2 moles per mole of the compound [IId]. The catalyst can be used in an amount of 0.01 to 1 moles, preferably 0.05 to 0.2 moles per one mole of the compound [IId] or [IIId]. The reaction can be carried out at -20 to 100°C, preferably 0 to 40°C.

The objective compound [I] of the present invention can be also prepared by intramolecularly converting the substituent(s) in R¹ of the compound [I] as obtained above to the other objective substituent(s). Such intramolecular conversion processes can be selected according to the kinds of the objective substituents, and may be carried out, for example, in the following methods (a) to (e).

### Method (a):

An objective compound [I] of the present invention having a substituent(s) containing a substituted lower alkoxy group in R¹ can be prepared (1) by reacting a compound [I] having a substituent(s) containing a hydroxyl group in R¹ with a lower alkyl halide having a corresponding substituent(s) in the presence of a base (e.g., sodium hydride, potassium carbonate), or (2) by reacting a compound [I] having a substituent(s) containing a hydroxyl group in R¹ with a lower alkanol having a corresponding substituent(s) in a solvent and in the presence of a dehydrating agent (e.g., tri-substituted phosphine such as triphenylphosphine or an lower alkyl azodicarboxylate such as isopropyl azodicarboxylate.

### Method (b):

An objective compound [I] of the present invention having a substituent(s) containing a lower alkyl-amino group in R¹ can be prepared by reacting a corresponding compound [I] having a substituent(s) containing a primary or secondary amino group in R¹ with a corresponding lower alkyl halide in an appropriate solvent in the presence of a base.

### Method (c):

An objective compound [I] of the present invention having a substituent(s) containing an acyl-amino group such as a lower alkanoyl-amino group in R¹, namely a compound of the following formula [I-D]: 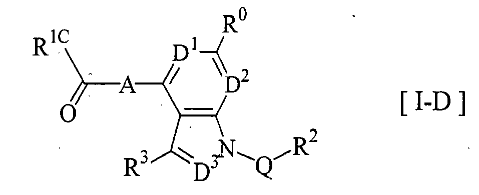 wherein R^{1C} is a group of the following formula: 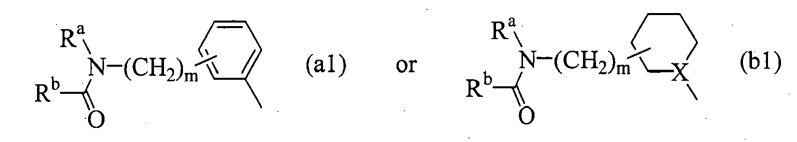 R^{a} is a hydrogen atom or a di(lower alkyl)amino-lower alkyl group, R^{b} is a lower alkyl group, a cyclo-lower alkyl group or a lower alkenyl group and the other symbols are the same as defined above can be prepared by reacting a corresponding compound [I] of the present invention having a substituent(s) containing a primary or secondary amino group in R¹, namely a compound of the following formula [I-E]: 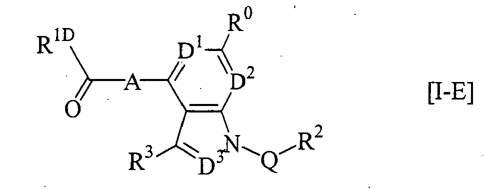 wherein R^{1D} is a group of the following formula (a2) or (b2): 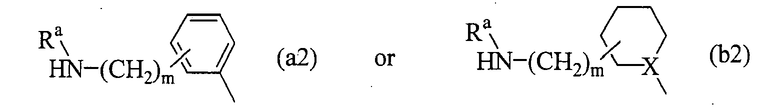 and other symbols are the same as defined above with a carboxylic acid compound of the following formula [IV]:

R^{b}-COOH [IV]

wherein the symbol is the same as defined above or its reactive derivative (e.g., a corresponding acid halide such as acid chloride) in the same manner as described in the above Process A.

### Method (d):

An objective compound [I] of the present invention having a substituent(s) containing a di-substituted amino group in R¹, namely a compound of the following formula [I-F] : 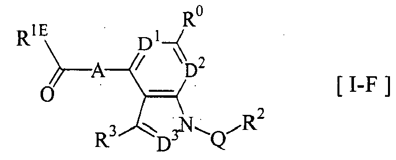 wherein R^{1E} is a group of the following formula (a3) or (b3): 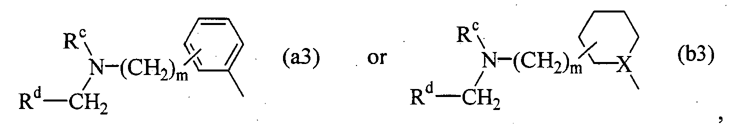 R^{c} is a lower alkyl group, a hydroxy-lower alkyl group, a cyclo-lower alkyl group, a lower alkoxy-lower alkyl group, a lower alkanoyl group, a lower alkenoyl group, a cyclo-lower alkyl-carbonyl group, a lower alkoxy-carbonyl group an amino-lower alkyl group (said amino moiety may be substituted by a lower alkyl group(s)) or a nitrogen-containing heterocyclic group-substituted lower alkyl group,
R^{d}-CH₂- is a lower alkyl group, a hydroxy-lower alkyl group, a lower alkoxy-lower alkyl group, an amino-lower alkyl group (said amino moiety may be substituted by a lower alkyl group(s)) or a nitrogen-containing heterocyclic group-substituted lower alkyl group, and other symbols are the same as defined above, can be prepared by reacting a corresponding compound [I] having a substituent(s) including secondary amino group in R¹, namely a compound of the following formula [I-G]: 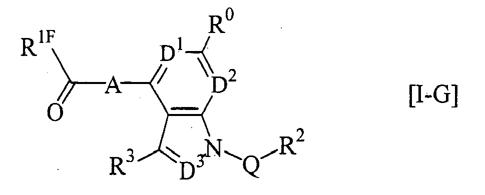 wherein R^{1F} is a group of the following formula (a4) or (b4): 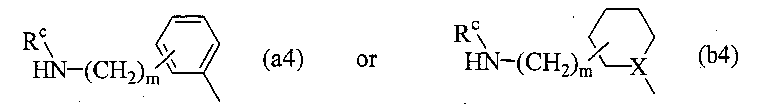 and other symbols are the same as defined above with an aldehyde compound of the formula [V]:

R^{d}-CHO [V]

wherein the symbol is the same as defined above or its hydrate in the same manner as described in the above Method (c).

### Method (e):

An objective compound [I] of the present invention having a substituent(s) in containing a nitrogen-containing hetero-monocyclic group of the formula: 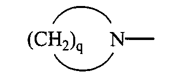 wherein q is an integer of 4 to 7 in R¹ can be prepared by reacting a corresponding compound [I] having a substituent(s) containing an amino group(s) with a compound [VIII] of the formula:

X^{a}-(CH₂)_{q}-X^{b} [VIII]

wherein X^{a} and X^{b} are a halogen atom and the other symbols are the same as defined above in an appropriate solvent in the presence of a base.

In conducting the above mentioned Processes A to D and Methods (a) to (e), when the starting materials or intermediate compounds have a functional group(s) such as amino group and the like, if necessary, the protection of the functional groups and the following deprotection thereof may be carried out in accordance with a conventional manner in synthetic chemistry.

Each of the intermediate compounds [IIa], [IIb], [IIc] and [IId] for preparing the compound [I] of the present invention can be prepared, for example, in accordance with a manner described in the following reaction scheme. 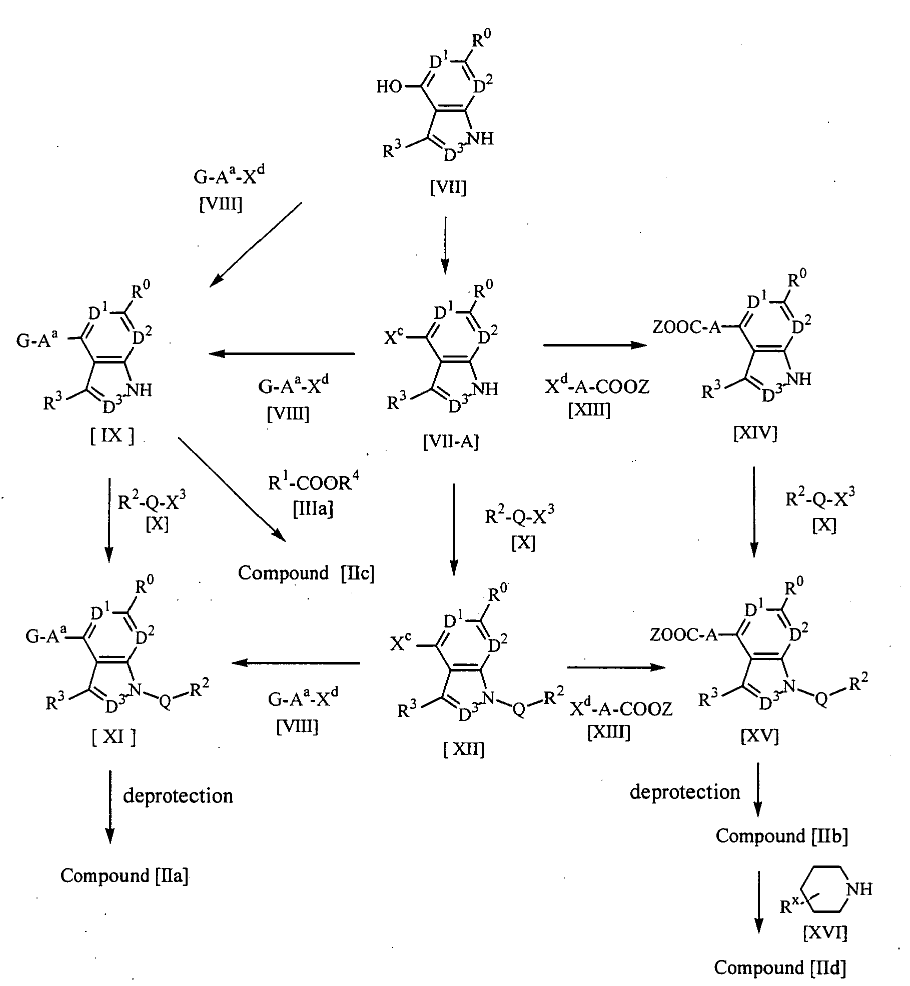 In the above reaction scheme, G is a hydrogen atom or an amino-protecting group, Z is a carboxy-protecting group, X³ is a hydroxyl group or a removing group, X^{c} is a halogen atom, a methylthio group, a methoxy group or a methylsulfinyl group, X^{d} is a hydrogen atom or a reactive residue and other symbols are the same as defined above.

The reaction for preparing the compound [IX] from the compound [VII] and the compound [VIII] can be carried out in a solvent or without any solvent in the presence or absence of an activating agent and in the presence or absence of an additive. Examples of the solvent include any solvent which does not disturb the reaction, such as xylene, toluene, dichloromethane, chloroform, 1,2-dichloroethane, tetrahydrofuran, 1,2-dimethoxyethane, 1,4-dioxane, acetonitrile and the like. Examples of the activating agent include hexamethyldisilazane, N,O-bis(trimethylsilyl)acetamide, chlorotrimethylsilane and the like. Eexamples of the additive include ammonium sulfate, triethylamine hydrochloride, pyridine hydrochloride and the like. The compound [VIII] can be used in an amount of 1 to 10 moles, preferably 1 to 4 moles per mole of the compound [VII]. The activating agent can be used in an amount of 1 to 20 moles, preferably 2 to 4 moles per one mole of the compound [VII]. The additive can be used in an amount of 0.01 to 10 moles, preferably 0.05 to 0.5 moles per one mole of the compound [VII]. The reaction can be carried out at 0 to 200 °C, preferably 80 to 150 °C.

The reaction for preparing the compound [VII-A] by halogenating the compound [VII] can be carried out in a solvent or without any solvent in the presence or absence of a base. Examples of the solvent include any solvent which does not disturb the reaction, such as toluene, xylene, dichloromethane, chloroform, 1,2-dichloroethane, tetrahydrofuran, dioxane, 1,2-dimethoxyethane and the like. The halogenating agent may be phosphorus oxychloride, phosphorus pentachloride, polyphosphoric acid, thionyl chloride, sulfuryl chloride and the like. The base my be diisopropylethylamine, triethylamine, pyridine, potassium carbonate, sodium carbonate, sodium hydrogencarbonate and the like.

The halogenating agent can be used in an amount of 1 to 50 moles, preferably 1 to 20 moles per one mole of the compound [VII]. The base can be used in an amount of 1 to 10 moles, preferably 1 to 2 moles per one mole of the compound [VII]. The reaction can be carried out at -20 to 150 °C, preferably 25 to 125 °C.

In case that the group X^{c} in the compound [VII-A] is a halogen atom, said group can be converted to a methylthio group, a methoxy group or a methylsulfinyl group, if necessary, and the compound having the desired group X^{c} can be selected and used in the following reaction step. The reaction for converting X^{c} (a halogen atom) to a methylthio group or a methoxy group can be carried out in a solvent or without any solvent in the presence of a methanethiol, methanol, a metal methylthiolate or a metal methoxide and in the presence or absence of a base. The metal in the metal methylthiolate and metal methoxide may be an alkali metal such as sodium, potassium, lithium or calcium. Examples of the solvent include any solvent which does not disturb the reaction, such as tetrahydrofuran, 1,4-dioxane, N,N-dimethylformamide, N,N-dimethylacetamide, 1-methyl-2-pyrrolidinone, dimethylsufoxide and the like. The base may be lithium diisopropylamide, potassium carbonate, sodium carbonate, potassium tert-butoxide, sodium tert-butoxide, diisopropylethylamine, triethylamine, 1,8-diazabicyclo[5,4,0]-7-undecene (DBU), 1,5-diazabicyclo[4.3.0]-5-nonene and the like.

The methanethiol, methanol, metal methylthiolate or metal methoxide can be used in an amount of 1 to 100 moles, preferably 1 to 20 moles per one mole of the compound [VII-A] in which X^{c} is a halogen atom. The base can be used in an amount of 1 to 10 moles, preferably 1 to 4 moles per one mole of the compound [VII-A] in which X^{c} is a halogen atom. The reaction can be carried out at -20 to 100 °C, preferably -20 to 30 °C.

The compound [VII-A] in which X^{c} is a methylsulfinyl group can be prepared by reacting the compound [VII-A] in which X^{c} is a methylthio group with an oxidizing agent. The present reaction can be carried out in a solvent or without any solvent. Examples of the solvent include any solvent which does not disturb the reaction such as toluene, xylene, dichloromethane, chloroform, 1,2-dichloroethane; tetrahydrofuran, 1,4-dioxane, 1,2-dimethoxyethane, methanol, ethanol, isopropanol, tert-buthanol and the like. The oxidizing agent may be m-chloroperbenzoic acid, sodium periodate and the like.

Such oxidizing agent can be used in an amount of 1 to 5 moles, preferably 1 to 1.5 moles per one mole of the compound [VII-A]. The reaction can be carried out at -20 to 80 °C, preferably 0 to 25 °C.

The reaction for preparing the compound [IX] from the compound [VII-A] and the compound [VIII] can be carried out in a solvent or without any solvent in the presence or absence of a base and in the presence or absence of an additive. Examples of the solvent include any solvent which does not disturb the reaction such as toluene, xylene, dichloromethane, chloroform, 1,2-dichloroethane, tetrahydrofuran, 1,4-dioxane, 1,2-dimethoxyethane, N,N-dimethylformamide, N,N-dimethylacetamide, dimethylsulfoxide, 1-methyl-2-pyrrolidone, acetonitrile, tert-buthanol and the like. The base may be potassium carbonate, sodium carbonate, potassium tert-butoxide, sodium tert-butoxide, diisopropylethylamine, triethylamine, 1 ,8-diazabicyclo[5,4,0]-7-undecene (DBU), 1,5-diazabicyclo[4.3.0]-5-nonene, lithium diisopropylamide and the like. The activating agent may be activated zinc powder, tetrakis(triphenylphosphine)palladium, dichlorobis-(triphenylphosphine)palladium, [1,1'-bis(diphenylphosphino)ferrocen]dichloroplladium, [1,2-bis(diphenylphosphino)ethane]dichloropalladium, bis(dibenzylideneacetone)palladium, tris(dibenzylidenacetone)dipalladium, triphenylphosphine, 1,1'-bis(diphenylphosphino)-ferrocen, tri(2-furyl)phosphine, 2,2'-bis(diphenylphosphino)-1,1'-binaphthyl (BINAP), 4,5-bis(diphenylphosphino)-9,9-dimethylxanthene and the like or a combination thereof. The compound [VIII] can be used in an amount of 0.5 to 10 moles, preferably 1 to 4 moles per one mole of the compound [VII-A]. The base can be used in an amount of 1 to 10 moles, preferably 1 to 4 moles per one mole of the compound [VII-A]. The additive can be used in an amount of 0.1 to 10 moles, preferably 0.1 to 3 moles per one mole of the compound [VII-A]. The reaction can be carried out at -20 to 150 °C, preferably 0 to 120 °C. Meanwhile, the aforementioned reactions of the compound [VII] with the compound [VII-A] and the compound [VII-A] with the compound [VIII] can be also carried out successively without isolating such intermediate compound [VII-A] in the same reaction vessel.

The reaction for preparing the compound [XI] from the compound [IX] and the compound [X] can be carried out in an appropriate solvent in the presence of a dehydrating agent or a base. Examples of the solvent include any solvent which does not disturb the reaction such as methylene chloride, chloroform, 1,2-dichloroethane, N,N-dimethylformamide, N,N-dimethylacetamide, 1-methylpyrrolidinone, tetrahydrofuran, 1,2-dimethoxyethane, 1,4-dioxane, toluene and the like. In case that X³ is a hydroxyl group, examples of the condensing agent include a combination of di(lower alkyl) azodicarboxylate such as diisopropyl azodicarboxylate and tri-substituted phosphine such as triphenylphosphine or a phosphorane. In case that X³ is a removing group (e.g., a halogen atom, a lower alkyl-sulfonyloxy group or an aryl-sulfonyloxy group), the base may be an alkali metal hydroxide such as lithium hydroxide, an alkali metal hydride, an alkali metal carbonate, an alkali metal lower alkoxide, lithium diisopropylamide (LDA) and the like.

The compound [X] can be used in an amount of 1 to 5 moles, preferably 1 to 2 moles per one mole of the compound [IX]. The dehydrating agent can be used in an amount of 1 to 5 moles, preferably 1 to 2 moles per one mole of the compound [IX] or [X]. The base can be used in an amount of 1 to 10 moles, preferably 1 to 2 moles per one mole of the compound [IX] or [X]. The reaction can be carried out at -20 to 100 °C, preferably 0 to 30°C.

The reaction for preparing the compound [XIV] from the compound [VII-A] and the compound [XIII], the reaction for preparing the compound [XV] from the compound [XII] and the compound [XIII] and the reaction for preparing the compound [XI] from the compound [XII] and the compound [VIII] can be carried out in the same manner as described in the reaction for preparing the compound [IX] from the compound [VII-A] and the compound [VIII].

The reaction for preparing the compound [XII] from the compound [VII-A] and the compound [X] and the reaction for preparing the compound [XV] from the compound [XIV] and the compound [X] can be carried out in the same manner as described in the reaction for preparing the compound [XI] from the compound [IX] and the compound [X].
The removal of the amino-protecting group G in the compound [XI] can be carried out in accordance with a conventional manner. The amino-protecting group may be a benzyl group, a lower alkoxy-carbonyl group such as an ethoxycarbonyl group or a tert-butoxycarbonyl group and the like.
The removal of the carboxy-protecting group Z in the compound [XV] can be carried out in a conventional manner. Examples of such carboxy-protecting group Z include a lower alkoxy group such as methoxy group, ethoxy group or tert-butoxy group and the like.

The reaction for preparing the compound [IIc] from the compound [IX] in which G is a hydrogen atom and the compound [IIIa] can be carried out in the same manner as described in the Process A.
The reaction for preparing the compound [IId] from the compound [IIb] and the compound [XVI] can be carried out in the same manner as described in the Process A.

The compound [IIIa], [IIIb], [IIIc] or [IIId] as a starting material in the present invention is a known compound or a compound being obtainable in accordance with a conventional manner in the filed of synthetic chemistry. For example, the compound [IIIa] can be prepared by subjecting a compound of the following formula: 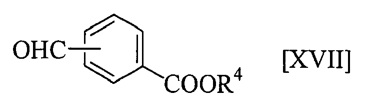 wherein the symbols are the same as defined above or a compound of the following formula [XVIII]: 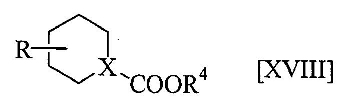 wherein R is a carboxyl group, a formyl group or an oxo group and the other symbols are the same as defined above to amidation followed by reduction or reductive amination in the presence of a compound of the following formula [XIX] :

(R¹¹)(R¹²)NH [XIX]

wherein the symbols are the same as defined above; or
by reacting a compound of the following formula [XX]: 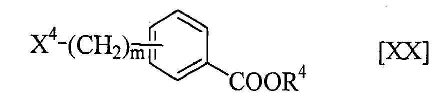 wherein X⁴ is a halogen atom and other symbols are the same as defined above with a compound [XIX].

Besides, the compound [IIIb] can be prepared by subjecting a compound of the following formula [XXI]: 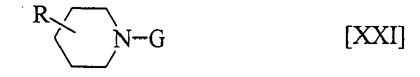 wherein R is a carboxyl group, a formyl group or an oxo group and other symbol is the same as defined above to amidation followed by reduction or reductive amination in the presence of the compound [XIX].

The thus-obtained intermediate compounds such as the compound [IIIa] can be also prepared by intramolecularly converting a substituent(s) (R¹¹ and/or R¹²) in R¹ in said compound to the other desired substituent(s). Such intramolecular conversion processes can be selected according to the kinds of the objective substituents, and N-alkylation, N-acylation, O-alkylation and the like can be applicable to such conversion.

Meanwhile, a compound [IIa-i], which is an intermediate compound for preparing a compound [IB], of the following formula: 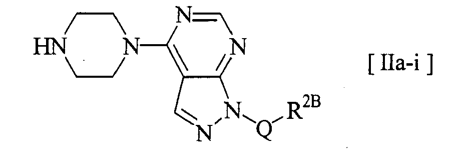 wherein the symbols are the same as defined above can be prepared, in accordance with the above-mentioned reaction scheme to obtain the compound [IIa], by reacting a compound of the formula [VII-i]: 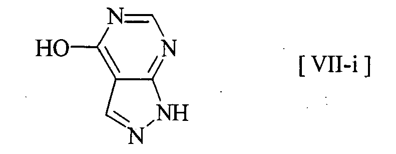 wherein the symbols are the same as defined above with a compound of the formula [VIII-i]: 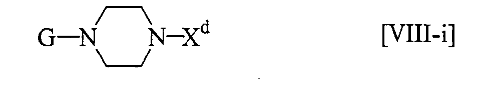 wherein the symbols are the same as defined above to give a compound of the formula [IX-i]: 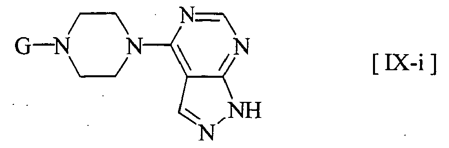 wherein the symbols are the same as defined above, reacting the product [IX-i] with a compound of the following formula [X-i]:

R^{2B}-Q-X³ [X-i]

wherein the symbols are the same as defined above, and removing the protecting group G.

If desired, the compounds [I] of the present invention obtained in the aforementioned Processes A to D or Methods (a) to (e) can be converted to a pharmaceutically acceptable salt thereof. Such conversion may be conducted by a process as known to persons having ordinary skills in the art.

In the present invention, the "lower alkyl" means a straight or branched chain alkyl having 1 to 6 carbon atoms, preferably 1 to 4 carbon atoms. The "lower alkoxy" means a straight or branched chain alkoxy having 1 to 6 carbon atoms, preferably 1 to 4 carbon atoms. The "lower alkylene" means a straight or branched chain alkylene having 1 to 6 carbon atoms, preferably 1 to 4 carbon atoms. The "lower alkanoyl" means a straight or branched chain alkanoyl having 2 to 7 carbon atoms, preferably 2 to 5 carbon atoms. The "cyclo-lower alkyl" means a cycloalkyl having 3 to 8 carbon atoms, preferably 3 to 6 carbon atoms. The "lower alkenyl" means a straight or branched chain alkenyl having 2 to 8 carbon atoms, preferably 2 to 4 carbon atoms. The "lower alkenoyl" means a straight or branched chain alkenoyl having 3 to 8 carbon atoms, preferably 3 to 6 carbon atoms. The "halogen atom" means fluorine, chlorine, bromine or iodine atom. The "heteroaryl" means a 5- to 14-membered nitrogen-, sulfur- or oxygen-containing heteroaryl, preferably a 5- to 6-membered monocyclic heteroaryl or a 9- to 10-membered bicyclic heteroaryl, which contains at least one nitrogen atom as a heteroatom.

### EXAMPLES

The present invention is illustrated in more detail by the following Examples and Reference Examples but should not be construed to be limited thereto.

### Example A1

(1) To a solution of methyl trans-4-[[N-(2-methoxyethyl)-N-(tert-butyl)amino]-methyl]cyclohexanecarboxylate (43 mg; compound obtained in Reference Example A1) in ethanol (1.0 mL) was added 2N sodium hydroxide solution (100 µL) and the mixture was stirred at 60 °C for 3 hours. To the reaction mixture was added 5N HCl (50 µL) and the mixture was concentrated in vacuo to give crude trans-4-[[N-(2-methoxyethyl)-N-(tert-butyl)amino]methyl]cyclohexanecarboxylic acid. To the compound was added successively chloroform (1.0 mL), 6-chloro-1-(3-ethoxy-benzyl)-4-(piperazin-1-yl)-1H-pyrazolo[3,4-d]pyrimidine dihydrochloride (38 mg; compound obtained in Reference Example A7), 1-hydroxybenzotriazole (21 mg), triethylamine (85 µL) and 1-ethyl-3-(3-dimethylaminopropyl)carbodiimide (38 mg) and the mixture was stirred at room temperature overnight. The reaction mixture was diluted with chloroform (2 mL) and thereto was added an aqueous saturated sodium hydrogencarbonate solution (3 mL). After stirring, the organic layer was separated and concentrated and the resultant crude product was purified by high performance liquid chromatography (HPLC) (Solvent; 10 mM ammonium carbonate/methanol = 80 : 20 → 5 : 95) to give 6-chloro-1-(3-ethoxybenzyl)-4-[4-[[trans-4-[[N-(2-methoxyethyl)-N-(tert-butyl)amino]methyl]cyclohexyl]carbonyl]piper azin-1-yl]-1H-pyrazolo[3,4-d]pyrimidine (51 mg, yield: 81 %) as an amorphous solid.
   MS(APCI)m/z; 626/628 [M+H]⁺

(2) To a suspension of the compound obtained in the above step (1) (51 mg) in water (200 µL) was added 2N HCl (41 µL) and the mixture was lyophilized to give 6-chloro-1-(3-ethoxybenzyl)-4-[4-[[trans-4-[[N-(2-methoxyethyl)-N-(tert-butyl)amino] methyl]cyclohexyl]carbonyl]piperazin-1-yl]-1H-pyrazolo[3,4-d]pyrimidine hydrochloride (47 mg, yield: 71 %) as an amorphous solid.
   MS(APCI)m/z; 626/628 [M+H]⁺

### Example A2

(1) To trans-4-(piperidin-1-yl)cyclohexanecarboxilic acid hydrochloride (30 mg; compound obtained in Reference Example A2) was added successively chloroform (1.0 mL), 6-chloro-1-(3-ethoxybenzyl)-4-piperazin-1-yl-1H-pyrazolo[3,4-d]pyrimidine dihydrochloride (38 mg), 1-hydroxybenzotriazole (21 mg), triethylamine (85 µL) and 1-ethyl-3-[3-(dimethylamino)propyl]carbodiimide hydrochloride (38 mg) and the mixture was stirred at room temperature overnight. The organic layer was separated and concentrated and the resultant crude product was purified by HPLC (Solvent; 10 mM ammonium carbonate/methanol = 80 : 20 → 5 : 95) to give 6-chloro-1-(3-ethoxybenzyl)-4-[4-[[trans-4-(piperidin-1-yl)cyclohexyl]carbonyl]piperazin-1-yl]-1H-pyrazolo[3,4-d]pyri midine (40 mg, yield: 71 %) as an amorphous solid. MS(APCI)m/z; 566/568 [M+H]⁺

(2) The compound obtained in the above step (1) (40 mg) was treated in the same manner as described in Example Al-(2) to give 6-chloro-1-(3-ethoxybenzyl)-4-[4-[[trans-4-(piperidin-1-yl)cyclohexyl]carbonyl]piperazin-1-yl]-1H-pyrazolo[3,4-d]pyrimidin e hydrochloride (40 mg, yield: 67 %) as an amorphous solid.
   MS(APCI)m/z; 566/568 [M+H]⁺

### Example A3

(1) Methyl 4-[[N-ethyl-N-(tert-butoxycarbonyl)amino]methyl]benzoate (36 mg, compound obtained in Reference Example A3) and 6-chloro-1-[(2-propyl-1,3-thiazol-4-yl)methyl]-4-(piperazin-1-yl)-1H-pyrazolo[3,4-d]pyrimidine dihydrochloride (38 mg; compound obtained in Reference Example A11) were treated in the same manner as described in Example A1 to give crude 6-chloro-1-[(2-propyl-1,3-thiazol-4-yl)methyl]-4-[4-[4-[[N-ethyl-N-(tert-butoxycarbonyl)amino]methyl]benzoyl]piperazin-1-yl]-1H-pyrazolo[3,4-d]pyrimidine as a crude product.

(2) To a suspension of the compound obtained in the above step (1) in methylenechloride (1.0 mL) was added trifluoroacetic acid (1.0 mL) and the mixture was stirred at room temperature for 1 hour. The reaction mixture was concentrated and the resultant crude product was purified by HPLC (Solvent; 10 mM ammonium carbonate/methanol = 80 : 20 → 5 : 95) to give 6-chloro-1-[(2-propyl-1,3-thiazol-4-yl)methyl]-4-[4-[4-(ethylaminomethyl)benzoyl]piperazin-1-yl]-1H-pyrazolo[3,4-d]pyrimidine (29 mg, yield: 54 %) as an amorphous solid.
   MS(APCI)m/z; 539/541 [M+H]⁺

(3) The compound obtained in the above step (2) (29 mg) was treated in the samemanner as described in Example A1-(2) to give 6-chloro-1-[(2-propyl-1,3- thiazol-4-yl)-methyl]-4-[4-[4-(ethylaminomethyl)benzoyl]piperazin-1-yl]-1H-pyrazolo[3,4-d]pyrimidine hydrochloride (29 mg, yield: 55 %) as an amorphous solid.
   MS(APCI)m/z; 539/541 [M+H]⁺

### Example A4

(1) Ethyl 4-[[2-(dimethylamino)ethyl]amino]benzoate (30 mg; compound obtained in Reference Example A4) was treated in the same manner as described in Example A1 to give crude 6-chloro-1-(3-ethoxybenzyl)-4-[4-[4-[2-(dimethylamino)ethylamino]benzoyl]-piperazin-1-yl]-1H-pyrazolo[3,4-d]pyrimidine.

(2) To a solution of the compound obtained in the above step (1) and pyridine (32mg) in chloroform was added acetyl chloride (16 mg) and the mixture was stirred at room temperature overnight. To the reaction mixture was added an aqueous saturated sodium hydrogencarbonate solution and the mixture was extracted with chloroform. The organic layer was concentrated and the resultant crude product was purified by HPLC (Solvent; 10 mM ammonium carbonate/methanol = 80 : 20 → 5 : 95) to give 6-chloro-1-(3-ethoxybenzyl)-4-[4-[4-N-acetyl-N-[2-(dimethylamino)ethyl]amino]benzoyl]piperazin-1-yl]-1H-pyrazolo[3,4-d]pyrimidine (35 mg, yield: 58 %) as an amorphous solid.
   MS(APCI)m/z; 605/607 [M+H]⁺

(3) The compound obtained in the above step (2) (35 mg) was treated in the samemanner as described in Example A1-(2) to give 6-chloro-1-(3-ethoxybenzyl)-4-[4-[4-N-acetyl-N-[2-(dimethylamino)ethyl]amino]benzoyl]piperazin-1-yl]-1H-pyrazolo[3,4-d]pyr imidine hydrochloride (35 mg, yield: 55 %) as an amorphous solid.
   MS(APCI)m/z; 605/607 [M+H]⁺

### Example A5

The corresponding starting material obtained in Reference Example A8 was treated in the same manner as described in Example A2 to give 1-(3-ethoxybenzyl)-3-methyl-4-[4-[[trans-4-(piperidin-1-yl)cyclohexyl]carbonyl]piperazin-1-yl]-1H-pyrazolo[3,4-d]pyri midine hydrochloride (46 mg, yield: 78 %) as an amorphous solid.
MS(APCI)m/z; 546 [M+H]⁺

### Example A6

(1) To 4-(4-carboxypiperidin-1-yl)-1-(3-ethoxybenzyl)-1H-pyrazolo[3,4-d] pyrimidine hydrochloride (50 mg; compound obtained in Reference Example A9-(3)) was added successively chloroform (1 mL), 4-piperidinopiperidine (26 mg), 1-hydroxybenzotriazole (24 mg), triethylamine (17 µL) and 1-ethyl-3-[3-(dimethylamino)-propyl]carbodiimide hydrochloride (35 mg) and the mixture was stirred at room temperature for 17 hours. The reaction mixture was diluted with chloroform (5 mL) and thereto was added an aqueous saturated sodium hydrogencarbonate solution (10 mL). After stirring, the organic layer was separated, dried over sodium sulfate and concentrated in vacuo. The resultant residue was purified by HPLC (Solvent; 10 mM ammonium carbonate/methanol = 80 : 20 → 5 : 95) to give 1-(3-ethoxybenzyl)-4-[4-[(4-piperidino-piperidin-1-yl)carbonyl]piperidin-1-yl]-1H-pyrazolo[3,4-d]pyrimidine (44 mg, yield: 69 %) as an amorphous solid.
   MS(APCI)m/z; 532 [M+H]⁺

(2) To a solution of the compound obtained in the above step (1) in ethanol was added 2N HCl (41 µL) and the mixture was concentrated in vacuo. A solution of the resultant residue in water (1 mL) was lyophilized to give 1-(3-ethoxybenzyl)-4-[4-[(4-piperidinopiperidin-1-yl)carbonyl]piperidin-1-yl]-1 H-pyrazolo[3,4-d]pyrimidine dihydrochloride (48 mg, yield: 66 %) as an amorphous solid.
   MS(APCI)m/z; 532 [M+H]⁺

### Example A7

The compound obtained in Reference Example A11 (34 mg) was treated in the same manner as described in Example A2 to give 1-(3-ethoxybenzyl)-4-[1-[[trans-4-(piperidin-1-yl)cyclohexyl]carbonyl]piperidin-4-yl]-1H-pyrazolo[3,4-d]pyrimidine hydrochloride (17 mg; yield: 50 %) as an amorphous solid.
MS(APCI)m/z; 531 [M+H]⁺

### Example A8

(1) The compound obtained in Reference Example A9 (4-(4-carboxy-piperidin-1-yl)-1-(3-ethoxybenzyl)-1H-pyrazolo[3,4-d]pyrimidine, 1.37 g) and 4-piperidone monohydrochloride monohydrate (755 mg) were treated in the same manner as described in Example A6-(1) to give 1-(3-ethoxybenzyl)-4-[4-[(4-oxopiperidin-1-yl)carbonyl]-piperazin-1-yl]-1H-pyrazolo[3,4-d]pyrimidine (1.52 g, yield: quantitative) as an amorphous solid.
   MS(APCI)m/z; 463 [M+H]⁺

(2) To a mixture of the compound obtained in the above step (1) (46 mg),N,N-dimethylethylenediamine (18 mg) and MP-cyanoborohydride (86 mg) in tetrahydrofuran (1 mL) was added acetic acid (15 µL) and the mixture was stirred at room temperature overnight. To the reaction mixture was added MP-isocyanate (100 mg) and the mixture was stirred at room temperature overnight. The reaction mixture was filtered and the filtrate was concentrated in vacuo. The resultant crude product was purified by HPLC (Solvent; 10 mM ammonium carbonate/methanol = 80 : 20 → 5 : 95) to give 4-[4-[[4-[(2-dimethylaminoethyl)amino]piperidin-1-yl]carbonyl]piperidin-1-yl]-1-(3-ethoxybenzyl)-1H-pyrazolo[3,4-d]pyrimidine as an amorphous solid.
   MS(APCI)m/z; 535 [M+H]⁺

(3) To a solution of the compound obtained in the above step (2) in dichloromethane (0.5 mL) was added a solution of acetyl chloride (14 µL) in dichloromethane (0.5 mL) and pyridine (20 µL) under ice-cooling and the mixture was stirred at room temperature overnight. To the reaction mixture was added an aqueous saturated sodium hydrogencarbonate solution (3 mL) and the mixture was vigorously stirred for 30 minutes. The organic layer was separated and the aqueous layer was extracted with chloroform (2 mL). The extract was concentrated in vacuo and the resultant residue was purified by HPLC (Solvent; 10 mM ammonium carbonate/methanol = 80 : 20 → 5 : 95) to give 4-[4-[[4-[N-acetyl-(2-dimethylaminoethyl)amino]piperidin-1-yl]-carbonyl]piperidin-1-yl]-1-(3-ethoxybenzyl)-1H-pyrazolo[3,4-d]pyrimidine (12 mg, Yield throughout the two steps: 21 %) as an amorphous solid.
   MS(APCI)m/z; 577 [M+H]⁺

(4) The compound obtained in the above step (3) (12 mg) was treated in the samemanner as described in Example A1-(2) to give 4-[4-[[4-[N-acetyl-(2-dimethylaminoethyl)-amino]piperidin-1-yl] carbonyl]piperidin-1-yl]-1-(3-ethoxybenzyl)-1H-pyrazolo[3,4-d]pyri midine hydrochloride (13 mg, yield : quantitative) as an amorphous solid.
   MS(APCI)m/z; 577 [M+H]⁺

### Examples A9 to A 114

The corresponding starting materials were treated in the same manner as described in either one of Examples A1 to A8 to give the compounds as shown in the following Tables 1 to 22.

**Table 1**

| | | | |
|---|---|---|---|
| 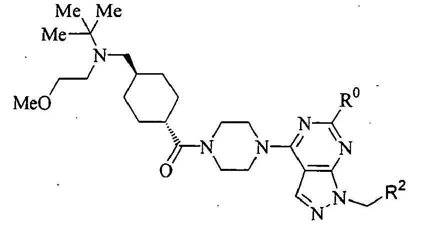 | | | |

| Ex. Nos. | R² | R⁰ | Physicochemical properties etc. |
|---|---|---|---|
| A9* | 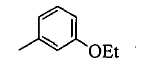 | MeO | amorphous solid MS(APCI)622[M+H]+ |
| A10* | 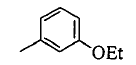 | Me | amorphous solid MS(APCI)606[M+H]+ |
| A11* | 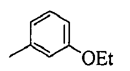 | 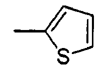 | amorphous solid MS(APCI)674[M+H]+ |
| A12* | 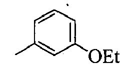 | CN | amorphous solid MS(APCI)617[M+H]+ |
| A13* | 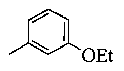 | NMe₂ | amorphous solid MS(APCI)635[M+H]+ |
| A14* | 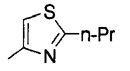 | Cl | amorphous solid MS(APCI)631/633[M+H]+ |
| A15* | 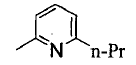 | Cl | amorphous solid MS(APCI)625/627[M+H]+ |
| A16* | 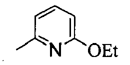 | EtO | amorphous solid MS(APCI)637[M+H]+ |

| | | | |
|---|---|---|---|
| *: hydrochloride Me: methyl group, Et: ethyl group, n-Pr: n-propyl group | | | |

**Table 2**

| | | | |
|---|---|---|---|
| 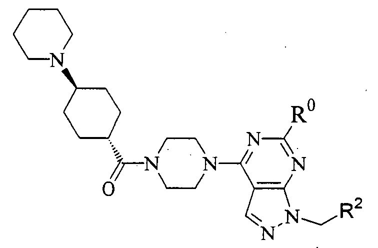 | | | |

| Ex. Nos. | R² | R⁰ | Physicochemical properties etc. |
|---|---|---|---|
| A17* | 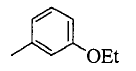 | MeO | amorphous solid MS(APCI)562[M+H]+ |
| A19* | 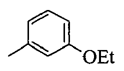 | 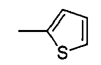 | amorphous solid MS(APCI)614[M+H]+ |
| A20* | 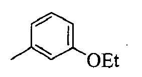 | CN | amorphous solid MS(APCI)557[M+H]+ |
| A21* | 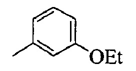 | NMe₂ | amorphous solid MS(APCI)575 [M+H]+ |
| A22* | 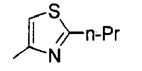 | Cl | amorphous solid MS(APCI)571/573[M+H]+ |
| A23* | 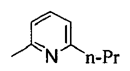 | Cl | amorphous solid MS(APCI)565/567[M+H]+ |
| A24* | 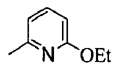 | EtO | amorphous solid MS(APCI)577[M+H]+ |

| | | | |
|---|---|---|---|
| *:hydrochloride Me: methyl group, Et: ethyl group, n-Pr: n-propyl group | | | |

**Table 3**

| | | | |
|---|---|---|---|
| 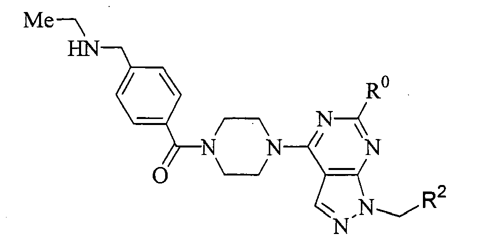 | | | |

| Ex. Nos. | R² | R⁰ | Physicochemical properties etc. |
|---|---|---|---|
| A26* | 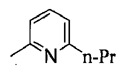 | C1 | amorphous solid MS(APCI)533/535[M+H]+ |
| A27* | 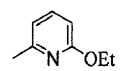 | EtO | amorphous solid MS(APCI)545[M+H]+ |

| | | | |
|---|---|---|---|
| *: hydrochloride Me: methyl group, Et: ethyl group, n-Pr: n-propyl group | | | |

**Table 4**

| | | | |
|---|---|---|---|
| 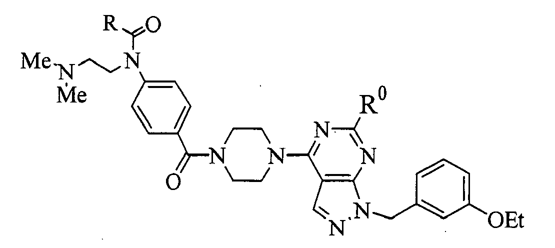 | | | |

| Ex. Nos. | R | R⁰ | Physicochemical properties etc.. |
|---|---|---|---|
| A28* | Me | NMe₂ | amorphous solid MS(APCI)614[M+H]⁺ |
| A29* | 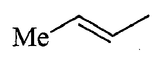 | Cl | amorphous solid MS(APCI)631/633[M+H]+ |

| | | | |
|---|---|---|---|
| *: hydrochloride Me: methyl group, Et: ethyl group | | | |

**Table 5**

| | | | |
|---|---|---|---|
| 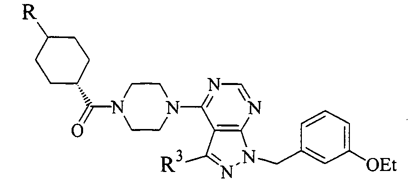 | | | |

| Ex. Nos. | R | R³ | Physicochemical properties etc.. |
|---|---|---|---|
| A30* | 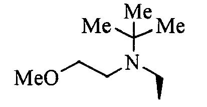 | Me | amorphous solid MS(APCI)606[M+H]+ |

| | | | |
|---|---|---|---|
| *: hydrochloride Me: methyl group, Et: ethyl group | | | |

**Table 6**

| | | |
|---|---|---|
| 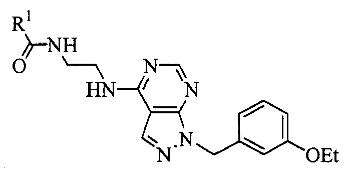 | | |

| Ex. Nos. | R¹ | Physicochemical properties etc. |
|---|---|---|
| A31* | 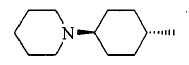 | amorphous solid MS(APCI)506[M+H]+ |
| A32* | 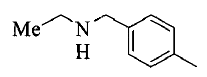 | amorphous solid MS(APCI)474[M+H]+ |
| A33* | 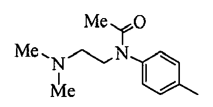 | amorphous solid MS(APCI)545[M+H]+ |
| A34* | 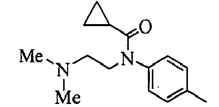 | amorphous solid MS(APCI)571 [M+H]+ |
| A35* | 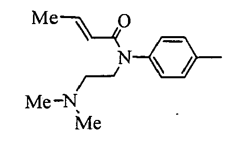 | amorphous solid MS(APCI)571[M+H]+ |

| | | |
|---|---|---|
| *: hydrochloride Me: methyl group, Et: ethyl group | | |

**Table 7**

| | | |
|---|---|---|
| 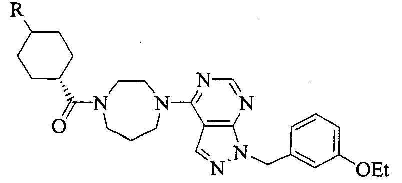 | | |

| Ex. Nos. | R¹ | Physicochemical properties etc. |
|---|---|---|
| A36* | 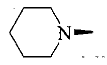 | amorphous solid MS(APCI)546[M+H]⁺ |
| A37* | 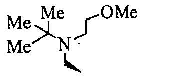 | amorphous solid MS(APCI)606[M+H]+ |

| | | |
|---|---|---|
| *: hydrochloride Me: methyl group, Et: ethyl group | | |

**Table 8**

| | | | | | |
|---|---|---|---|---|---|
| 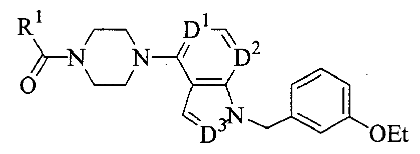 | | | | | |

| Ex. Nos. | R¹ | D¹ | D² | D³ | Physicochemical properties etc. |
|---|---|---|---|---|---|
| A38* | 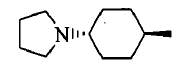 | N | N | CH | amorphous solid MS(APCI)517[M+H]+ |
| A39* | 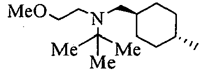 | N | N | CH | amorphous solid MS(APCI)591[M+H]+ |
| A40* | 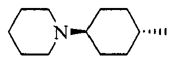 | N | N | CH | amorphous solid MS(APCI)531[M+H]+ |
| A41* | 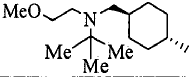 | CH | N | N | amorphous solid MS(APCI)591[M+H]+ |
| A42* | 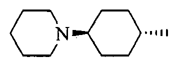 | N | CH | N | amorphous solid MS(APCI)531[M+H]+ |
| A43* | 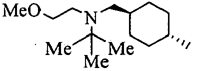 | N | CH | N | amorphous solid MS(APCI)591[M+H]+ |

| | | | | | |
|---|---|---|---|---|---|
| *: hydrochloride, Me: methyl group, Et: ethyl group, t-Bu: tert-butyl group | | | | | |

**Table 9**

| | | |
|---|---|---|
| 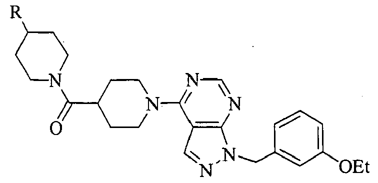 | | |

| Ex. Nos. | R | Physicochemical properties etc. |
|---|---|---|
| A44** | 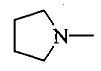 | amorphous solid MS(APCI)518 [M+H]+ |
| A45** | 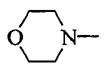 | amorphous solid MS(APCI)534 [M+H]+ |

| | | |
|---|---|---|
| **: dihydrochloride Et: ethyl group | | |

**Table 10**

| | | |
|---|---|---|
| 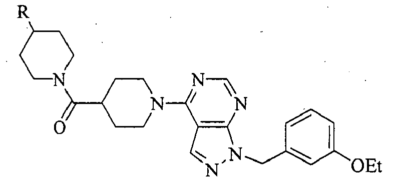 | | |

| Ex. Nos. | R | Physicochemical properties etc. |
|---|---|---|
| A46* | 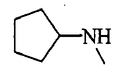 | amorphous solid MS(APCI)532[M+H]+ |
| A47* | 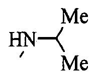 | amorphous solid MS(APCI)506[M+H]+ |
| A48* | 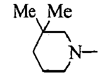 | amorphous solid MS(APCI)560[M+H]+ |
| A49* | 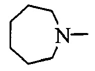 | amorphous solid MS(APCI)546[M+H]+ |
| A50* | 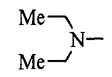 | amorphous solid MS(APCI)520[M+H]+ |
| A51* | 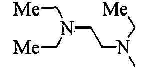 | amorphous solid MS(APCI)591 [M+H]+ |
| A52* | 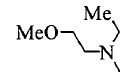 | amorphous solid MS(APCI)550[M+H]+ |
| A53* | 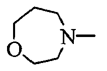 | amorphous solid MS(APCI)548[M+H]+ |
| A54* | 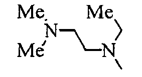 | amorphous solid MS(APCI)563[M+H]+ |

| | | |
|---|---|---|
| *: hydrochloride, Me: methyl group, Et: ethyl group | | |

**Table 11**

| | | |
|---|---|---|
| 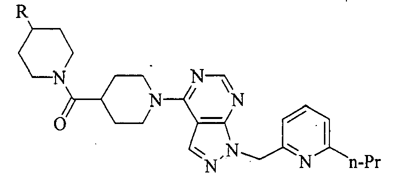 | | |

| Ex. Nos. | R | Physicochemical properties etc. |
|---|---|---|
| A55** | 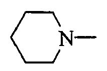 | amorphous solid MS(APCI)534 [M+H]+ |
| A56** | 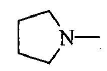 | amorphous solid MS(APCI)517 [M+H]+ |
| A57* | 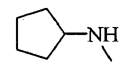 | amorphous solid MS(APCI)531 [M+H]+ |
| A58* | 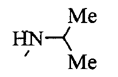 | amorphous solid MS(APCI)505[M+H]+ |
| A59* | 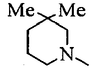 | amorphous solid MS(APCI)559[M+H]+ |
| A60* | 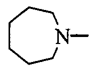 | amorphous solid MS(APCI)545 [M+H]+ |
| A61* | 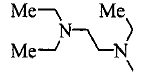 | amorphous solid MS(APCI)590[M+H]+ |
| A62* | 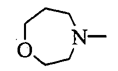 | amorphous solid MS(APCI)547[M+H]+ |

| | | |
|---|---|---|
| *: hydrochloride, **: dihydrochloride, Me: methyl group, n-Pr: n-propyl group | | |

**Table 12**

| | | | |
|---|---|---|---|
| 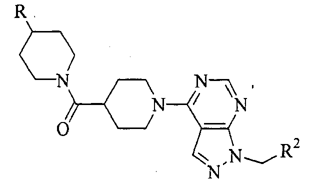 | | | |

| Ex. Nos. | R | R² | Physicochemical properties etc. |
|---|---|---|---|
| A63* | 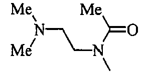 | 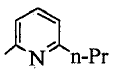 | amorphous solid MS(APCI)576[M+H]+ |
| A65* | 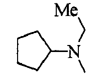 | 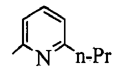 | amorphous solid MS(APCI)559[M+H]+ |
| A66* | 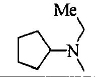 | 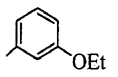 | amorphous solid MS(APCI)560[M+H]+ |
| A67* | 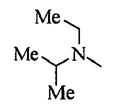 | 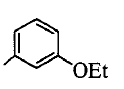 | amorphous solid MS(APCI)534[M+H]+ |

| | | | |
|---|---|---|---|
| *: hydrochloride, Me: methyl group, Et: ethyl group, n-Pr: n-propyl group | | | |

**Table 13**

| | | |
|---|---|---|
| 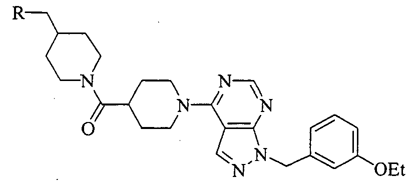 | | |

| Ex. Nos. | R | Physicochemical properties etc. |
|---|---|---|
| A68* | 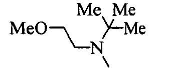 | amorphous solid MS(APCI)592[M+H]+ |
| A69* | 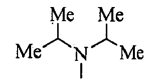 | amorphous solid MS(APCI)562[M+H]+ |
| A70* | 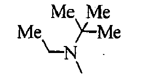 | amorphous solid MS(APCI)562[M+H]+ |

| | | |
|---|---|---|
| *: hydrochloride, Me: methyl group, Et: ethyl group | | |

**Table 14**

| | | |
|---|---|---|
| 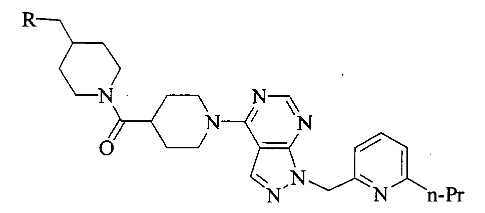 | | |

| Ex. Nos. | R | Physicochemical properties etc. |
|---|---|---|
| A71* | 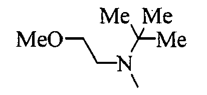 | amorphous solid MS(APCI)591 [M+H]+ |
| A72* | 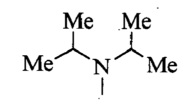 | amorphous solid MS(APCI)561[M+H]+ |
| A73* | 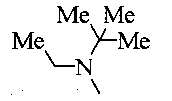 | amorphous solid MS(APCI)561[M+H]+ |

| | | |
|---|---|---|
| *: hydrochloride, Me: methyl group, n-Pr: n-propyl group | | |

**Table 15**

| | | |
|---|---|---|
| 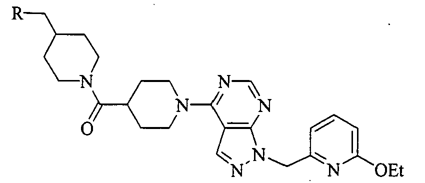 | | |

| Ex. Nos. | R | Physicochemical properties etc. |
|---|---|---|
| A74* | 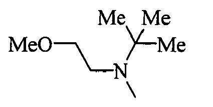 | amorphous solid MS(APCI)593 [M+H]+ |
| A75* | 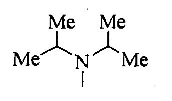 | amorphous solid MS(APCI)563[M+H]+ |
| A76* | 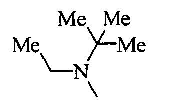 | amorphous solid MS(APCI)563[M+H]+ |
| A77* | 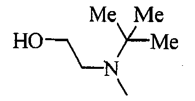 | amorphous solid MS(APCI)579[M+H]+ |
| A78* | 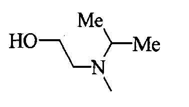 | amorphous solid MS(APCI)565[M+H]+ |

| | | |
|---|---|---|
| *: hydrochloride, Me: methyl group, Et: ethyl group | | |

**Table 16**

| | | |
|---|---|---|
| 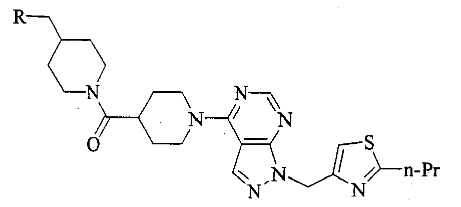 | | |

| Ex. Nos. | R | Physicochemical properties etc. |
|---|---|---|
| A79* | 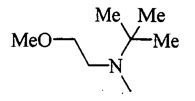 | amorphous solid MS(APCI)597[M+H]+ |
| A80* | 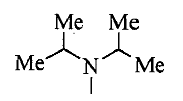 | amorphous solid MS(APCI)567[M+H]+ |
| A81* | 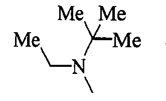 | amorphous solid MS(APCI)567[M+H]+ |
| A82* | 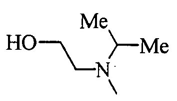 | amorphous solid MS(APCI)569[M+H]+ |

| | | |
|---|---|---|
| *: hydrochloride, Me: methyl group, n-Pr: n-propyl group | | |

**Table 17**

| | | | |
|---|---|---|---|
| 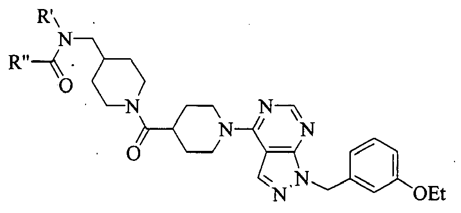 | | | |

| Ex. Nos. | R' | R" | Physicochemical properties etc. |
|---|---|---|---|
| A83* | 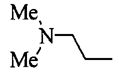 | Me | amorphous solid MS(APCI)591[M+H]+ |
| A84* | 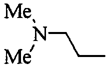 | 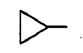 | amorphous solid MS(APCI)617[M+H]+ |
| A85* | 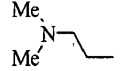 | t-Bu | amorphous solid MS(APCI)633 [M+H]+ |
| A86* | 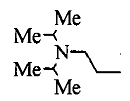 | Me | amorphous solid MS(APCI)647[M+H]+ |
| A87* | 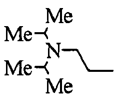 | 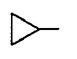 | amorphous solid MS(APCI)673[M+H]+ |
| A88* | 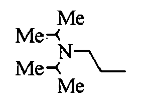 | t-Bu | amorphous solid MS(APCI)689[M+H]+ |
| A89* | 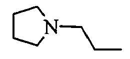 | Me | amorphous solid MS(APCI)617[M+H]+ |
| A90* | 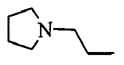 | 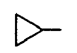 | amorphous solid MS(APCI)643[M+H]+ |
| A91* | 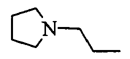 | t-Bu | amorphous solid MS(APCI)659[M+H]+ |

| | | | |
|---|---|---|---|
| *: hydrochloride Me: methyl group, Et: ethyl group, t-Bu: tert-butyl group | | | |

**Table 18**

| | | | |
|---|---|---|---|
| 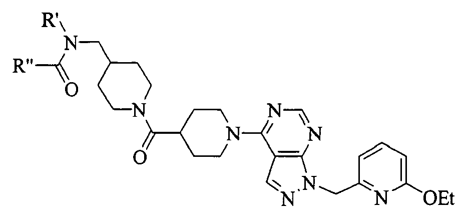 | | | |

| Ex. Nos. | R' | R" | Physicochemical properties etc: |
|---|---|---|---|
| A92* | 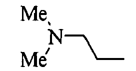 | Me | amorphous solid MS(APCI)592[M+H]+ |
| A93 * | 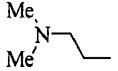 | 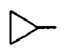 | amorphous solid MS(APCI)618[M+H]+ |
| A94* | 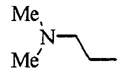 | t-Bu | amorphous solid MS(APCI)634[M+H]+ |
| A95 * | 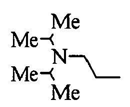 | Me | amorphous solid MS(APCI)648 [M+H]+ |
| A96* | 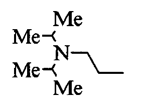 | 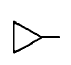 | amorphous solid MS(APCI)674[M+H]+ |
| A97* | 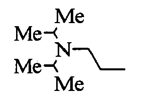 | t-Bu | amorphous solid MS(APCI)690[M+H]+ |
| A98* | 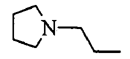 | Me | amorphous solid MS(APCI)618[M+H]+ |
| A99* | 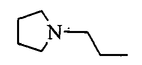 | 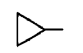 | amorphous solid MS(APCI)644[M+H]+ |
| A100* | 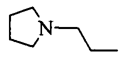 | t-Bu | amorphous solid MS(APCI)660[M+H]+ |

| | | | |
|---|---|---|---|
| *: hydrochloride, Me: methyl group, Et: ethyl group, t-Bu: tert-butyl group | | | |

**Table 19**

| | | | |
|---|---|---|---|
| 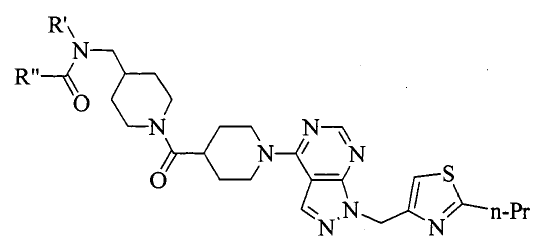 | | | |

| Ex. Nos. | R' | R" | Physicochemical properties etc. |
|---|---|---|---|
| A101* | 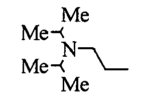 | 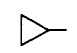 | amorphous solid MS(APCI)678[M+H]+ |
| A102* | 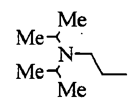 | t-Bu | amorphous solid MS(APCI)694[M+H]+ |
| A103* | 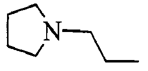 | Me | amorphous solid MS(APCI)622[M+H]+ |
| A104* | 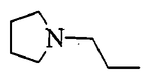 | 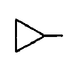 | amorphous solid MS(APCI)648[M+H]+ |
| A105* | 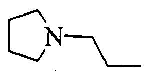 | t-Bu | amorphous solid MS(APCI)664[M+H]+ |

| | | | |
|---|---|---|---|
| *: hydrochloride Me: methyl group, n-Pr: n-propyl group, t-Bu: tert-butyl group | | | |

**Table 20**

| | | | |
|---|---|---|---|
| 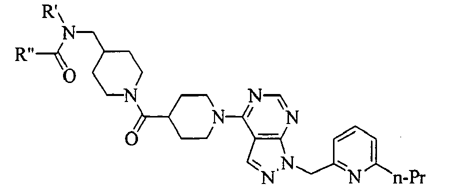 | | | |

| Ex. Nos. | R' | R" | Physicochemical properties etc. |
|---|---|---|---|
| A106* | 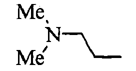 | Me | amorphous solid MS(APCI)590[M+H]+ |
| A107* | 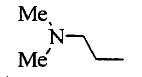 | 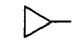 | amorphous solid MS(APCI)616[M+H]+ |
| A108* | 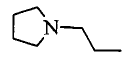 | Me | amorphous solid MS(APCI)616[M+H]+ |
| A109* | 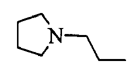 | 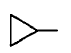 | amorphous solid MS(APCI)642[M+H]+ |
| A110* | 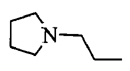 | t-Bu | amorphous solid MS(APCI)658[M+H]+ |

| | | | |
|---|---|---|---|
| *: hydrochloride Me: methyl group, n-Pr: n-propyl group, t-Bu: tert-butyl group | | | |

**Table 21**

| | | |
|---|---|---|
| 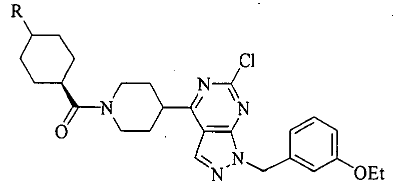 | | |

| Ex. Nos. | R | Physicochemical properties etc. |
|---|---|---|
| A111* | 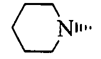 | amorphous solid MS(APCI)565/567[M+H]+ |
| A112* | 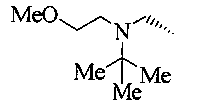 | amorphous solid MS(APCI)625/627[M+H]+ |

| | | |
|---|---|---|
| *: hydrochloride, Me: methyl group, Et: ethyl group | | |

**Table 22**

| | | |
|---|---|---|
| 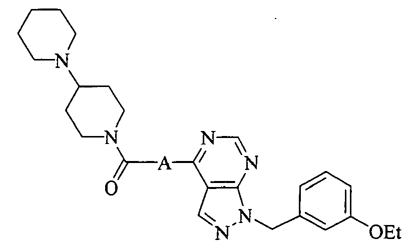 | | |

| Ex. Nos. | A | Physicochemical properties etc. |
|---|---|---|
| A113** | 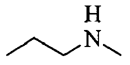 | amorphous solid MS(APCI)492[M+H]+ |
| A114** | 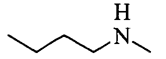 | amorphous solid MS(APCI)506[M+H]+ |

| | | |
|---|---|---|
| **: dihydrochloride, Et: ethyl group | | |

### Example A115

To a solution of the compound obtained in Reference Example A13-(2) (54 mg) in N,N-dimethylformamide (1.0 mL) was added a 60 % oily dispersion of sodium hydride (15 mg) at 0 °C. After stirring the mixture for 0.5 hour, thereto was added dropwise a solution of the compound obtained in Reference Example A12-(1) (42 mg) in tetrahydrofuran (1.0 mL) and the mixture was stirred at room temperature overnight. To the reaction mixture was added water and the mixture was extracted with chloroform. The extract was dried over magnesium sulfate and concentrated. The resultant crude product was purified by HPLC (Solvent; 10 mM ammonium carbonate/methanol = 80 : 20 → 5 : 95) and treated with hydrochloric acid to give 1-(3-ethoxybenzyl)-4-[4-[[4-[[N-(2-methoxyethyl)-N-(tert-butyl)amino]methyl]cyclohexyl]carbonyl]piperazin-1-yl]-1H-pyrazolo [3,4-b]pyridine hydrochloride (10 mg, yield: 15 %) as an amorphous solid.
MS(APCI)m/z; 591 [M+H]⁺

### Example A116

The corresponding starting materials were treated in the same manner as described in Example A2 and A8(2) to give 1-(3-ethoxybenzyl)-6-methoxy-4-[4-[4-N-acetyl-N-[2-(dimethylaminoethyl)amino]benzoyl]piperazin-1-yl]-1H-pyrazolo [3,4-d]pyrimidine hydrochloride (37 mg, yield: 59 %) as an amorphous powder.
MS(APCI)m/z; 601 [M+H]⁺

### Example B1

(1) To a solution of methyl trans-4-[[N-(2-methoxyethyl)-N-(tert-butyl)-amino]methyl]cyclohexanecarboxylate (43 mg, a compound obtained in Reference Example A1) in ethanol (1 mL) was added 2N sodium hydroxide solution (100 µL) and the mixture was stirred at 60 °C for 2 hours. After cooling to room temperature, to the reaction mixture was added 5N HCl (50 µL) and the mixture was concentrated. To the residue was added successively chloroform (1 mL), 1-(6-dimethylaminopyridin-2-yl)-methyl-4-piperazin-1-yl-1H-pyrazolo[3,4-d] pyrimidine trihydrochloride (34 mg), 0.5 M solution of 1-hydroxybenzotriazole in dimethylformamide (0.3 mL), triethylamine (84 µL) and 0.5 M solution of 1-ethyl-3-(3-dimethylaminopropyl)carbodiimide hydrochloride in chloroform (0.4 mL), and the mixture was stirred at room temperature for 12 hours. The reaction mixture was diluted with chloroform (2 mL) and an aqueous saturated sodium hydrogencarbonate solution (3 mL) was added thereto. After stirring, the organic layer was separated and concentrated. The resultant crude product was purified by HPLC (XTerra PrepMS C₁₈ column; Waters Ltd., Solvent: 10 mM ammonium carbonate/methanol = 1 : 1 → 5 : 95) to give 1-[(6-dimethylaminopyridin-2-yl)methyl]-4-[4-[trans-4-[[N-(2-methoxyethyl)-N-(tert-butyl)amino]methyl]cyclohexyl]carbonyl]piperazin-1-yl]-1H-pyrazo lo[3,4-d] pyrimidine.

(2) To a solution of the compound obtained in the above step (1) in ethanol (1 mL) was added 1N hydrochloric acid and the mixture was concentrated. The residue was dissolved in water and lyophilized to give 1-[(6-dimethylaminopyridin-2-yl)methyl]-4-[4-[trans-4-[[N-(2-methoxyethyl)-N-(tert-butyl)amino]methyl]cyclohexyl]c arbonyl]piperazin-1-yl]-1H-pyrazolo[3,4-d]pyrimidin hydrochloride (40 mg, yield: 64 %) as an amorphous solid. MS(APCI)m/z; 592[M+H]⁺

### Example B2

(1) To trans-4-(piperidin-1-yl)cyclohexanecarboxylate hydrochloride (30 mg, the compound obtained in Reference Example A2) was added successively chloroform (1 mL), 1-[(6-dimethylaminopyridin-2-yl)methyl]-4-piperazin-1-yl-1H-pyrazolo[3,4-d]pyrimidine trihydrochloride (34 mg), 0.5M solution of 1-hydroxybenzotriazole in dimethylformamide (0.3 mL), triethylamine (84 µL) and 0.5 M solution of 1-ethyl-3-(3-dimethylaminopropyl)carbodiimide hydrochloride in chloroform (0.4 mL), and the mixture was stirred at room temperature for 12 hours. The reaction mixture was diluted with chloroform (2 mL) and an aqueous saturated sodium hydrogencarbonate solution (1.5 mL) and water (1.5 mL) were added thereto. After stirring, the organic layer was separated and concentrated. The resultant crude product was purified by HPLC (XTerra PrepMS C₁₈ column; Waters Ltd., Solvent: 10 mM ammonium carbonate/methanol = 1 : 1 → 5 : 95) to give 1-[(6-dimethylaminopyridin-2-yl)methyl]-4-[4-[trans-4-(piperidin-1-yl)cyclohexyl]carbony 1]piperazin-1-yl]-1H-pyrazolo[3,4-d] pyrimidine.

(2) The product obtained in the above step (1) was treated in the same manner asdescribed in Example B1(2) to give 1-[(6-dimethylaminopyridin-2-yl)methyl]-4-[4-[[trans-4-(piperidin-1-yl)cyclohexyl]carbonyl]piperazin-1-yl]-1H-pyrazolo[3,4-d]pyrimidine hydrochloride (43 mg, yield: 76 %) as an amorphous solid.
   MS(APCI)m/z; 532[M+H]⁺

### Example B3

(1) To a solution of 1-(3-ethoxybenzyl)-4-piperazin-1-yl-1H-pyrazolo [3,4-d]pyrimidine dihydrochloride (60 mg) in methylene chloride (1.2 mL) was added N,N-diisopropylethylamine (85 µL) and p-nitrophenyl chloroformate (49 mg) at 0 °C and the mixture was stirred at room temperature for 2.5 hours. The reaction mixture was diluted with chloroform (1 mL) and an aqueous saturated sodium hydrogen carbonate solution (1.5 mL) was added thereto. After stirring, the organic layer was separated and the aqueous layer was extracted with chloroform. The combined organic layer was dried over anhydrous sodium sulfate and filtered. The filtrate was concentrated and the residue was purified by flash column chromatography on silica gel (solvent; n-hexane/ethyl acetate = 9 : 1 → 0 : 10) to give 1-(3-ethoxybenzyl)-4-[4-[(4-nitrophenoxy)carbonyl]-piperazin-1-yl]-1H-pyrazolo[3,4-d]pyrimidine (72 mg, yield: 100 %) as an amorphous solid.
   MS(APCI)m/z; 504 [M+H]⁺

(2) To a solution of the compound obtained in the above step (1) (35 mg) in N,N-dimethylacetamide (0.4 mL) was added N,N-diisopropylethylamine (61 µL) and 0.5 M solution of 4-[[N-(2-methoxyethyl)-N-(tert-butyl)amino]methyl]piperidine obtained in Reference Example A1(4) in N,N-dimethylacetamide (0.42 mL) at room temperature and the mixture was stirred at 85 °C for 2 days. After cooling to room temperature, the reaction mixture was diluted with chloroform (2 mL) and thereto was added an aqueous saturated sodium hydrogencarbonate solution (1.5 mL) and water (1.5 mL). After stirring, the organic layer was separated and concentrated and the resultant crude product was purified by HPLC (XTerra PrepMS C₁₈ column; Waters Ltd., Solvent: 10 mM ammonium carbonate/methanol = 1 : 1 → 5 : 95) to give 1-(3-ethoxybenzyl)-4-[4-[[4-[[N-(2-methoxyethyl)-N-(tert-butyl)amino]methyl]piperidin-1-yl]carbonyl]piperazin-1-yl]-1H-p yrazolo[3,4-d]pyrimidine.

(3) The product obtained in the above step (2) was treated in the same manner asdescribed in Example B1(2) to give 1-(3-ethoxybenzyl)-4-[4-[[4-[[N-(2-methoxyethyl)-N-(tert-butyl)amino]methyl]piperidin-1-yl]carbonyl]piperazin-1-yl]-1H-pyrazolo[3,4-d] pyrimidine hydrochloride (30 mg, yield: 73 %) as an amorphous solid.
   MS(APCI)m/z; 593 [M+H]⁺

### Examples B4 to B62

The corresponding starting materials are treated in the same manner as described in each one of Examples B1 to B3 to give a compound as shown in the following Tables 23 to 38.

**Table 23**

| | | |
|---|---|---|
| 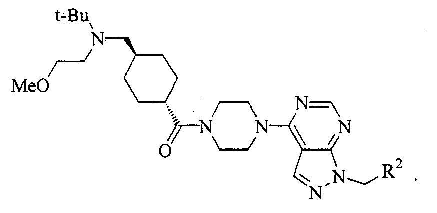 | | |

| Example Nos. | R² | Physicochemical properties etc. |
|---|---|---|
| B4* | 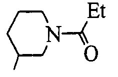 | amorphous solid MS(APCI)611[M+H]+ |
| B5* | 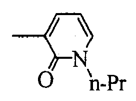 | amorphous solid MS(APCI)607[M+H]+ |
| B6* | 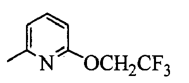 | amorphous solid MS(APCI)647[M+H]+ |

| | | |
|---|---|---|
| :*: hydrochloride, Me: methyl group, Et: ethyl group, n-Pr: n-propyl group t-Bu: tert-butyl group | | |

**Table 24**

| | | |
|---|---|---|
| 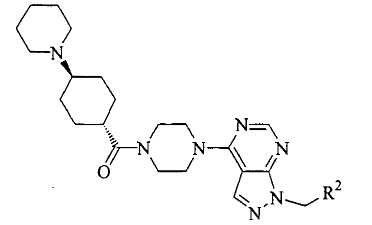 | | |

| Example Nos. | R² | Physicochemical properties etc. |
|---|---|---|
| B7 | 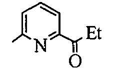 | amorphous solid MS(APCI)545[M+H]+ |
| B8 | 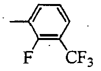 | amorphous solid MS(APCI)574[M+H]+ |
| B9* | 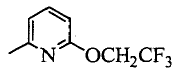 | amorphous solid MS(APCI)587[M+H]+ |

| | | |
|---|---|---|
| *: hydrochloride, Et: ethyl group | | |

**Table 25**

| | | |
|---|---|---|
| 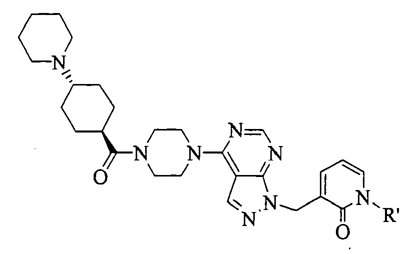 | | |

| Example Nos. | R' | Physicochemical properties etc. |
|---|---|---|
| B10* | -CH(CH₃)₂ | amorphous solid MS(APCI)547[M+H]+ |
| B11* | -C₂H₅ -C₂H₅ | amorphous solid MS(APCI)533[M+H]+ |
| B12* | -CH₃ -CH₃ | amorphous solid MS(APCI)519[M+H]+ |

| | | |
|---|---|---|
| *: hydrochloride | | |

**Table 26**

| | | |
|---|---|---|
| 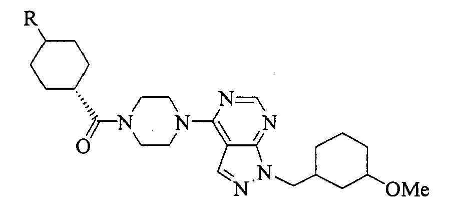 | | |

| Example Nos. | R | Physicochemical properties etc. |
|---|---|---|
| B13* | 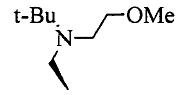 | amorphous solid MS(APCI)584[M+H]+ |
| B14* | 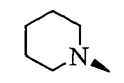 | amorphous solid MS(APCI)524[M+H]+ |

| | | |
|---|---|---|
| *: hydrochloride Me: methyl group, t-Bu: tert-butyl group | | |

**Table 27**

| | | |
|---|---|---|
| 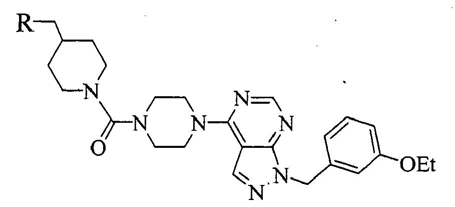 | | |

| Example Nos. | R | Physicochemical properties etc. |
|---|---|---|
| B15* | 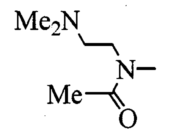 | amorphous solid MS(APCI)597[M+H]+ |
| B16* | 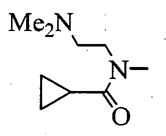 | amorphous solid MS(APCI)618[M+H]+ |
| B17* | 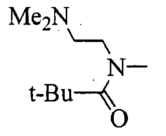 | amorphous solid MS(APCI)634[M+H]+ |
| B18* | 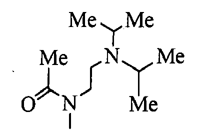 | amorphous solid MS(APCI)648[M+H]+ |
| B19* | 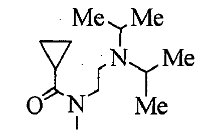 | amorphous solid MS(APCI)674[M+H]+ |

| | | |
|---|---|---|
| *: hydrochloride Me: methyl group, Et: ethyl group, t-Bu: tert-butyl group | | |

**Table 28**

| | | |
|---|---|---|
| 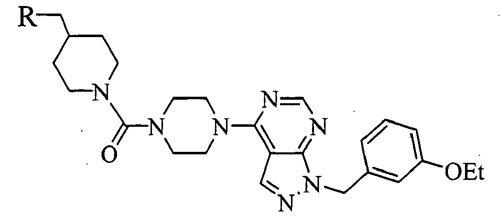 | | |

| Example Nos. | R | Physicochemical properties etc. |
|---|---|---|
| B20* | 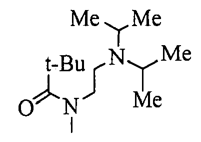 | amorphous solid MS(APCI)690[M+H]+ |
| B21* | 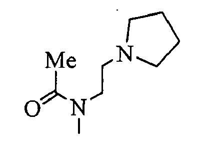 | amorphous solid MS(APCI)618[M+H]+ |
| B22* | 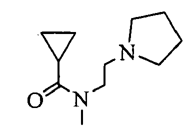 | amorphous solid MS(APCI)644[M+H]+ |
| B23* | 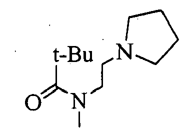 | amorphous solid MS(APCI)660[M+H]+ |

| | | |
|---|---|---|
| *: hydrochloride Me: methyl group, Et: ethyl group, t-Bu: tert-butyl group | | |

**Table 29**

| | | |
|---|---|---|
| 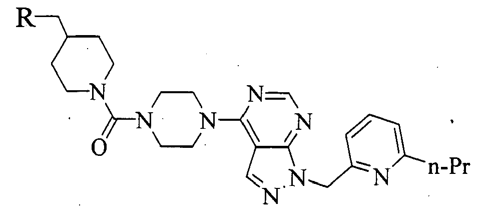 | | |

| Example Nos. | R | Physicochemical properties etc. |
|---|---|---|
| B24* | 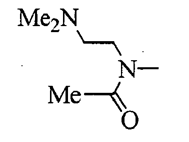 | amorphous solid MS(APCI)591[M+H]+ |
| B25* | 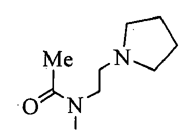 | amorphous solid MS(APCI)617[M+H]+ |
| B26* | 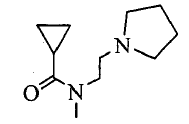 | amorphous solid MS(APCI)643[M+H]+ |
| B27* | 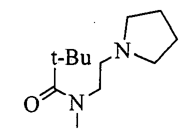 | amorphous solid MS(APCI)659[M+H]+ |

| | | |
|---|---|---|
| *: hydrochloride Me: methyl group, n-Pr: n-propyl group, t-Bu: tert-butyl group | | |

**Table 30**

| | | |
|---|---|---|
| 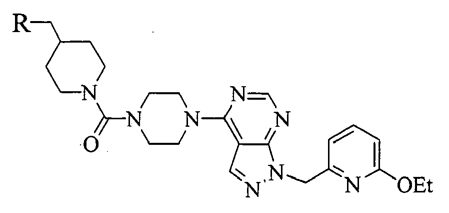 | | |

| Example Nos. | R | Physicochemical properties etc. |
|---|---|---|
| B28* | 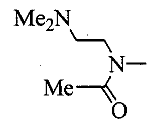 | amorphous solid MS(APCI)593 [M+H]+ |
| B29* | 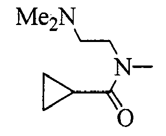 | amorphous solid MS(APCI)619[M+H]+ |
| B30* | 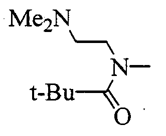 | amorphous solid MS(APCI)635[M+H]+ |
| B31* | 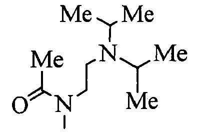 | amorphous solid MS(APCI)649[M+H]+ |
| B32* | 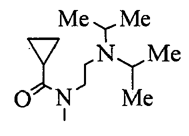 | amorphous solid MS(APCI)675[M+H]+ |

| | | |
|---|---|---|
| *: hydrochloride, Me: methyl group, Et: ethyl group, t-Bu: tert-butyl group | | |

**Table 31**

| | | |
|---|---|---|
| 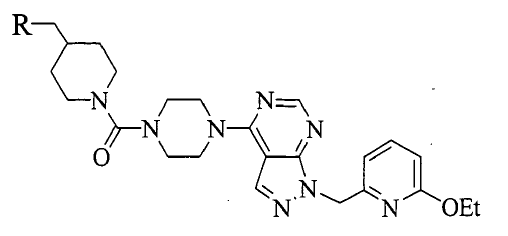 | | |

| Example Nos. | R | Physicochemical properties etc. |
|---|---|---|
| B33* | 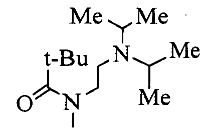 | amorphous solid MS(APCI)691[M+H]+ |
| B34* | 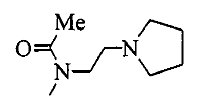 | amorphous solid MS(APCI)619[M+H]+ |
| B35* | 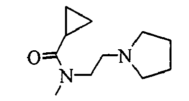 | amorphous solid MS(APCI)645[M+H]+ |
| B36* | 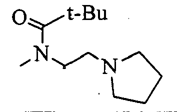 | amorphous solid MS(APCI)661[M+H]+ |

| | | |
|---|---|---|
| *: hydrochloride Me: methyl group, Et: ethyl group, t-Bu: tert-butyl group | | |

**Table 32**

| | | |
|---|---|---|
| 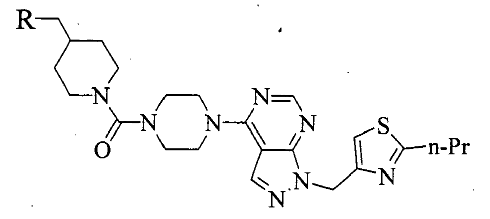 | | |

| Example Nos. | R | Physicochemical properties etc. |
|---|---|---|
| B37* | 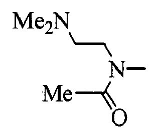 | amorphous solid MS(APCI)597[M+H]+ |
| B38* | 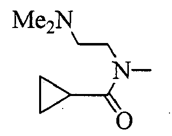 | amorphous solid MS(APCI)623[M+H]+ |
| B39* | 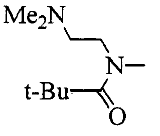 | amorphous solid MS(APCI)639[M+H]+ |
| B40* | 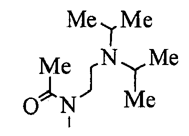 | amorphous solid MS(APCI)653[M+H]+ |
| B41* | 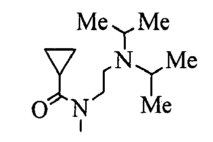 | amorphous solid MS(APCI)679[M+H]+ |

| | | |
|---|---|---|
| * : hydrochloride Me: methyl group, n-Pr: n-propyl group, t-Bu: tert-butyl group | | |

**Table 33**

| | | |
|---|---|---|
| 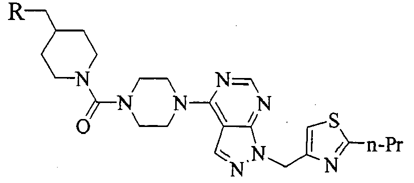 | | |

| Example Nos. | R | Physicochemical properties etc. |
|---|---|---|
| B42* | 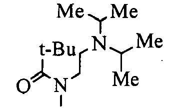 | amorphous solid MS(APCI)695[M+H]+ |
| B43* | 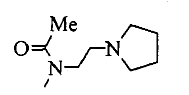 | amorphous solid MS(APCI)623[M+H]+ |
| B44* | 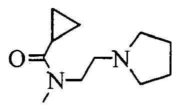 | amorphous solid MS(APCI)649[M+H]+ |
| B45* | 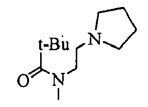 | amorphous solid MS(APCI)665[M+H]+ |

| | | |
|---|---|---|
| *: hydrochloride Me: methyl group, n-Pr: n-propyl group, t-Bu: tert-butyl group | | |

**Table 34**

| | | | |
|---|---|---|---|
| 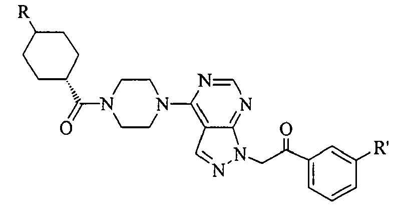 | | | |

| Example Nos. | R | R' | Physicochemical properties etc. |
|---|---|---|---|
| B46* | 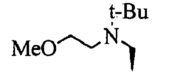 | MeO | amorphous solid MS(APCI)606[M+H]+ |
| B47* | 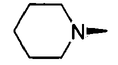 | MeO | amorphous solid MS(APCI)546[M+H]+ |

| | | | |
|---|---|---|---|
| *: hydrochloride, Me: methyl group, t-Bu: tert-butyl group | | | |

**Table 35**

| | | |
|---|---|---|
| 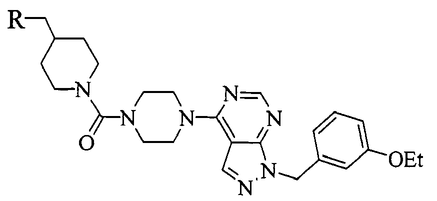 | | |

| Example Nos. | R | Physicochemical properties etc. |
|---|---|---|
| B48* | 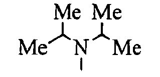 | amorphous solid MS(APCI)563 [M+H]+ |
| B49* | 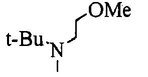 | amorphous solid MS(APCI)593 [M+H]+ |
| B50* | 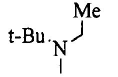 | amorphous solid MS(APCI)563 [M+H]+ |

| | | |
|---|---|---|
| *: hydrochloride, Me: methyl group, Et: ethyl group, t-Bu: tert-butyl group | | |

**Table 36**

| | | |
|---|---|---|
| 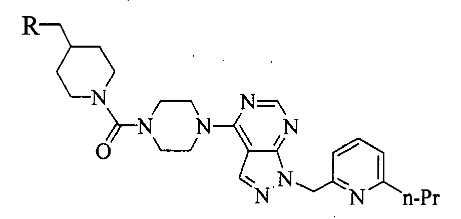 | | |

| Example Nos. | R | Physicochemical properties etc. |
|---|---|---|
| B51* | 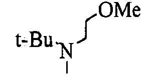 | amorphous solid MS(APCI)592 [M+H]+ |
| B52* | 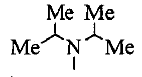 | amorphous solid MS(APCI)562 [M+H]+ |
| B53* | 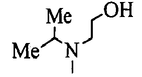 | amorphous solid MS(APCI)564 [M+H]+ |
| B54* | 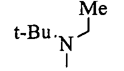 | amorphous solid MS(APCI)562 [M+H]+ |

| | | |
|---|---|---|
| * : hydrochloride Me: methyl group, n-Pr: n-propyl group, t-Bu: tert-butyl group | | |

**Table 37**

| | | |
|---|---|---|
| 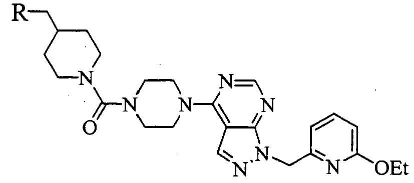 | | |

| Example Nos. | R | Physicochemical properties etc. |
|---|---|---|
| B55* | 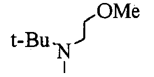 | amorphous solid MS(APCI)594 [M+H]+ |
| B56* | 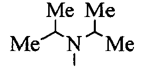 | amorphous solid MS(APCI) 564 [M+H]+ |
| B57* | 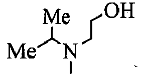 | amorphous solid MS(APCI)566 [M+H]+ |
| B58* | 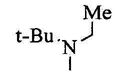 | amorphous solid MS(APCI)564 [M+H]+ |
| B59* | 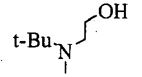 | amorphous solid MS(APCI)580 [M+H]+ |

| | | |
|---|---|---|
| *: hydrochloride Me: methyl group, Et: ethyl group, t-Bu: tert-butyl group | | |

**Table 38**

| | | |
|---|---|---|
| 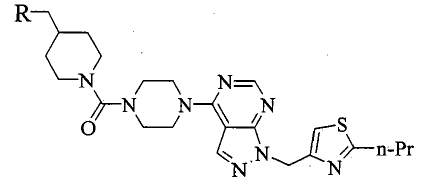 | | |

| Example Nos. | R | Physicochemical properties etc. |
|---|---|---|
| B60* | 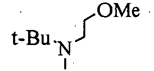 | amorphous solid MS(APCI)598 [M+H]+ |
| B61* | 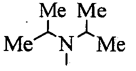 | amorphous solid MS(APCI)568 [M+H]+ |
| B62* | 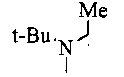 | amorphous solid MS(APCI)568 [M+H]+ |

| | | |
|---|---|---|
| *: hydrochloride, Me: methyl group, n-Pr: n-propyl group | | |

### Reference Example A1

(1) To methanol (1500 mL) was added dropwise thionyl chloride (254 mL) at -30 °C over a period of 1 hour. After stirring the mixture at room temperature for 30 minutes, thereto was added trans-cyclohexane-1,4-dicarboxylic acid (500.0 g) and the mixture was stirred at room temperature for 17 hours. The reaction mixture was concentrated in vacuo and the resultant residue was diluted with chloroform. The solution was washed with an aqueous saturated sodium hydrogencarbonate solution and brine. The organic layer was separated, dried over sodium sulfate and concentrated in vacuo. The resultant residue was crystallized from n-hexane and the crystals were collected by filtration and dried to give dimethyl trans-cyclohexane-1,4-dicarboxylate (545.0 g).
   MS(APCI)m/z; 201 [M+H]⁺

(2) To a solution of dimethyl trans-cyclohexane-1,4-dicarboxylate obtained in the above step (1) (150.0 g) in tetrahydrofuran (1500 mL) was added dropwise a mixture of 28 % sodium methoxide/methanol solution (149 g) and water (13.2 g) under ice-cooling and the mixture was stirred at room temperature for 3.5 hours. To the reaction mixture was added n-hexane (1500 mL) and the precipitates were collected by filtration. The precipitates was added to a mixture of conc.HCl (50 mL), water (450 mL) and chloroform (1000 mL) under ice-cooling and the mixture was stirred at room temperature for 20 minutes. The organic layer was separated and the aqueous layer was extracted with chloroform. The combined organic layer was dried over sodium sulfate and concentrated in vacuo. The resultant residue was crystallized from n-hexane and the precipitated crystals were collected by filtration and dried to give monomethyl trans-cyclohexane-1,4-dicarboxylate (106.0 g).
   MS(ESI)m/z; 185 [M-H]⁻

(3) Under argon gas atmosphere to a solution of momomethyl trans-cyclohexane-1,4-dicarboxylate (14.3 g) in tetrahydrofuran (78 mL) was added dropwise 1.0 M solution of borane-tetrahydrofuran complex in tetrahydrofuran (100 mL) at -50 °C over a period of 1 hour and the mixture was stirred at -10 °C for 1 hour. To the reaction mixture was added water (160 mL) and an aqueous saturated sodium hydrogencarbonate solution (160 mL) under ice-cooling and the mixture was extracted with ethyl acetate (160 mL x 4). The extract was washed with brine, dried over sodium sulfate and concentrated in vacuo. The resultant residue was purified by a flash column chromatography on silica gel (Solvent; chloroform/methanol = 20 : 1) to give methyl trans-4-(hydroxymethyl)cyclohexanecarboxylate (13.25 g, yield: 100 %) as an oil.
   MS(APCI)m/z; 173 [M+H]⁺

(4) Under argon gas atmosphere to a solution of oxalyl chloride (4.48 mL) in methylene chloride (50 mL) was added dropwise a solution of dimethysulfoxide (4.55 g) in methylene chloride (5 mL) at -60 °C and the mixture was stirred at the same temperature for 15 minutes. To the mixture was added dropwise a solution of the compound obtained in the above step (3) (5.9 g) in methylene chloride (30 mL) over a period of 30 minutes and the mixture was stirred at the same temperature for 1 hour. To the reaction mixture was added dropwise triethylamine (16.7 mL) at -60 °C and the mixture was stirred at the same temperature for 30 minutes and then stirred at 0 °C for 1 hour. The reaction mixture was diluted with chloroform, washed successively with water, an aqueous 5 % citric acid, water and brine, dried over sodium sulfate and concentrated in vacuo to give methyl trans-4-formylcyclohexanecarboxylate (5.32 g, yield: 91 %) as an oil.

(5) To a solution of the compound obtained in the above step (4) (1.81 g) and (2-methoxyethyl)(tert-butyl)amine (2.80 g) in methylene chloride (20 mL) was added successively sodium triacetoxyborohydride (3.38 g) and acetic acid (1.28 g) under ice-cooling and the mixture was stirred at room temperature for 4 days. To the reaction mixture was added an aqueous saturated sodium hydrogencarbonate solution and the mixture was extracted with chloroform (x 2). The extract was concentrated in vacuo and the resultant crude product was purified by a flash column chromatography on silica gel (Solvent; chloroform/methanol/aqueous ammonia = 9 : 1 : 0.1) to give methyl trans-[(2-dimethylaminoethylamino)methyl]cyclohexanecarboxylate (455 mg, yield: 15 %) as an oil.
   MS(APCI)m/z; 286 [M+H]⁺

### Reference Example A2

(1) To a solution of monomethyl trans-cyclohexane-1,4-dicarboxylate (compound obtained in the above Reference Example Al(2), 100.0 g) in tert-buthanol (1000 mL) was added diphenylphosphoryl azide (155 g) and triethylamine (78.6 mL) and the mixture was stirred at 60 °C for 1 hour and then refluxed under heating for 17 hours. After cooling, to the reaction mixture was added ice-water and the mixture was extracted with ethyl acetate. The organic layer was washed with an aqueous saturated sodium hydrogencarbonate solution and brine, dried over sodium sulfate and concentrated in vacuo. To a solution of the resultant residue in methanol (250 mL) was added water (750 mL) and the mixture was stirred under ice-cooling for 30 minutes. The precipitates were collected by filtration, washed successively with water/methanol (3:1, 1000 mL) and n-hexane and dried to give methyl trans-4-(tert-butoxycarbonylamino)cyclohexanecarboxylate (117.0 g).
   MS(APCI)m/z; 275 [M+H]⁺

(2) To a solution of the compound obtained in the above step (1) (234.0 g) in dioxane (500 mL) was added 4N HCl/dioxane (500 mL) and the mixture was stirred at room temperature for 19 hours. The reaction mixture was concentrated in vacuo and the resultant residue was suspended in diethylether and the precipitates were collected by filtration to give methyl trans-4-aminocyclohexanecarboxylate hydrochloride (121.9 g).
   MS(APCI)m/z; 158 [M+H]⁺

(3) A suspension of the compound obtained in the above step (2) (10 g), 1,5-diiodopentane (9.2 mL) and sodium carbonate (16.4 g) in tetrahydrofuran (300 mL)/N,N-dimethylacetamide (60 mL) was stirred at 70 °C for 20 hours. The reaction mixture was concentrated in vacuo and the residue was dissolved in ethyl acetate/water. The organic layer was separated and washed successively with water and brine, dried over sodium sulfate and concentrated in vacuo. The resultant residue was purified by a column chromatography on NH-silica gel (Solvent; ethyl acetate/n-hexane = 1 : 5) to give methyl trans-4-(1-piperidyl)cyclohexanecarboxylate (10.17 g).
   MS(APCI)m/z; 226 [M+H]⁺

(4) To a solution of the compound obtained in the above step (3) (10.17 g) in dioxane (130 mL) was added 2N hydrochloric acid (70 mL) and the mixture was stirred at 105 °C for 5 hours while evaporating methanol. The reaction mixture was concentrated in vacuo and the resultant residue was suspended in diethylether. The precipitates were collected by filtration to give trans-4-(1-piperidyl)cyclohexanecarboxylic acid hydrochloride (11.78 g, yield: quant.) as an amorphous solid.
   MS(APCI)m/z; 212 [M+H]⁺

### Reference Example A3

(1) To a solution of methyl 4-(aminomethyl)benzoate (5.08 g) in methylene chloride (30 mL) was added di-tert-butyl dicarbonate (6.4 g) under ice-cooling and the mixture was stirred at room temperature for 24 hours. The reaction mixture was diluted with methylene chloride (20 mL) and thereto was added water (40 mL). After stirring, the organic layer was separated and concentrated in vacuo to give crude methyl 4-[N-(tert-butoxycarbonyl)aminomethyl] benzoate.

(2) To a solution of the compound obtained in the above step (1) (1.5 g) in tetrahydrofuran (15 mL) was added 60 % oily dispersion of sodium hydride (407 mg) under ice-cooling and the mixture was stirred at room temperature for 30 minutes. Thereto was added dropwise ethyl iodide (2.26 mL) under ice-cooling and the mixture was stirred at 60 °C for 1 hour. After cooling to room temperature, the reaction mixture was diluted with ethyl acetate (10 mL) and thereto was added water (20 mL). After stirring, the organic layer was separated and concentrated and the resultant residue was purified by a flash column chromatography on NH-silica gel (Solvent; n-hexane/ethyl acetate = 20 : 1) to give methyl 4-[[N-ethyl-N-(tert-butoxycarbonyl)]aminomethyl]benzoate (929 mg, yield: 56 %) as an amorphous solid.
   MS(APCI)m/z; 294 [M+H]⁺

### Reference Example A4

Under argon gas atmosphere a solution of ethyl 4-fluorobenzoate (20 g), N,N-dimethylethylenediamine (20 g) and potassium carbonate (32.9 g) in dimethylsulfoxide (200 mL) was stirred at 80 °C for 3 days. After cooling to room temperature, to the reaction mixture was added ethyl acetate and water. After stirring, the reaction mixture was extracted with ethyl acetate (x 2) and the organic layer was extracted with 10 % hydrochloric acid. The aqueous layer was washed with ethyl acetate, neutralized with 10 % sodium hydroxide solution and then extracted with ethyl acetate (x 3). The extract was dried over sodium sulfate and concentrated in vacuo. The resultant residue was purified by a flash column chromatography on NH-silica gel (Solvent; n-hexane/ethyl acetate = 8 : 1 → 4 : 1) to give ethyl 4-[[2- (dimethylamino)ethyl]amino]benzoate (12.45 g, yield: 44 %) as an amorphous solid.
MS(APCI)m/z; 237 [M+H]⁺

### Reference Example A5

(1) To a solution of 4-carboxyl-1-(tert-butoxycarbonyl)piperidine (2.0 g) in methylene chloride (26 mL) was added successively N,N-disopropylethylamine (3.0 mL), bromo-tris(pyrrolidino)phosphonium hexafluorophosphate (PyBroP, 6.1 g) and (2-methoxyethyl)(tert-butyl)amine (1.6 g) and the mixture was stirred at room temperature for 4 days. The reaction mixture was diluted with chloroform and thereto was added an aqueous saturated sodium hydrogencarbonate solution. After stirring, the organic layer was separated and the aqueous layer was extracted again with chloroform. The combined organic layer was dried over sodium sulfate, filtered and concentrated in vacuo. The resultant residue was purified by a flash column chromatography on silica gel (Solvent; n-hexane/ethyl acetate = 10 : 0 → 70 : 30) to give 4-[tert-butyl(2-methoxyethyl)-carbamoyl]-1-(tert-butoxycarbonyl)piperidine (887 mg, yield: 30 %) as an amorphous solid.
   MS(APCI)m/z; 343 [M+H]⁺

(2) To a solution of the compound obtained in the above step (1) (1.2 g) in tetrahydrofuran (11 mL) was added 1M solution of borane-tetrahydrofuran-complex in tetrahydrofuran (6.7 mL) and the mixture was refluxed under heating for 7 hours. To the reaction mixture was added 10 % hydrochloric acid at room temperature and the mixture was stirred at 65°C for 40 minutes and concentrated in vacuo. The resultant residue was treated with chloroform and water and thereto was added potassium carbonate under stirring (pH to >10). The mixture was extracted with chloroform (x 2) and the combined chloroform layer was dried over sodium sulfate, filtered and concentrated. To the resultant residue was added n-hexane/ethyl acetate (8 : 1) and the mixture was stirred. The precipitates were removed by filtration and the filtrate was concentrated in vacuo to give 4-[[N-(2-methoxyethyl)- N-(tert-butyl)amino]methyl]piperidine (573 mg, yield: 70 %) as an yellow oil.
   MS(APCI)m/z; 229 [M+H]⁺

### Reference Example A6

(1) To a solution of 4-formyl-1-(tert-butoxycarbonyl)piperidine (1.0 g) and 2-dimethylaminoethylamine (766 µL) in chloroform (12 mL) was added successively sodium triacetoxyborohydride (2.0 g) and acetic acid (537 µL) under ice-cooling and the mixture was stirred at room temperature for 1 day. The reaction mixture was diluted with chloroform and thereto was added an aqueous saturated sodium hydrogencarbonate solution and potassium carbonate powder (pH 10). The mixture was extracted with chloroform (x 2) and the extract was dried over sodium sulfate and concentrated to give crude 4-[[[2-(dimethylamino)ethyl]amino]methyl]-1-(tert-butoxycarbonyl)piperidine (3 g) as a colorless oil.

(2) To a solution of the compound obtained in the above step (1) in methylene chloride (10 mL) was added N-(methylpolystyrene)-4-(methylamino)pyridine (PS-DMAP, 6 g, 1.57 mmol/g) and acetyl chloride (1.7 mL) and the mixture was shaken at room temperature for 18 hours. The reaction mixture was filtered to remove PS-DMAP and precipitates and the filtrate was concentrated in vacuo to give crude 4-[[N-acetyl-N-[2-(dimethyamino)ethyl]amino]methyl]-1-(tert-butoxycarbonyl)piperidine (1.3 g) as a colorless powder.

(3) To a solution of the compound obtained in the above step (2) (1.3 g) in dioxane (5.8 mL) was added 4N HCl/dioxane (5.8 mL) and the mixture was stirred at room temperature for 20 hours. The reaction mixture was diluted with anhydrous ether to precipitate the objective compound as an oil. After removing the supernatant, the residue was washed with ether, dried in vacuo, dissolved in water and lyophilized to give 4-[[N-acetyl-N-[2-(dimethylamino)ethyl]amino]methyl]piperidine dihydrochloride (918 mg, yield: 65 %) as an amorphous solid.
   MS(APCI)m/z; 228 [M+H]⁺

### Reference Example A7

(1) N,N-Dimethylformamide (39.3 mL) was added dropwise to phosphorus oxychloride (250 mL) at a temperature bellow 10 °C and thereto was added portionwise barbituric acid (50 g) over a period of 20 minutes and the mixture was stirred at room temperature and then stirred at 90 °C overnight. The reaction mixture was evaporated to remove excess amount of phosphorus oxychloride. The resultant residue was gradually poured to water under stirring. The mixture was extracted with chloroform and the organic layer was dried over sodium sulfate and concentrated in vacuo. The resultant precipitates were triturated in isopropylether and collected by filtration to give 5-formyl-2,4,6-trichloropyrimidine (57.5 g, yield: 70 %) as yellow crystals.
   MS(APCI)m/z; Not detectable.

(2) To a solution of the compound obtained in the above step (1) (40 g) in methanol (950 mL) was added dropwise a solution of hydrazine monohydrate (9.45 mL) in methanol (240 mL) at -10 °C and thereto was added dropwise a solution of triethylamine (26.8 mL) in methanol (240 mL) at -10 °C. The mixture was stirred at the same temperature for 2 hours and evaporated in vacuo. The residue was suspended in hot isopropyl alcohol and the insoluble materials were removed by filtration. The combined filtrate was concentrated in vacuo to give 4,6-dichloro-1H-pyrazolo[3,4-d]pyrimidine (23.49 g, yield: 67 %) as a yellow amorphous solid.
   MS(APCI)m/z; Not detectable.

(3) To a solution of the compound obtained in the above step (2) (500 mg) in N,N-dimethylformamide (10 mL) was added gradually N-tert-butoxycarbonylpiperazine (591 mg), and triethylamine (0.73 mL) at 0 °C and the mixture was stirred at the same temperature for 10 minutes. To the reaction mixture was added water and the mixture was extracted with ethyl acetate. The extract was dried over sodium sulfate and concentrated in vacuo. The residue was triturated in ispropylether and the precipitates were collected by filtration to give 6-chloro-4-[4-(tert-butoxycarbonyl)piperazin-1-yl]-1H-pyrazolo[3,4-d]pyrimidine (826 mg, yield: 92 %) as a yellow powder.
   MS(APCI)m/z; 339/341 [M+H]⁺

(4) To a solution of the compound obtained in the above step (3) (820 mg), 3-ethoxybenzylalcohol (550 mg) and triphenylphosphine (1.26 g) in tetrahydrofuran (10 mL) was added diisopropyl azodicarboxylate (1.0 mL) under ice-cooling and the mixture was stirred at room temperature overnight. The reaction mixture was concentrated in vacuo and the residue was purified by a column chromatography on silica gel (Solvent; n-hexane/ethyl acetate = 1 : 1) to give 6-chloro-1-(3-ethoxybenzyl)-4-[4-(tert-butoxycarbonyl)piperazin-1-yl]-1H-pyrazolo[3,4-d]pyrimidine (1.5 g) as a pale yellow oil.
   MS(APCI)m/z; 473/475[M+H]⁺

(5) To a solution of the compound obtained in the above step (4) (1.54 g) in dioxane (10 mL) was added 4N HCl/dioxane (6.0 mL) and the mixture was stirred at room temperature overnight. The reaction mixture was diluted with ethylether and the precipitates were collected by filtration to give 6-chloro-1-(3-ethoxybenzyl)-4-(piperazin-1-yl)-1H-pyrazolo[3,4-d]pyrimidine dihydrochloride (556 mg, yield throughout the two steps: 52%) as a yellow powder.
   MS(APCI)m/z; 373/375[M+H]⁺

### Reference Example A8

(1) To a solution of 4-(4-tert-butoxycarbonylpiperazin-1-yl)-1H-pyrazolo [3,4-d]pyrimidine (10 g) in N,N-dimethylformamide (100 mL) was added N-bromosuccinimide (7 g) and the mixture was stirred at 100 °C for 220 minutes. After cooling, the reaction mixture was concentrated in vacuo and the resultant residue was dissolved in chloroform (150 mL). Thereto was added n-hexane (100 mL) and the precipitates were removed by filtration. The filtrate was concentrated in vacuo and the residue was dissolved in chloroform (20 mL) and thereto was added n-hexane. The precipitated crystals were collected by filtration and dried to give 3-bromo-4-(4-tert-butoxy-carbonylpiperazin-1-yl)-1H-pyrazolo[3,4-d]pyrimidine (11.22 g, yield: 89 %) as an amorphous solid.
   MS(APCI)m/z; 383/385[M+H]⁺

(2) To a solution of the compound obtained in the above step (1) (5 g), triphenylphosiphine (4.45 g) and 3-ethoxybenzylalcohol (2.4 mL) in tetrahydrofuran (40 mL) was added dropwise diisopropyl azodicarboxylate (3.35 mL) under ice-cooling and the mixture was stirred at room temperature for 3 days. The reaction mixture was diluted with chloroform and washed with an aqueous saturated sodium hydrogencarbonate solution. The organic layer was separated. The combined organic layer was dried over sodium sulfate and concentrated in vacuo. The resultant residue was purified by a column chromatography on silica gel (Solvent; n-hexane/ethyl acetate = 7 : 3 → chloroform/methanol = 20 : 1) to give 3-bromo-1-(3-ethoxybenzyl)-4-(piperazin-1-yl)-1H-pyrazolo[3,4-d]pyrimidine (5.2 g, yield: 77 %) as an amorphous solid.
   MS(APCI)m/z; 517/519 [M+H]⁺

(3) To a solution of the compound obtained in the above step (2) (400 mg) in dioxane was added trimethylboroxine (108 µL), [1,1'-bis(diphenylphosphino) ferrocene]dichloropalladium (127 mg) and potassium phosphate (492 mg) and the mixture was heated in a microwave reactor at 120 °C for 1 hour. The reaction mixture was purified by a column chromatography on silica gel (Solvent; n-hexane/ethyl acetate = 7 : 3) to give 4-(4-tert-butoxycarbonyl)-1-(3-ethoxybenzyl)-3-methyl-1H-pyrazolo[3,4-d]-pyrimidine (281 mg, yield: 80 %) as an amorphous solid.
   MS(APCI)m/z; 453 [M+H]⁺

(4) To a solution of the compound obtained in the above step (3) (381 mg) in dioxane (5 mL) was added 4N HCl/dioxane (1.9 mL) and the mixture was stirred at room temperature overnight. Thereto was added diethylether, and after stirring for 30 minutes, the precipitates were collected by filtration and dried to give 1-(3-ethoxybenzyl)-3-methyl-1H-pyrazolo[3,4-d]pyrimidine dihydrochloride (320 mg, yield: 89 %) as an amorphous solid.
   MS(APCI)m/z; 353 [M+H]⁺

### Reference Example A9

(1) A suspension of allopurinol (4-hydroxy-1H-pyrazolo[3,4-d]pyrimidine, 25 g) and dimethylaniline (75 mL) in phosphorus oxychloride (350 mL) was stirred under heating for 1.5 hours. After cooling to room temperature, the reaction mixture was concentrated in vacuo and the residue was poured to ice water. The mixture was extracted with ethyl acetate (x 3, 2.2 L in total). The extract was washed with brine, dried over sodium sulfate and concentrated in vacuo to give crude 4-chloro-1H-pyrazolo[3,4-d]pyrimidine (19 g) as crystals. To a solution of the compound (19 g) in N,N-dimethylformamide (200 mL) was added N,N-diisopropylethylamine (36.6 mL) and ethyl isonipecotate (22 mL) and the mixture was stirred at room temperature overnight. The reaction mixture was concentrated and to the residue was added an aqueous saturated sodium hydrogencarbonate solution and the mixture was extracted with ethyl acetate (x 2). The extract was washed with brine, dried over sodium sulfate and concentrated in vacuo. The residue was purified by a flash column chromatography on silica gel (Solvent; chloroform/methanol = 50 : 1) to give 4-(4-ethoxycarbonylpiperidin-1-yl)-1H-pyrazolo[3,4-d]pyrimidine (11.1 g, yield: 22 %) as a pale yellow powder.
   MS(APCI)m/z; 276 [M+H]⁺

(2) To a solution of 4-(4-ethoxycarbonylpiperidin-1-yl)-1H-pyrazolo[3,4-d]-pyrimidine (compound obtained in the above step (1), 2.0 g), 3-ethoxybenzylalcohol (1.66 g) and triphenylphosphine (2.86 g) in tetrahydrofuran (20 mL) was added diisopropyl azodicarboxylate (2.15 mL) under ice-cooling and the mixture was stirred at room temperature for 1 hour. To the reaction mixture was added ethyl acetate and the mixture was extracted with 10 % hydrochloric acid. The extract was neutralized with aqueous saturated sodium hydrogencarbonate solution and the mixture was extracted with chloroform. The extract was dried over sodium sulfate and concentrated in vacuo, and the residue was purified by a column chromatography on silica gel (Solvent; n-hexane/ethyl acetate = 3 : 1 → 1 : 1) to give 1-(3-ethoxybenzyl)-4-(4-ethoxycarbonylpiperidin-1-yl)-¹H-pyrazolo- [3,4-d]pyrimidine (1.8 g, yield: 61 %) as a pale yellow oil.
   MS(APCI)m/z; 410 [M+H]⁺

(3) To a solution of the compound obtained in the above step (2) (1.0 g) in dioxane (10 mL) was added 2N hydrochloric acid and the mixture was stirred under heating for 3 hours. After cooling to room temperature, the reaction mixture was concentrated in vacuo and the residue was triturated in ethylether and collected by filtration to give 4-(4-carboxypiperidin-1-yl)-1-(3-ethoxybenzyl)-1H-pyrazolo[3,4-d]pyrimidine (867 mg, yield: 85 %) as a colorless powder.
   MS(APCI)m/z; 382 [M+H]⁺

### Reference Example A10

(1) A suspension of allopurinol (20 g) and N,N-diisopropylethylamine (41 mL) in phosphorus oxychloride (245 mL) was refluxed under heating for 3 hours. After cooling to room temperature, the reaction mixture was concentrated in vacuo and the residue was diluted with ethyl acetate. Thereto was added cold water and the mixture was extracted with ethyl acetate (x 3, 2.5 L in total). The organic layer was separated and concentrated in vacuo to give crude 4-chloro-1H-pyrazolo[3,4-d]pyrimidine (21.3 g) as crystals.

(2) To a suspension of the compound obtained in the above step (1) (5 g) in toluene (45 mL)/N,N-dimethylformamide (15 mL) was added sodium thiomethoxide (2.7 g) under ice-cooling and the mixture was stirred at or bellow 25 °C overnight. The reaction mixture was diluted with toluene and the precipitates were collected by filtration and dried to give 4-methylthio-1H-pyrazolo[3,4-d]pyrimidine (5.25 g, yield: 97.8 %) as an amorphous solid.
   MS(APCI)m/z; 167 [M+H]⁺

(3) To a solution of the compound obtained in the above step (2) (2 g), 3-ethoxybenzylalcohol (2.2 mL) and triphenylphosphine (4.1 g) in tetrahydrofuran (30 mL) was added diisopropyl azodicarboxylate (3.1 mL) under ice cooling and the mixture was stirred at room temperature overnight. The reaction mixture was concentrated in vacuo and the resultant residue was purified by a column chromatography on silica gel (Solvent; n-hexane/ethyl acetate = 97 : 3 → 95 : 5) to give 1-(3-ethoxybenzyl)-4-methylthio-1H-pyrazolo[3,4-d]pyrimidine (1.2 g, yield: 35 %) as an amorphous solid.
   MS(APCI)m/z; 301 [M+H]⁺

(4) To a solution of the compound obtained in the above step (3) (250 mg) in tetrahydrofuran (4.2 mL) was added methachloroperbenzoic acid (215 mg) and the mixture was stirred at room temperature for 2 hours. Thereto was added methyl 4-aminobutyrate hydrochloride (384 mg) and triethylamine (384 µL) and the mixture was stirred at room temperature for 17 hours. The reaction mixture was diluted with chloroform and thereto was added an aqueous saturated sodium hydrogencarbonate solution. The organic layer was separated, dried over sodium sulfate and concentrated in vacuo. The resultant residue was purified by a column chromatography on silica gel (Solvent; chloroform → chloroform/methanol (9 : 1)) to give 1-(3-ethoxybenzyl)-4-[N-(3-ethoxycarbonylpropyl)-amino]-1H-pyrazolo[3,4-d]pyrimidine (264 mg, yield: 86 %) as a colorless amorphous solid.
   MS(APCI)m/z; 370 [M+H]⁺

(5) To the compound obtained in the above step (4) (263 mg) was added methanol (2.1 mL) and 4N sodium hydroxide solution (0.5 mL) and the mixture was stirred at room temperature overnight. To the reaction mixture was added 2N hydrochloric acid (1.1 mL) and the mixture was stirred at room temperature for 3 hours. The reaction mixture was filtered and the filtrate was concentrated in vacuo. The residue was triturated in methanol/ethylether and the precipitates were collected to give 1-(3-ethoxybenzyl)-4-[N-(3-carboxypropyl)- amino]-1H-pyrazolo[3,4-d]pyrimidine (215 mg, yield: 77 %) as a colorless amorphous solid.
   MS(APCI)m/z; 354 [M-H]⁻

### Reference Example A11

(1) 4,6-Dichloro-1H-pyrazolo[3,4-d]pyrimidine (1.0 g) was treated in the same manner as described in Reference Example A9-(2) to give 4,6-dichloro-1-(3-ethoxybenzyl)-1H-pyrazolo[3,4-d]pyrimidine (982 mg, 57 %) as a colorless amorphous solid.
   MS(APCI)m/z; 323/325 [M+H]⁺

(2) To a suspension of zinc powder (227 mg) in N,N-dimethylacetamide (3 mL) was added iodine (50 mg) and the mixture was stirred for 10 minutes. To the suspension was added dropwise a solution of tert-butyl 4-iodopiperidin-1-carboxylate (722 mg) in N,N-dimethylacetamide (3 mL) and the mixture was stirred at 80 °C for 260 minutes. After cooling, to the reaction mixture was added tetrakis(triphenylphosphine) palladium(0) (100 mg), a solution of the compound obtained in the above step (1) (500 mg) in N,N-dimethylacetamide (3 mL) and 0.5M solution of zinc chloride in tetrahydrofuran (9.3 mL) at room temperature and the mixture was stirred at 90 °C for 15 hours. After cooling, the reaction mixture was filtered through Cerite and to the filtrate was added water. The mixture was extracted with ethyl acetate and the extract was dried over sodium sulfate and concentrated in vacuo. The residue was purified by a column chromatography on silica gel (Solvent; n-hexane/ethyl acetate = 5 : 1) to give 6-chloro-1-(3-ethoxybenzyl)-4-(1-tert-butoxycarbonyl-4-piperidyl)-1H-pyrazolo[3,4-d] pyrimidine (594 mg, yield: 81 %) as a yellow oil.
   MS(APCI)m/z; 472/474 [M+H]⁺

(3) To a solution of the compound obtained in the above step (2) (594 mg) in dioxane (3.1 mL) was added 4N HCl/dioxane (3.1 mL) and the mixture was diluted with ethylether. The resultant precipitates were collected by filtration to give 6-chloro-1-(3-ethoxybenzyl)-4-(4-piperidyl)-1H-pyrazolo[3,4-d]pyrimidine hydrochloride (404 mg, yield: 99 %) as an yellow powder.
   MS(APCI)m/z; 372/374 [M+H]⁺

(4) To a solution of the compound obtained in the above step (3) (150 mg) in methanol (4 mL) was added palladium-carbon (30 mg) and the mixture was stirred under atmospheric pressure of hydrogen gas at room temperature for 24 hours. The reaction mixture was filtered and the filtrate was concentrated in vacuo. To the residue was added an aqueous saturated sodium hydrogencarbonate solution and the mixture was extracted with chloroform. The extract was dried over sodium sulfate and concentrated in vacuo and the residue was purified by a column chromatography on silica gel (Solvent; chloroform/methanol/28 % aqueous ammonia = 10 : 1 : 0.1) to give 1-(3-ethoxybenzyl)-4-(4-piperidyl)-1H-pyrazolo[3,4-d]pyrimidine (22 mg, yield: 18 %) as a colorless oil.
   MS(APCI)m/z; 338 [M+H]⁺

### Reference Example A12

(1) To a solution of 3-ethoxybenzylalcohol (3.0 g) in methylene chloride (30 mL) was added methanesulfonyl chloride (1.8 mL) and triethylamine (4.2 mL) at 0 °C and the mixture was stirred at the same temperature for 0.5 hour. To the reaction mixture was added water and the mixture was extracted with chloroform. The extract was dried over magnesium sulfate and concentrated in vacuo to give 3-ethoxybenzyl methanesulfonate (4.5 g, 99 %) as a yellow oil.
   MS(APCI)m/z; 248 [M+NH₄]⁺

(2) To a solution of the compound obtained in the above step (1) (1.5 g) in N,N-dimethylsulfoxide (15 mL) was added 6-chloro-7-deazapurine (1.0 g) and potassium carbonate (1.0 g) and the mixture was stirred at room temperature for 18 hours. To the reaction mixture was added water and the mixture was extracted with ethyl acetate. The extract was dried over magnesium sulfate and concentrated in vacuo. The resultant crude product was purified by flash column chromatography on silica gel (Solvent; n-hexane/ethyl acetate = 10 : 1) to give 4-chloro-1-(3-ethoxybenzyl)-1H-pyrrolo[2,3-d]pyrimidine (1.39 g, yield: 74 %) as a colorless oil.
   MS(APCI)m/z; 288/290 [M+H]⁺

(3) To a solution of piperazine (2.4 g) in water (1.0 mL) and N,N-dimethylformamide (4.0 mL) was added the compound obtained in the above step (2) (0.83 g) and the mixture was stirred at room temperature for 3 hours. To the reaction mixture was added water and the mixture was extracted with ethyl acetate. The extract was dried over magnesium sulfate and concentrated in vacuo. The resultant crude product was purified by a flash column chromatography on silica gel (Solvent; chloroform/methanol = 20 : 1) to give 4-(1-piperazinyl)-1-(3-ethoxybenzyl)-1H-pyrrolo[2,3-d]pyrimidine dihydrochloride (1.08 g) as a colorless powder.
   MS(APCI)m/z; 338 [M+H]⁺

### Reference Example A13

(1) A mixture of 4-chloro-1H-pyrazolo[3,4-b]pyridine (200 mg) and piperazine (5 g) was stirred at 150 °C for 1 hour and the reaction mixture was purified by a flash column chromatography on silica gel (Solvent; chloroform/methanol = 5 : 1) to give 4-(1-piperazinyl)-1H-pyrazolo[3,4-b]pyridine (180 mg, yield: 68 %) as colorless crystals.
   MS(APCI)m/z; 204 [M+H]⁺

(2) The compound obtained in the above step (1) (35 mg) was treated in the same manner as described in Example A1 to give 4-[4-[[4-[[N-(2-methoxyethyl)-N-(tert-butyl)-amino]methyl]cyclohexyl]carbonyl]piperazin-1-yl]-1H-pyrazolo[3,4-b]pyridine (54 mg, yield: 69 %) as a yellow powder.
   MS(APCI)m/z; 457 [M+H]⁺

### Reference Example A14

(1) To a solution of 4-nitro-3-pyridinecarbaldehyde 1-oxide (6.4 g) in ethanol (200 mL) was added dropwise hydrazine hydrate (5.6 mL) and the mixture was refluxed under heating for 3 hours. The reaction mixture was concentrated and the residue was purified by a flash column chromatography on silica gel (Solvent; chloroform/methanol = 10 : 1) to give 1H-pyrazolo[4,3-c]pyridine 5-oxide (2.4 g, yield: 47 %) as yellow crystals.
   MS(APCI)m/z; 136 [M+H]⁺

(2) To the compound obtained in the above step (1) (1.4 g) was added phosphorus oxychloride (30 mL) and the mixture was refluxed under heating for 3 hours. The reaction mixture was concentrated and to the residue was added an aqueous saturated sodium hydrogencarbonate solution. The mixture was extracted with ethyl acetate and the extract was dried over magnesium sulfate and concentrated to give a mixture of 4-chloro-1H-pyrazolo[4,3-c]pyridine and 6-chloro-1H-pyrazolo[4,3-c]pyridine (0.81 g, yield: 51 %).
   MS(APCI)m/z; 154/156 [M+H]⁺

(3) To a solution of the compound obtained in the above step (2) (0.72 g) in N-methylpyrrolidone (5.0 mL) was added N-tert-butoxycarbonylpiperazine (1.7 g) and the mixture was stirred at 140 °C for 2 hours. After cooling, to the reaction mixture was added water and the mixture was extracted with ethyl acetate. The extract was dried over magnesium sulfate and concentrated in vacuo. The residue was purified by a flash column chromatography on NH-silica gel (Solvent; n-hexane/ethyl acetate = 1 : 1) to give 4-[4-(tert-butoxycarbonyl)piperazin-1-yl]-1H-pyrazolo[4,3-c]pyridine (0.64 g) as colorless crystals.
   MS(APCI)m/z; 304 [M+H]⁺

(4) The compound obtained in the above step (3) (0.74 g) was treated in the same manner as described in Example A115 to give 1-(3-ethoxybenzyl)-4-(1-piperazinyl)-1H-pyrazolo[4,3-c]pyridine (0.52g, yield: 52 %) as colorless crystals.

### Reference Example A15

The corresponding starting materials were treated in the same manner as described in Example A8-(1) to give a compound as shown in the following Table 39.

**Table 39**

| | | |
|---|---|---|
| 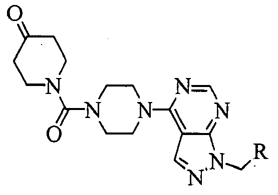 | | |

| Ref. Ex. Nos. | R | Physicochemical properties etc. |
|---|---|---|
| A15 | 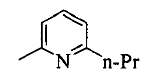 | amorphous solid MS(APCI)462 [M+H]+ |

| | | |
|---|---|---|
| n-Pr: n-propyl group | | |

### Reference Examples A16 to A29

The corresponding starting materials were treated in the same manner as described in either one of the Reference Examples A1 to A6 to give a compound as shown the following Tables 40 and 41.

**Table 40**

| | | |
|---|---|---|
| 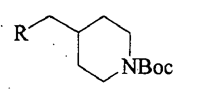 | | |

| Ref. Ex. Nos. | R | Physicochemical properties etc. |
|---|---|---|
| A16 | 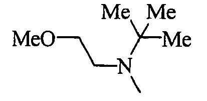 | amorphous solid MS(APCI)229[M+H]+ |
| A17 | 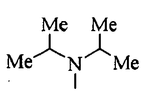 | amorphous solid MS(APCI)199[M+H]+ |
| A18 | 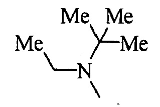 | amorphous solid MS(APCI)199[M+H]+ |
| A19 | 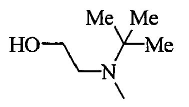 | amorphous solid MS(APCI)215[M+H]+ |
| A20 | 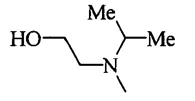 | amorphous solid MS(APCI)201[M+H]+ |

| | | |
|---|---|---|
| Me: methyl group, Boc: tert-butoxycarbonyl group | | |

**Table 41**

| | | | |
|---|---|---|---|
| 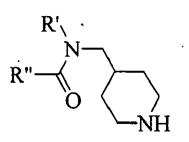 | | | |

| Ref. Ex. Nos. | R' | R" | Physicochemical properties etc. |
|---|---|---|---|
| A21 | 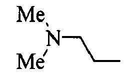 | Me | amorphous solid MS(APCI)228[M+H]+ |
| A22 | 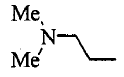 | 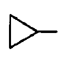 | amorphous solid MS(APCI)254[M+H]+ |
| A23 | 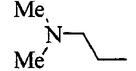 | t-Bu | amorphous solid MS(APCI)270[M+H]+ |
| A24 | 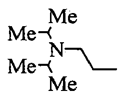 | Me | amorphous solid MS(APCI)284[M+H]+ |
| A25 | 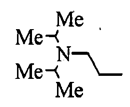 | 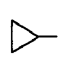 | amorphous solid MS(APCI)310[M+H]+ |
| A26 | 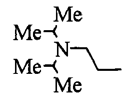 | t-Bu | amorphous solid MS(APCI)326[M+H]+ |
| A27 | 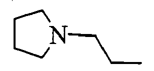 | Me | amorphous solid MS(APCI)254[M+H]+ |
| A28 | 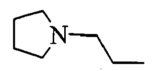 | 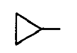 | amorphous solid MS(APCI)280[M+H]+ |
| A29 | 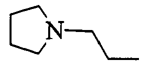 | t-Bu | amorphous solid MS(APCI)296[M+H]+ |

| | | | |
|---|---|---|---|
| Me: methyl group, Boc: tert-butoxycarbonyl group | | | |

### Reference Example A30

To a suspension of 6-chloro-1-(3-ethoxybenzyl)-4-(piperazin-1-yl)-1H-pyrazolo[3,4-d]pyrimidine dihydrochloride (200 mg, compound obtained in Reference Example A7-(5)) in methanol (5.0 mL) was added sodium methylate (0.5 mL, 28 % solution in methanol) and the mixture was refluxed under heating overnight. The reaction mixture was concentrated in vacuo and the resultant residue was purified by a flash column chromatography on silica gel (Solvent; chloroform/methanol = 20 : 1) and treated with hydrochloric acid to give 1-(3-ethoxybenzyl)-6-methoxy-4-(piperazin-1-yl)-1H-pyrazolo[3,4-d]pyrimidine hydrochloride (124 mg, yield: 15 %) as an amorphous solid.
MS(APCI)m/z; 591 [M+H]⁺

### Reference Example A31

To a suspension of the compound obtained in Reference Example A7-(5) (200 mg) in dioxane (2.0 mL) was added trimethylboroxine (68 mg), [1,1'-bis(diphenylphosphino)-ferrocene]dichloropalladium(II) (35 mg) and cesium carbonate (730 mg) and the mixture was stirred at 100°C overnight. The reaction mixture was diluted with ethyl acetate and the mixture was filtered to remove precipitates. The filtrate was concentrated and the resultant residue was purified by a flash column chromatography on silica gel (Solvent; chloroform/methanol = 20 : 1) and treated with hydrochloric acid to give 1-(3-ethoxybenzyl)-6-methyl-4-(piperazin-1-yl)-1 H-pyrazolo[3,4-d]pyrimidine hydrochloride (73 mg, yield: 42 %) as a colorless powder.
MS(APCI)m/z; 353 [M+H]⁺

### Reference Example A32

To a suspension of the compound obtained in Reference Example A7-(5) (200 mg) in dimethoxyethane (2.0 mL) was added successively 2-thienyl boronic acid (115 mg), dichlorobis(triphenylphosphine)palladium(II) (31 mg) and an aqueous 2M sodium carbonate solution (2.2 mL) and the mixture was stirred at 90 °C for 20 hours. The reaction mixture was extracted with ethyl acetate and the extract was dried over magnesium sulfate and evaporated to remove solvent. The resultant residue was purified by a flash column chromatography on silica gel to give 1-(3-ethoxybenzyl)-4-(piperazin-1-yl)-6-(2-thienyl)-1H-pyrazolo[3,4-d] pyrimidine (149 mg, yield: 67 %) as a brown powder.
MS(APCI)m/z; 421 [M+H]⁺

### Reference Example A33

(1) To a solution of 6-chloro-1-(3-ethoxybenzyl)-4-(4-tert-butoxycarbonyl-piperazin-1-yl)-1H-pyrazolo[3,4-d]pyrimidine (100 mg, compound obtained in Reference Example A7-(4)) in N,N-dimethylacetamide (1.0 mL) was added sodium cyanate (28 mg) and dichlorobis(triphenylphosphine)palladium(II) (6 mg) and the mixture was stirred at 150 °C for 24 hours. To the reaction mixture was added an aqueous saturated sodium hydrogencarbonate solution and the mixture was extracted with ethyl acetate. The extract was dried over magnesium sulfate and evaporated to remove solvent. The resultant crude product was purified by a flash column chromatography on silica gel (Solvent; n-hexane/ethyl acetate = 1 : 1) to give 6-cyano-1-(3-ethoxybenzyl)-4-(4-tert-butoxy-carbonylpiperazin-1-yl)-1H-pyrazolo[3,4-d]pyrimidine (42 mg, yield: 45 %) as a yellow powder.
   MS(APCI)m/z; 464 [M+H]⁺

(2) To a solution of the compound obtained in the above step (1) (158 mg) in methylene chloride (0.1 mL) was added trifluoroacetic acid (0;05 mL) and the mixture was stirred at room temperature for 1 hour. To the reaction mixture was added an aqueous saturated sodium hydrogencarbonate solution and the mixture was extracted with ethyl acetate. The extract was dried over magnesium sulfate and evaporated to remove solvent. The resultant crude product was purified by a flash column chromatography on silica gel to give 6-cyano-1-(3-ethoxybenzyl)-4-(1-piperazinyl)-1H-pyrazolo[3,4-d]pyrimidine (100 mg, yield: 81 %) as a yellow powder.
   MS(APCI)m/z; 364 [M+H]⁺

### Reference Example A34

To the compound obtained in Reference Example A7-(4) (500 mg) was added 2.0M solution of dimethylamine in methanol (8.0 mL) and the mixture was stirred at 100 °C overnight. The reaction mixture was concentrated and to the residue was added water. The mixture was extracted with ethyl acetate and the extract was dried over magnesium sulfate and evaporated to remove solvent. The resultant crude product was treated with hydrochloric acid to give 6-(dimethyamino)-1-(3-ethoxybenzyl)-4-(1-piperazinyl)-1H-pyrazolo[3,4-d]pyrimidine dihydrochloride (458 mg, yield: 95 %) as colorless crystals.
MS(APCI)m/z; 382 [M+H]⁺

### Reference Examples A35 to A36

The corresponding staring materials were treated in the same manner as described in either one of the above Reference Examples to give compounds as shown in the following Table 42.

**Table 42**

| | | | |
|---|---|---|---|
| 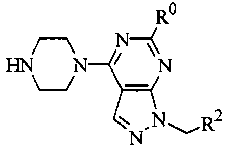 | | | |

| Ref. Ex. Nos. | R⁰ | R² | Physicochemical properties etc. |
|---|---|---|---|
| A35 | Cl | 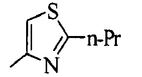 | amorphous solid MS(APCI)372/374[M+H]+ |
| A36 | Cl | 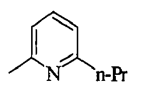 | amorphous solid MS(APCI)384[M+H]+ |

| | | | |
|---|---|---|---|
| n-Pr: n-propyl group | | | |

### Reference Example A37

The corresponding staring materials were treated in the same manner as described in Reference Example A7-(4) to give 1-[(6-bromopyridin-2-yl)methyl]-6-chloro-4-(1-piperazinyl)-1H-pyrazolo[3,4-d]pyrimidine dihydrochloride. To a suspension of the compound (200 mg) in ethanol (2.0 mL) was added sodium ethylate (1.5 mL, 20 % ethanol solution) and the mixture was refluxed under heating overnight. The reaction mixture was concentrated and the residue was purified by flash column chromatography on silica gel (Solvent; chloroform/methanol = 20 : 1) to give 6-ethoxy-1-[(6-ethoxypyridin-2-yl)-methyl]-4-(piperazin-1-yl)-1H-pyrazolo[3,4-d]pyrimidine (118 mg, yield: 73 %) as a brown oil.
MS(APCI)m/z; 384 [M+H]⁺

### Reference Examples A38 to A43

The corresponding staring materials were treated in the same manner as described in either one of the above Reference Examples to give compounds as shown in the following Tables 43 to 45.

**Table 43**

| | | |
|---|---|---|
| 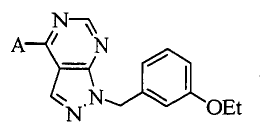 | | |

| Ref. Ex. Nos. | A | Physicochemical properties etc. |
|---|---|---|
| A38 | 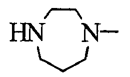 | amorphous solid MS(APCI)353[M+H]+ |
| A39 | 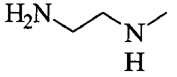 | amorphous solid MS(APCI)313[M+H]+ |

| | | |
|---|---|---|
| Et: ethyl group | | |

**Table 44**

| | | |
|---|---|---|
| 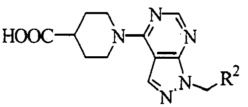 | | |

| Ref. Ex. Nos. | R² | Physicochemical properties etc. |
|---|---|---|
| A40* | 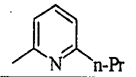 | amorphous solid MS(APCI)379[M+H]- |
| A41* | 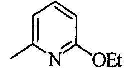 | amorphous solid MS(APCI)381[M-H]- |
| A42* | 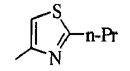 | amorphous solid MS(APCI)385[M+H]- |

| | | |
|---|---|---|
| *: hydrochloride, Et: ethyl group, n-Pr: n-propyl group | | |

**Table 45**

| | | |
|---|---|---|
| 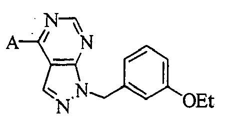 | | |

| Ref. Ex. Nos. | A | Physicochemical properties etc. |
|---|---|---|
| A43 | 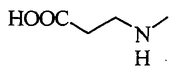 | amorphous solid MS(APCI)340[M-H]- |

| | | |
|---|---|---|
| Et: ethyl group | | |

### Reference Example B1

To a solution of the compound obtained in Reference Example A1-(4) (2.78 g) and (2-methoxyethyl)(tert-butyl)amine (4 g) in chloroform (30 mL) was added successively sodium triacetoxyborohydride (5.19 g) and acetic acid (1.87 mL) under ice-cooling and the mixture was stirred at room temperature for 2 days. To the reaction mixture was neutralized with an aqueous saturated sodium hydrogencarbonate solution (200 mL) and the mixture was stirred at room temperature overnight. The organic layer was separated and the aqueous layer was extracted with chloroform (x 2) and the combined organic layer was washed with brine, dried over sodium sulfate and concentrated in vacuo. To a solution of the resultant crude products in chloroform (50 mL) and thereto was added cation-exchange resins (Amberlyt 15 dry, 4.7 meq/g, 40 g). After allowing to stand for 2 hours, the mixture was filtered through a glass filter. The resins were washed with methanol/methylene chloride (1:1, 200 mL) and treated with 2N solution of ammonia in methanol to elute the resultant product. The eluted fraction was concentrated in vacuo. The resultant oily residue was purified by a flash column chromatography on silica gel (Solvent; chloroform/ethyl acetate = 1 : 1 → 1 : 3) to give methyl trans-[(2-dimethylaminoethylamino)methyl]cyclohexanecarboxylate (1.92 g, yield: 42 %) as a yellow oil.
MS(APCI)m/z; 286 [M+H]⁺

### Reference Example B2

(1) To a suspension of 4-(4-tert-butoxycarbonylpiperazin-1-yl)-1H-pyrazolo [3,4-d]pyrimidine (5 g), 4-ethoxybenzylalcohol (5 g) and triphenylphosphine (8.62 g) in tetrahydrofuran (50 mL) was added dropwise diisopropyl azadicarboxylate (6.98 g) under ice-cooling and the mixture was stirred at room temperature for 18 hours. The reaction mixture was concentrated in vacuo and the resultant residue was purified by a column chromatography on silica gel (Solvent; chloroform: methanol = 100 : 1) to give 1-(3-ethoxybenzyl)-4-(4-tert-butoxycarbonylpiperazin-1-yl)-1H-pyrazolo[3,4-d] pyrimidine (12.95 g, yield: quantitative) as an oil.
   MS(APCI)m/z; 439 [M+H]⁺

(2) To a solution of the compound obtained in the above step (1) (12.9 g) in methanol (28 mL) was added 4N HCl/dioxane (28 mL) and the mixture was stirred at room temperature for 16 hours. Thereto was added ethyl acetate (56 mL) and the resultant precipitates were collected by filtration and washed with ethyl acetate to give 1-(3-ethoxybenzyl)-4-piperazin-1-yl-1H-pyrazolo[3,4-d]pyrimidine dihydrochloride (4.19 g, yield: 62 %) as colorless crystals.
   MS(APCI)m/z; 339 [M+H]⁺

### Reference Example B3

(1) To a solution of 3-hydroxymethyl-1-propyl-1H-pyridin-2-one (1.17 g, the compound obtained in Reference Example B16) in chloroform (10 mL) was added methanesulfonyl chloride (812 µL) and triethylamine (1.46 mL) under ice-cooling and the mixture was stirred at room temperature overnight. The reaction mixture was diluted with chloroform, washed with an aqueous 10 % citric acid solution and an aqueous saturated sodium hydrogencarbonate solution, dried over sodium sulfate and concentrated in vacuo to give crude (2-oxo-1-propyl-1,2-dihydropyridin-3-yl)methyl methanesulfonate.

(2) To a solution of the compound obtained in the above step (1) and 4-(4-tert-butoxycarbonylpiperazin-1-yl)-1H-pyrazolo[3,4-d]pyrimidine (1.94 g) in dimethylacetamide (20 mL) was added lithium hydroxide (457 mg) at room temperature and the mixture was stirred overnight. The reaction mixture was poured to ice water (200 mL) and the mixture was extracted with ethyl acetate (x 2). The extract was concentrated in vacuo and the resultant residue was purified by a flash column chromatography on silica gel (Solvent; chloroform/methanol = 50 : 1) to give 1-[(2-oxo-1-propyl-1,2-dihydro-pyridin-3-yl)methyl]-4-(4-tert-butoxycarbonylpiperazin-1-yl)-1H-pyrazolo [3,4-d]pyrimidin e (2.92 g) as a solid.

(3) A solution of the compound obtained in the above step (2) (2.92 g) in chloroform (2 mL) and trifluoroacetic acid (2 mL) was stirred at room temperature for one day. The reaction mixture was concentrated in vacuo and to the residue was added an aqueous saturated sodium hydrogencarbonate solution. The mixture was extracted with chloroform (x 3) and the extract was concentrated in vacuo. The resultant crude product was purified by a flash column chromatography on silica gel (chloroform/methanol/aqueous ammonia = 19 : 1 : 0.2) to give 1-[(2-oxo-1-propyl-1,2-dihydropyridin-3-yl)-methyl]-4-(piperazin-1-yl)-1H-pyrazolo[3,4-d]pyrimidine (1.23 g) as a yellow solid.
   MS(APCI)m/z; 354 [M+H]⁺

### Reference Examples B4 to B14

The corresponding starting materials are treated in the same manner as described in the above-mentioned Reference Example B2 or Reference Example B3-(2) to give a compound as shown in the following Tables 46 to 47.

**Table 46**

| | | |
|---|---|---|
| 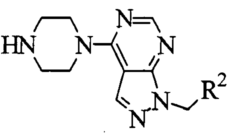 | | |

| Ref. Ex. Nos. | R² | Physicochemical properties etc. |
|---|---|---|
| B4*** | 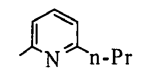 | amorphous solid MS(APCI)338[M+H]+ |
| B5*** | 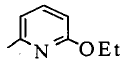 | amorphous solid MS(APCI)340[M+H]+ |
| B6*** | 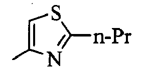 | amorphous solid MS(APCI)344[M+H]+ |
| B7*** | 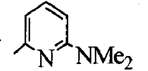 | amorphous solid MS(APCI)339[M+H]+ |

| | | |
|---|---|---|
| ***: trihydrochloride Me: methyl group, Et: ethyl group, n-Pr: n-propyl group | | |

**Table 47**

| | | |
|---|---|---|
| 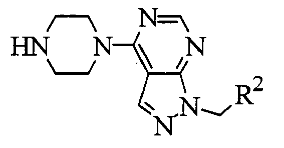 | | |

| Ref. Ex. Nos. | R² | Physicochemical properties etc. |
|---|---|---|
| B8** | 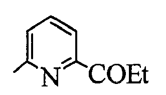 | amorphous solid MS(ESI)352[M+H]+ |
| B9** | 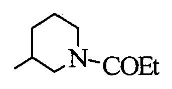 | amorphous solid MS(APCI)358[M+H]+ |
| B10 | 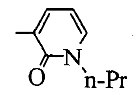 | amorphous solid MS(APCI)354[M+H]+ |
| B11 | 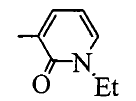 | amorphous solid MS(APCI)340[M+H]+ |
| B12 | 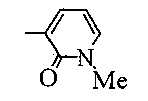 | amorphous solid MS(APCI)326[M+H]+ |
| B13** | 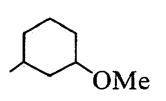 | amorphous solid MS(APCI)331 [M+H]+ |
| B14 | 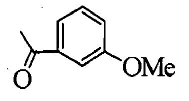 | amorphous solid MS(APCI)353 [M+H]+ |

| | | |
|---|---|---|
| ** : dihydrochloride Me: methyl group, Et: ethyl group, n-Pr: n-propyl group | | |

### Reference Examples B15

The corresponding starting materials are treated in the same manner as described in the above-mentioned Reference Example B1 to give a compound as shown in the following Table 48.

**Table 48**

| | | | |
|---|---|---|---|
| 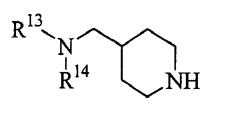 | | | |

| Ref. Ex. Nos. | R¹³ | R¹⁴ | Physicochemical properties etc. |
|---|---|---|---|
| B15 | t-Bu | Et | amorphous solid MS(APCI)199[M+H]+ |

| | | | |
|---|---|---|---|
| Et: ethyl group, t-Bu: tert-butyl group | | | |

### Reference Example B16

(1) To a solution of 2-hydroxynicotinic acid (5.5 g) in methanol (50 mL) and water (8.0 mL) was added 86 % potassium hydroxide solution (5.1 g) and the mixture was refluxed under heating for 20 minutes. After cooling to room temperature, to the reaction mixture was added n-propyl iodide (10 mL) and the mixture was refluxed under heating for 2 hours. After cooling to room temperature, the reaction mixture was concentrated in vacuo and the residue was dissolved in 2N hydrochloric acid. The solution was extracted with chloroform (x 2) and the combined extract was washed successively with 10 % sodium thiosulfate solution and brine, dried over sodium sulfate and concentrated in vacuo. The resultant crude product was purified by a flash column chromatography on silica gel (Solvent; chloroform/methanol = 97 : 3) and recrystallized from n-hexane/ethyl acetate to give 2-oxo-1-propyl-1,2-dihydropyridin-3-carboxylic acid (3.8 g, yield: 54 %) as crystals.
   MS(ESI)m/z; 180 [M-H]⁻

(2) To a suspension of the compound obtained in the above step (1) (3.22 g) in methylene chloride (15 mL) was added oxalyl chloride (3.1 mL) and a drop of dimethylformamide and the mixture was stirred at room temperature for 4.5 hours. The reaction mixture was concentrated in vacuo and the residue was distilled azeotropically with methylene chloride and dried in vacuo. The resultant product was dissolved in acetonitrile (15 mL) and tetrahydrofuran (15 mL) and thereto was added sodium borohydride (2.0 g). The mixture was stirred at room temperature overnight. To the reaction mixture was added an aqueous saturated sodium hydrogencarbonate solution and the mixture was stirred at room temperature for 3 days. The reaction mixture was extracted with chloroform (x 2) and the extract was dried over sodium sulfate and concentrated in vacuo. The resultant crude product was purified by a flash column chromatography on silica gel (Solvent; chloroform/methanol = 20 : 1) to give 3-hydroxymethyl-1-propyl-1H-pyridin-2-one (1.85 g, yield: 62 %) as a colorless oil.
   MS(APCI)m/z; 168 [M+H]⁺

### Reference Example B17

(1) To a suspension of 2-cyano-6-methylpyridine (1.5 g), tert-butyldimethylsilyl chloride (2.22 g) and copper(I) bromide (55 mg) in tetrahydrofuran (15 mL) was added dropwise 0.86M solution of ethylmagnesium bromide in tetrahydrofuran (23.6 mL) at -70 °C under argon gas atmosphere and the mixture was stirred for 10 minutes. The mixture was stirred at room temperature for 2.5 hours. Thereto was added water under ice-cooling and the mixture was extracted with ethyl acetate. The extract was dried over magnesium sulfate and concentrated in vacuo. The resultant crude product was purified by a flash column chromatography on silica gel (Solvent; n-hexane/ethyl acetate = 20 : 1) to give 1-(6-methylpyridin-2-yl)propan-1-one (1.24 g, yield: 66 %) as a red oil.
   MS(APCI)m/z; 150 [M+H]⁺

(2) To a solution of the compound obtained in the above step (1) (950 mg) in toluene (15 ml) was added ethylene glycol (2.0 mL) and p-toluenesulfonic acid monohydrate (125 mg) and the mixture was refluxed under heating for 1 day. During the reaction, ethylene glycol (2.0 mL) and p-toluenesulfonic acid monohydrate (125 mg) were further added (x 2) to the mixture. After cooling to room temperature, to the reaction mixture was basified with an aqueous saturated sodium hydrogencarbonate solution and the mixture was extracted with ethyl acetate. The extract was washed with brine, dried over sodium sulfate and concentrated in vacuo to give 2-(2-ethyl-[1,3]dioxolan-2-yl)-6-methylpyridine (1.23 g) as a yellow oil.
   MS(APCI)m/z; 174 [M+H]⁺

(3) A mixture of the compound obtained in the above step (2) (1.15 g), N-bromosuccinimide (2.1 g), 2,2'-azobis(isobutylonitrile) (AIBN, 5 mg) and carbon tetrachloride (1.0 mL) was stirred at 110 °C for 15 hours. After cooling to room temperature, to the reaction mixture was added water and the mixture was extracted with ethyl acetate. The extract was washed successively with an aqueous saturated sodium hydrogencarbonate solution and brine and concentrated in vacuo. The resultant crude product was purified by a flash column chromatography on silica gel (Solvent; n-hexane/ethyl acetate = 10 : 1) to give 2-bromomethyl-6-(2-ethyl-[1,3]dioxolan-2-yl)-methylpyridine (155 mg, yield: 10 %) as a yellow oil.
   MS(APCI)m/z; 272/274 [M+H]⁺

### Reference Example B18

To a solution of 3-piperidinemethanol (1.0 g) in 10 % sodium hydroxide solution (8 mL) was added dropwise a solution of propionyl chloride (760 µL) in ether (10 mL) and the mixture was vigorously stirred at room temperature for 3 hours. The reaction mixture was extracted with chloroform and the extract was dried over magnesium sulfate and concentrated in vacuo to give 1-(3-hdroxymethylpiperidin-1-yl)propan-1-one (1.3 g, yield: 87 %) as a yellow oil.
MS(APCI)m/z; 172 [M+H]⁺

### Reference Example B19

To a solution of 3-piperidine methanol (1.0 g) and propionaldehyde (1.2 mL) in chloroform (10 mL) was added successively sodium triacetoxyborohydride (3.54 g) and acetic acid (1.0 mL) under ice-cooling and the mixture was stirred at room temperature for 21 hours. The reaction mixture was diluted with chloroform and acidified (pH 10) with an aqueous saturated sodium hydrogencarbonate solution under stirring. The aqueous layer was saturated with potassium carbonate powder and extracted with chloroform (x 2). The extract was dried over sodium sulfate and concentrated to give (1-propyl-piperidin-3-yl)methanol (1.82 g) as a crude product.
MS(APCI)m/z; 158 [M+H]⁺

### Reference Example B20

To a solution of 3-methoxycyclohexanecarboxylic acid (2.5 g) in tetrahydrofuran (10 mL) was added dropwise 2.0M solution of borane/dimethylsulfide complex in tetrahydrofuran (10 mL) at -78 °C and the mixture was stirred for 5 hours. To the reaction mixture was added an aqueous saturated sodium hydrogencarbonate solution and the mixture was extracted with ethyl acetate. The extract was dried over magnesium sulfate and concentrated in vacuo to give (3-methoxycyclohexyl)methanol (2.64 g) as a crude product.
MS(APCI)m/z; 162 [M+H]⁺

### INDUSTRIAL APPLICABILITY

A compound [I] of the present invention or a pharmaceutically acceptable salt thereof shows a potent SK channel-blocking activity, and therefore, it can be useful as a medicine for treatment and/or prophylaxis of SK channel-related diseases (e.g., gastrointestinal motility disorders such as constipation, post-operative illeus or gastroesophageal reflux, central nervous system disorders such as learning/memory disorders, emotional disorders, Arzheimer's disease, depression or Perkinson's disease, myotonic muscular dystrophy or sleep apnea and the like).

## Claims

1. A compound of the following formula [I]: 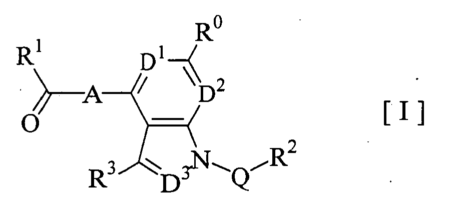 wherein
R⁰ is a hydrogen atom, a halogen atom, a cyano group, a lower alkoxy group, a lower alkyl group, an amino group (said amino group may be substituted by a lower alkyl group(s)) or a heteroaryl group,
R¹ is a group of the following formula: 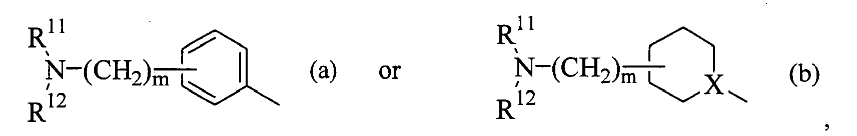
R¹¹ and R ¹² are the same or different and a hydrogen atom, a lower alkyl group, a hydroxy-lower alkyl group, a cyclo-lower alkyl group, a lower alkanoyl group, a lower alkenoyl group, a lower alkoxy-lower alkyl group, a cyclo-lower alkyl-carbonyl group, an amino-lower alkyl group (said amino moiety may be substituted by a lower alkyl group(s)), a lower alkoxy-carbonyl group, a nitrogen-containing heterocyclic group-substituted lower alkyl group or a nitrogen-containing heterocyclic group optionally substituted by a lower alkyl group, or both groups combine each other at their termini together with an adjacent nitrogen atom to form a nitrogen-containing heterocyclic group (said heterocyclic group may be substituted by a lower alkyl group), X is N or CH, m is an integer of 0 or 1,
A is a group of the formula: 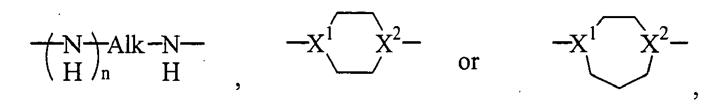 one of X¹ and X² is N or CH and another is N, Alk is a lower alkylene group, n is an integer of 0 or 1,
D¹, D² and D³ are N or CH,
R² is an aryl (or heteroaryl) group optionally substituted by one or two groups selected from a halogen atom, a lower alkyl group optionally substituted by a halogen atom, a lower alkoxy group optionally substituted by a halogen atom, a lower alkanoyl group and an amino group optionally substituted by one or two lower alkyl groups,
R³ is a hydrogen atom or a lower alkyl group, and
Q is a lower alkylene group,
or a pharmaceutically acceptable salt thereof.

2. A fused bicyclic compound of the following formula [IA]: 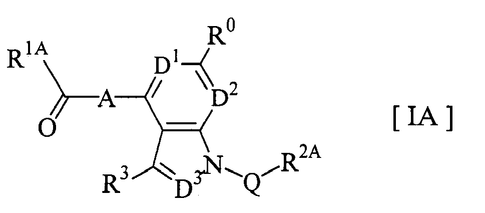 wherein
R⁰ is a hydrogen atom, a halogen atom, a cyano group, a lower alkoxy group, a lower alkyl group, an amino group (said amino group may be substituted by a lower alkyl group(s)) or a heteroaryl group,
R^{1A} is a group of the following formula: 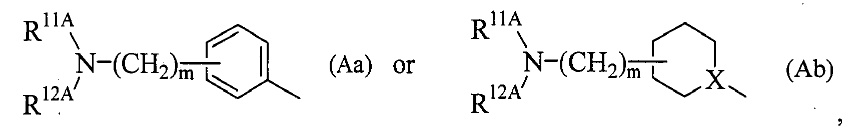
R^{11A} and R^{12A} are the same or different and a hydrogen atom, a lower alkyl group, a hydroxy-lower alkyl group, a cyclo-lower alkyl group, a lower alkanoyl group, a lower alkenoyl group, a lower alkoxy-lower alkyl group, a cyclo-lower alkyl-carbonyl group, an amino-lower alkyl group (said amino moiety may be substituted by a lower alkyl group(s)), a lower alkoxy-carbonyl group or a nitrogen-containing heterocyclic group-substituted lower alkyl group or both groups combine each other at their termini together with an adjacent nitrogen atom to form a nitrogen-containing heterocyclic group (said heterocyclic group may be substituted by a lower alkyl group), X is =N- or =CH-, m is an integer of 0 or 1,
A is a group of the formula: 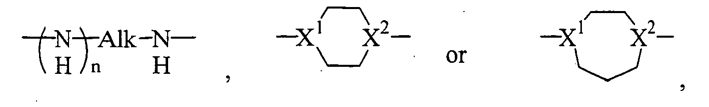 one of X¹ and X² is N or CH and another is N, Alk is a lower alkylene group, n is an integer of 0 or 1,
D¹, D² and D³ are N or CH,
R^{2A} is an aryl group optionally substituted by a lower alkoxy group or a heteroaryl group optionally substituted by a group(s) selected from a lower alkyl group and a lower alkoxy group,
R³ is a hydrogen atom or a lower alkyl group and
Q is a lower alkylene group;
provided that: (i) both R⁰ and R³ are not simultaneously a hydrogen atom when all of D¹, D² and D³ are N and A is a group of the formula: 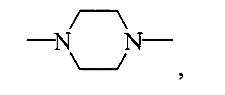
or a pharmaceutically acceptable salt thereof.

3. The compound according to Claim 2 in which R^{1A} is a group of the formula (Aa) and one of R^{11A} and R^{12A} is a hydrogen atom or an amino-lower alkyl group (said amino moiety may be substituted by a lower alkyl group(s)) and another is a lower alkyl group, a lower alkanoyl group, a cyclo-lower alkyl-carbonyl group or a lower alkenoyl group.

4. The compound according to Claim 2 in which R^{1A} is a group of the formula (Ab) and one of R^{11A} and R^{12A} is a hydrogen atom, a lower alkyl group, an amino-lower alkyl group (said amino moiety may be substituted by a lower alkyl group(s)) or a nitrogen-containing heterocyclic group-substituted lower alkyl group and another is a lower alkyl group, a cyclo-lower alkyl group, a hydroxy-lower alkyl group, a lower alkoxy-lower alkyl group, a lower alkanoyl group or a cyclo-lower alkyl-carbonyl group or both of them combine each other at their termini together with an adjacent nitrogen atom to form a nitrogen-containing heterocyclic group (said heterocyclic group may be substituted by a lower alkyl group(s)).

5. The compound according to Claim 4 in which the heterocyclic group in R^{11A} and R^{12A} is a saturated or unsaturated 5- to 8-membered nitrogen-containing hetero-monocyclic group (said heterocyclic group may further contain an oxygen atom(s) as a heteroatom(s) other than the nitrogen atom).

6. The compound according to Claim 3 or 4 in which R^{2A} is a lower alkoxy-phenyl group, a lower alkyl-pyridyl group, a lower alkoxy-pyridyl group or a lower alkyl-thiazolyl group.

7. The compound according to Claim 6 in which Q is a methylene group.

8. A compound of the formula [IA-a]: 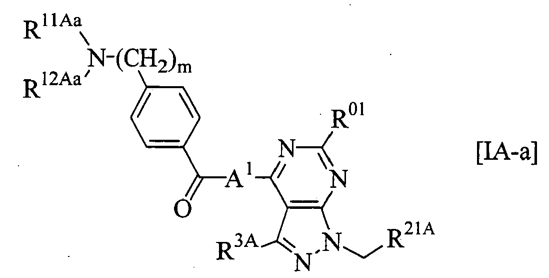 wherein R⁰¹ is a hydrogen atom, a halogen atom, a cyano group, a lower alkyl group, a lower alkoxy group, an amino group (said amino group may be substituted by a lower alkyl group(s)) or a thienyl group, one of R^{11Aa} and R^{12Aa} is a hydrogen atom or an amino-lower alkyl group (said amino moiety may be substituted by a lower alkyl group(s)) and another is a lower alkyl group, a lower alkanoyl group, a lower alkenoyl group or a cyclo-lower alkyl-carbonyl group, R^{21A} is a lower alkoxy-phenyl group, a lower alkyl-pyridyl group, a lower alkoxy-pyridyl group or a lower alkyl-thiazolyl group, R^{3A} is a hydrogen atom or a lower alkyl group, m is an integer of 0 or 1, A¹ is a group of the following formula: 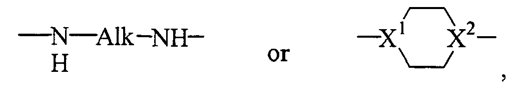 Alk is a lower alkylene group and one of X¹ and X² is N or CH and another is N, provided that both R⁰¹ and R^{3A} are not simultaneously a hydrogen atom when A¹ is a group of the following formula: 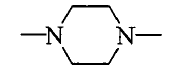 or a pharmaceutically acceptable salt thereof.

9. A compound of the following formula [IA-b]: 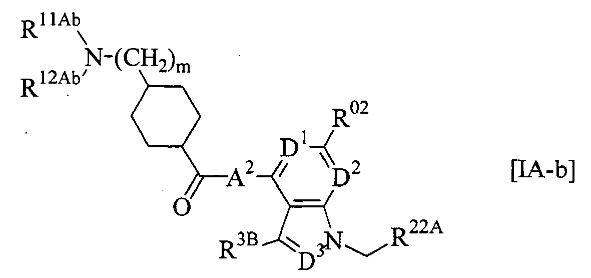 wherein R⁰² is a hydrogen atom, a halogen atom, a cyano group, a lower alkyl group, a lower alkoxy group, an amino group (said amino group may be substituted by a lower alkyl group(s)) or a thienyl group, one of R^{11Ab} and R^{12Ab} is a lower alkyl group and another is a lower alkoxy-lower alkyl group or both of them combine each other at their termini together with an adjacent nitrogen atom to form a saturated or unsaturated 5- to 8-membered nitrogen-containing hetero-monocyclic group (said hetero-monocyclic group may be substituted by a lower alkyl group(s) and may further contain an oxygen atom(s) as a heteroatom(s)), R^{22A} is a lower alkoxy-phenyl group, a lower alkoxy-pyridyl group or a lower alkyl-thiazolyl group, R^{3B} is a hydrogen atom or a lower alkyl group, m is an integer of 0 or 1, A² is a group of the following formula: 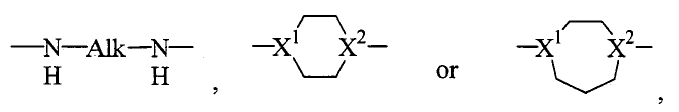 Alk is a lower alkylene group, one of X¹ and X² is N or CH and another is N, and , D¹, D² and D³ are N or CH, provided that both R⁰² and R^{3B} are not simultaneously a hydrogen atom when all of D¹, D² and D³ are N and A² is a group of the following formula: 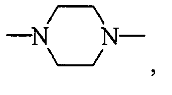 or a pharmaceutically acceptable salt thereof.

10. A compound of the following formula [IA-c] : 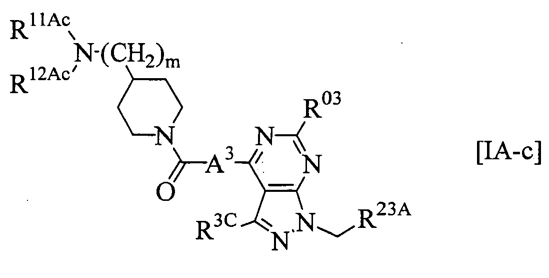 wherein R⁰³ is a hydrogen atom, a halogen atom, a cyano group, a lower alkyl group, a lower alkoxy group, an amino group (said amino group may be substituted by a lower alkyl group(s)) or a thienyl group, one of R^{11Ac} and R^{12Ac} is a hydrogen atom, a lower alkyl group, an amino-lower alkyl group (said amino moiety may be substituted by a lower alkyl group(s)) or a nitrogen-containing heterocyclic group-substituted lower alkyl group and another is a lower alkyl group, a cyclo-lower alkyl group, a hydroxy-lower alkyl group, a lower alkoxy-lower alkyl group, a lower alkanoyl group or a cyclo-lower alky-carbonyl group or both of them combine each other at their termini together with an adjacent nitrogen atom to form a saturated or unsaturated 5- to 8-membered nitrogen-containing hetero-monocyclic group (said hetero-monocyclic group may be substituted by a lower alkyl group(s) and may further contain an oxygen atom(s) as a heteroatom(s)), R^{23A} is a lower alkoxy-phenyl group, a lower alkyl-pyridyl group, a lower alkoxy-pyridyl group or a lower alkyl-thiazolyl group, R^{3C} is a hydrogen atom or a lower alkyl group, m is an integer of 0 or 1, A³ is a group of the following formula: 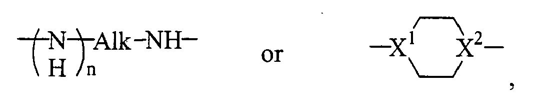 Alk is a lower alkylene group, one of X¹ and X² is N or CH and another is N and n is an integer of 0 or 1, provided that both R⁰³ and R^{3C} are not simultaneously a hydrogen atom when A³ is a group of the following formula: 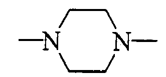 or a pharmaceutically acceptable salt thereof.

11. A compound which is:
7-(3-ethoxybenzyl)-4-[4-[[trans-4-[[N-(2-methoxyethyl)-N-(tert-butyl)amino]meth yl]cyclohexyl]carbonyl]piperazin-1-yl]-7H-pyrrolo[2,3-d]pyrimidine;
1-(3-ethoxybenzyl)-4-[4-[[trans-4-[[N-(2-methoxyethyl)-N-(tert-butyl)amino]meth yl]cyclohexyl]carbonyl]piperazin-1-yl]-1H-pyrazolo[3,4-b]pyridine;
6-chloro-4-[4-[(trans-4-piperidin-1-ylcyclohexyl)carbonyl]piperazin-1-yl]-1-[(2-pr opyl-1,3-thiazol-4-yl)methyl]-1H -pyrazolo[3,4-d]pyrimidine;
1-(3-ethoxybenzyl)-4-[1-[(trans-4-piperidin-1-ylcylohexyl)carbonyl]piperidin-4-y 1]-1H-pyrazolo[3,4-d]pyrimidine;
6-ethoxy-1-[(6-ethoxypyridin-2-yl)methyl]-4-[4-[(trans-4-piperidin-1-ylcyclohexyl )carbonyl]piperazin-1-yl]-1H-pyrazolo[3,4-d]pyrimidine;
1-(3-ethoxybenzyl)-4-[4-[[trans-4-[[N-(2-methoxyethyl)-N-(tert-butyl)amino]meth yl]cyclohexyl]carbonyl]piperazin-1-yl]-1H-pyrazolo[4,3-c]pyridine;
6-chloro-1-[(2-propyl-1,3-thiazol-4-yl)methyl]-4-[4-[[trans-4-[[N-(2-methoxyethyl )-N-(tert-butyl)amino]methyl]cyclohexyl]carbonyl]piperazin-1-yl]-1H-pyrazolo[3,4-d]pyri midine;
6-ethoxy-1-[(6-ethoxypyridin-2-yl)methyl]-4-[4-[[trans-4-[[N-(2-methoxyethyl)-N -(tert-butyl)amino]methyl]cyclohexyl]carbonyl]piperazin-1-yl]-1H-pyrazolo[3,4-d]pyrimidi ne;
1-(3-ethoxybenzyl)-4-[4-[[4-[[N-ethyl-N-(tert-butyl)amino]methyl]piperidin-1-yl]c arbonyl]piperidin-1-yl]-1H-pyrazolo[3,4-d]pyrimidine;
1-[(6-propylpyridin-2-yl)methyl]-4-[4-[[4-[[N-(2-methoxyethyl)-N-(tert-butyl)ami no]methyl]piperidin-1-yl]carbonyl]piperidin-1-yl]-1H-pyrazolo[3,4-d]pyrimidine;
1-[(6-propylpyridin-2-yl)methyl]-4-[4-[[4-[[N-ethyl-N-(tert-butyl)amino]methyl]pi peridin-1-yl]carbonyl]piperidin-1-yl]-1H-pyrazolo[3,4-d]pyrimidine;
1-[(6-ethoxypyridin-2-yl)methyl]-4-[4-[[4-[[N-(2-methoxyethyl)-N-(tert-butyl)ami no]methyl]piperidin-1-yl]carbonyl]piperidin-1-yl]-1H-pyrazolo[3,4-d]pyrimidine;
1-[(6-ethoxypyridin-2-yl)methyl]-4-[4-[[4-[[N,N-(diisopropyl)amino]methyl]piper idin-1-yl]carbonyl]piperidin-1-yl]-1H-pyrazolo[3,4-d]pyrimidine;
1-[(6-ethoxypyridin-2-yl)methyl]-4-[4-[[4-[[N-(2-hydroxyethyl)-N-(tert-butyl)ami no]methyl]piperidin-1-yl] carbonyl]piperidin-1-yl]-1H-pyrazolo[3,4-d]pyrimidine;
1-[(6-ethoxypyridin-2-yl)methyl]-4-[4-[[4-[[N-(2-hydroxyethyl)-N-isopropylamin o]methyl]piperidin-1-yl]carbonyl]piperidin-1-yl]-1H-pyrazolo[3,4-d]pyrimidine;
1-[(2-propyl-1,3-thiazol-4-yl)methyl]-4-[4-[[4-[[N-(2-methoxyethyl)-N-(tert-butyl)amino]methyl]piperidin-1-yl]carbonyl]piperidin-1-yl]-1H-pyrazolo[3,4-d]pyrimidine; or a pharmaceutically acceptable salt thereof.

12. A compound of the formula [IB]: 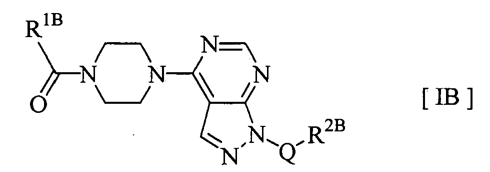 wherein
R^{1B} is a substituted cyclohexyl (or substituted piperidyl) group of the formula (Ba) or (Bb): 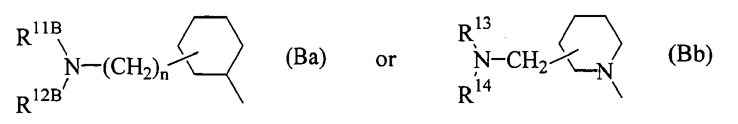
R^{11B} and R^{12B} are the same or different and a hydrogen atom, a lower alkyl group, a hydroxy-lower alkyl group, a lower alkoxy-lower alkyl group, a lower alkanoyl group, a lower alkoxy-carbonyl group, a lower alkenoyl group, an amino-lower alkyl group (amino moiety of said group may be substituted by a lower alkyl group) or a cyclo-lower alkyl-carbonyl group, or both of them combine each other at their termini together with an adjacent nitrogen atom to form a nitrogen-containing heterocyclic group, R¹³ and R¹⁴ are the same or different and a hydrogen atom, a lower alkyl group, a hydroxy-lower alkyl group, a lower alkoxy-lower alkyl group, a lower alkanoyl group, a lower alkenoyl group, a cyclo-lower alkyl-carbonyl group, an amino-lower alkyl group (amino moiety of said group may be substituted by a lower alkyl group), a nitrogen-containing heterocyclic group optionally substituted by a lower alkyl group or a lower alkyl group substituted by a nitrogen-containing heterocyclic group, n is an integer of 0 or 1,
Q is a lower alkylene group,
R^{2B} is a group of the following formula: 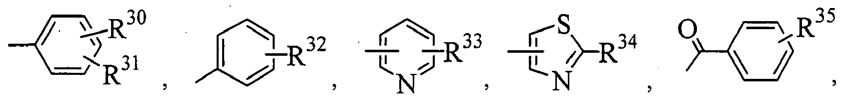 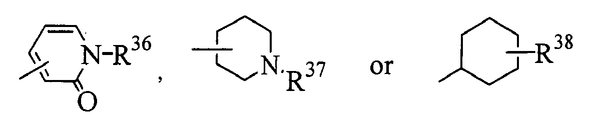 R³⁰ is a halogen atom, R³¹ is a lower alkyl group optionally substituted by a halogen atom, R³², R³³, R³⁴ and R³⁵ are a lower alkyl group, a lower alkoxy group, a halogeno-lower alkoxy group, a lower alkanoyl group or an amino group (said amino group being optionally substituted by a lower alkyl group), R³⁶, R³⁷ and R³⁸ are a lower alkyl group, a lower alkoxy group or a lower alkanoyl group;
provided that:
(i) R³², R³³ and R³⁴ are not a lower alkyl group or a lower alkoxy group, when A is a group of the formula (Ba), and
(ii) R^{2B} is not a group of the formula: 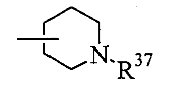
when n is an integer of 0;
or a pharmaceutically acceptable salt thereof.

13. The compound according to Claim 12 in which compound in which R^{1B} is a substituted cyclohexyl group of the formula (Ba), one of R^{11B} and R^{12B} is a C₁₋₆ alkyl group, a hydroxy-C₁₋₆ alkyl group, a C₁₋₆ alkoxy-C₁₋₆ alkyl group, a di(C₁₋₆)alkyl)amino-C₁₋₆ alkyl group or a nitrogen-containing heterocyclic group-substituted C₁₋₆ alkyl group and another is a C₁₋₆ alkyl group, a C₂₋₇ alkanoyl group or a C₃₋₈ cycloalkyl-carbonyl group, or both of them combine each other at their termini together with an adjacent nitrogen atom to form a nitrogen-containing heterocyclic group, Q is a C₁₋₆ alkylene group, R^{2B} is a group of the formula: 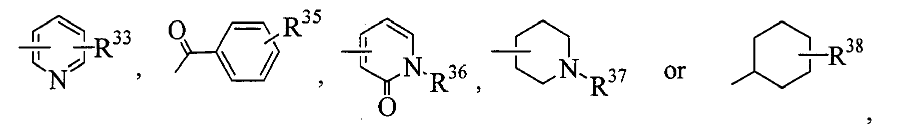 R³³ is a halogeno-C₁₋₆ alkoxy group, a C₂₋₇ alkanoyl group or a di(C₁₋₆ alkyl)amino group, R³⁵ is a C₁₋₆ alkoxy group, R³⁶ is a C₁₋₆ alkyl group, R³⁷ is a C₂₋₇ alkanoyl group and R³⁸ is a C₁₋₆ alkoxy group.

14. The compound according to Claim 12 in which R^{1B} is a group of the formula (Bb), one of R¹³ and R¹⁴ is a C₁₋₆ alkyl group, a hydroxy-C₁₋₆ alkyl group, a C₁₋₆ alkoxy-C₁₋₆ alkyl group, a di(C₁₋₆ alkyl)amino-C₁₋₆ alkyl group or a nitrogen-containing heterocyclic group-substituted C₁₋₆ alkyl group and another is a C₁₋₆ alkyl group, a C₁₋₆ alkyl-substituted nitrogen-containing heterocyclic group, a C₂₋₇ alkanoyl group or a C₃₋₈ cycloalkyl-carbonyl group, Q is a C₁₋₆ alkylene group, R^{2B} is a group of the formula: 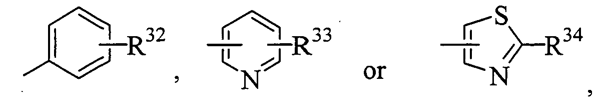 R³² is a C₁₋₆ alkoxy group, R³³ is a C₁₋₆ alkyl group or a C₁₋₆ alkoxy group and R³⁴ is a C₁₋₆ alkyl group.

15. The compound according to Claim 13 or 14 in which the nitrogen-containing heterocyclic group is a saturated or unsaturated nitrogen-containing 5- or 6-membered heterocyclic group.

16. The compound according to Claim 15 in which Q is methylene group.

17. A compound of the following formula [IB-a]: 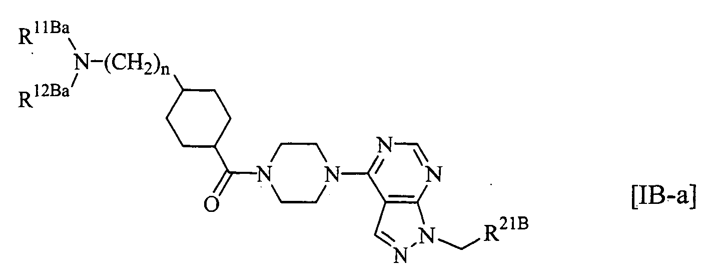 in which one of R^{11Ba} and R^{12Ba} is a lower alkyl and another is a hydroxy-lower alkyl group or a lower alkoxy-lower alkyl group, or both of them combine each other at their termini together with an adjacent nitrogen atom to form a 5- or 6-membered nitrogen-containing heterocyclic group, R^{21B} is a group of the following formula: 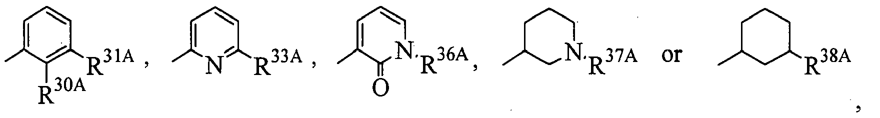 R^{30A} is a halogen atom, R^{31A} is a halogeno-lower alkyl group, R^{33A} is an amino group optionally substituted by a lower alkyl group or a lower alkanoyl group, R^{36A} is a lower alkyl group, R^{37A} is a lower alkanoyl group, R^{38A} is a lower alkoxy group and n is an integer of 0 or 1;
provided that R^{21B} is not a group of the formula: 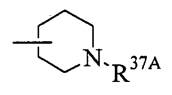 when n is an integer of 0;
or a pharmaceutically acceptable salt thereof.

18. A compound of the following formula [IB-b]: 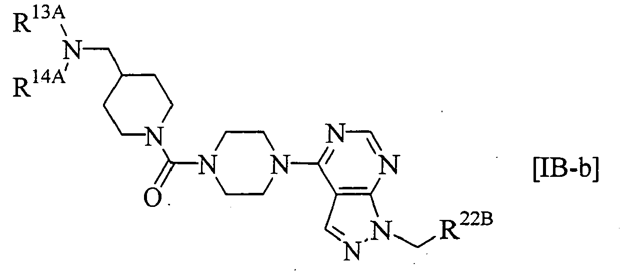 in which one of R^{13A} and R^{14A} is a lower alkyl group, an amino-lower alkyl group (the amino moiety of said group being optionally substituted by a lower alkyl group), a nitrogen-containing 5- or 6-membered heterocyclic group optionally substituted by a lower alkyl group or a nitrogen-containing 5- or 6-membered heterocyclic group-substituted lower alkyl group and another is a lower alkyl group, a hydroxy-lower alkyl group, a lower alkoxy-lower alkyl group, a lower alkanoyl group or a cyclo-lower alkyl-carbonyl group, R^{22B} is a group of the following formula: 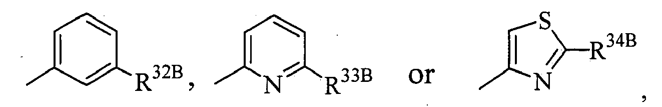 R^{32B} is a lower alkoxy group, R^{33B} is a lower alkyl group or a lower alkoxy group and R^{34B} is a lower alkyl group or a pharmaceutically acceptable salt thereof.

19. The compound according to Claim 17 in which one of R^{11Ba} and R^{12Ba} is a C₁₋₄ alkyl group and another is a hydroxy-C₁₋₄ alkyl group or a C₁₋₄ alkoxy-C₁₋₄alkyl group, or both of them combine each other at their termini together with an adjacent nitrogen atom to form a nitrogen-containing 6-membered heterocyclic group, R^{30A} is fluorine atom, R^{31A} is a trifluoro-C₁₋₄ alkyl group, R^{33A} is a di(C₁₋₄ alkyl)amino group or a C₂₋₅ alkanoyl group, R^{36A} is a C₁₋₄ alkyl group, R^{37A} is a C₂₋₅ alkanoyl group, and R^{38A} is a C₁₋₄ alkoxy group.

20. The compound according to Claim 18 in which one of R^{13A} and R^{14A} is a C₁₋₄ alkyl group or a C₁₋₄ alkyl-substituted nitrogen-containing 6-membered heterocyclic group and another is a C₁₋₄ alkyl group, a hydroxy-C₁₋₄ alkyl group or a C₁₋₄ alkoxy-C₁₋₄ alkyl group, R^{32B} is a C₁₋₄ alkoxy group, R^{33B} is a C₁₋₄ alkyl group or a C₁₋₄ alkoxy group, and R^{34B} is a C₁₋₄ alkyl group.

21. The compound according to Claim 18 in which one of R^{13A} and R^{14A} is a di(C₁₋₄ alkyl)amino-C₁₋₄ alkyl group or a nitrogen-containing 5-membered heterocyclic group-substituted C₁₋₄ alkyl group and another is a C₂₋₅ alkanoyl group or a C₃₋₆ cycloalkyl-carbonyl group, R^{32B} is a C₁₋₄ alkoxy group, R^{33B} is a C₁₋₄ alkyl group or a C₁₋₄ alkoxy group and R^{34B} is a C₁₋₄ alkyl group.

22. A compound which is:
4-[4-[(trans-4-piperidin-1-ylcyclohexyl)carbonyl]piperazin-1-yl]-1-[(6-propionylp yridin-2-yl)methyl]-1 H-pyrazolo [3,4-d]pyrimidine;
4-[4-[[trans-4-[[N-(2-methoxyethyl)-N-(tert-butyl)amino]methyl]cyclohexyl]carbo nyl]piperazin-1-yl]-1-(1-propionylpiperidin-3-yl)-1H-pyrazolo[3,4-d]pyrimidime;
1-[(3-methoxycyclohexyl)methyl]-4-[4-[[trans-4-[[N-(2-methoxyethyl)-N-(tert-but yl)amino]methyl]cyclohexyl]carbonyl]piperazin-1-yl]-1H-pyrazolo[3,4-d]pyrimidine;
1-(2-oxo-1-propyl-1,2-dihydropyridin-3-yl)-4-[4-[[trans-4-[[N-tert-butyl-(2-metho xyethyl)ammo]methyl]cyclohexyl]carbonyl]piperazin-1-yl]-1H-pyrazolo[3,4-d]pyrimidine;
1-(2-oxo-1-propyl-1,2-dihydropyridin-3-yl)-4-[4-[(trans-4-piperidin-1-ylcyclohexy 1)carbonyl]piperazin-1-yl]-1H-pyrazolo[3,4-d]pyrimidine;
1-[2-fluoro-3-(trifluoromethyl)benzyl]-4-[4-[(trans-4-piperidin-1-ylcyclohexyl)car bonyl]piperazin-1-yl]-1H-pyrazolo[3,4-d]pyrimidine;
1-(3-ethoxybenzyl)-4-[4-[[4-[[N-[2-(diisopropylamino)ethyl]-N-pivaloylamino]me thyl]piperidin-1-yl]carbonyl]piperazin-1-yl]-1H-pyrazolo[3,4-d]pyrimidine;
1-[[(2-propyl-1,3-thiazol-4-yl)methyl]-4-[4-[[4-[[N-[2-(diisopropylamino)ethyl]-N -pivaloylamino]methyl]piperidin-1-yl]carbonyl]piperazin-1-yl]-1H-pyrazolo[3,4-d]pyrimidi ne;
1-[(6-ethoxypyridin-2-yl)methyl]-4-[4-[[4-[[N-(cyclopropylcarbonyl)-N-[2-(dimet hylamino)ethyl]ammo]methyl]piperidin-1-yl]carbonyl]piperazin-1-yl]-1H-pyrazolo[3,4-d]p yrimidine;
1-[(6-ethoxypyridin-2-yl)methyl]-4-[4-[[4-[[N-(cyclopropylcarbonyl)-N-[2-(diisop ropylamino)ethyl]amino]methyl]piperidin-1-yl]carbonyl]piperazin-1-yl]-1H-pyrazolo[3,4-d ]pyrimidine;
1-[(6-ethoxypyridin-2-yl)methyl]-4-[4-[[4-[[N-[2-(diisopropylamino)ethyl-N-pival oylamino]methyl]piperidin-1-yl]carbonyl]piperazin-1-yl]-1H-pyrazolo[3,4-d]pyrimidine;
1-(3-ethoxybenzyl)-4-[4-[[4-[[N-(2-methoxyethyl)-N-(tert-butyl)amino]methyl]pip eridin-1-yl]carbonyl]piperazin-1-yl]-1H-pyrazolo[3,4-d]pyrimidine;
1-(3-ethoxybenzyl)-4-[4-[[4-[[N,N-diisopropylamino)methyl]piperidin-1-yl]carbo nyl]piperazin-1-yl]-1H-pyrazolo[3,4-d]pyrimidine;
1-(3-ethoxybenzyl)-4-[4-[[4-[[N-ethyl-N-(tert-butyl)amino]methyl]piperidin-1-yl]c arbonyl]piperazin-1-yl]-1H-pyrazolo[3,4-d]pyrimidine;
1-[(2-propyl-1,3-thiazol-4-yl)methyl]-4-[4-[[4-[[N-ethyl-N-(tert-butyl)amino]meth yl]piperidin-1-yl]carbonyl]piperazin-1-yl]-1H-pyrazolo[3,4-d]pyrimidine;
1-[(2-propyl-1,3-thiazol-4-yl)methyl]-4-[4-[[4-[(N,N-diisopropylamino)methyl]pip eridin-1-yl]carbonyl]piperazin-1-yl]-1H-pyrazolo[3,4-d]pyrimidine;
1-[(6-ethoxypyridin-2-yl)methyl]-4-[4-[[4-[[N-(2-methoxyethyl)-N-(tert-butyl)ami no]methyl]piperidin-1-yl]carbonyl]piperazin-1-yl]-1H-pyrazolo[3,4-d]pyrimidine;
1-[(6-ethoxypyridin-2-yl)methyl]-4-[4-[[4-[(N,N-diisopropylamino)methyl]piperid in-1-yl]carbonyl]piperazin-1-yl]-1H-pyrazolo[3,4-d]pyrimidine;
1-[(6-ethoxypyridin-2-yl)methyl]-4-[4-[[4-[[N-ethyl-N-(tert-butyl)amino]methyl]p iperidin-1-yl]carbonyl]piperazin-1-yl]-1H-pyrazolo[3,4-d]pyrimidine;
1-[(6-ethoxypyridin-2-yl)methyl]-4-[4-[[4-[[N-(2-hydroxyethyl)-N-(tert-butyl)ami no]methyl]piperidin-1-yl]carbonyl]piperazin-1-yl]-1 H-pyrazolo[3,4-d]pyrimidine;
1-[(6-ethoxypyridin=2-yl)methyl]-4-[4-[[4-[[N-(2-hydroxyethyl)-N-isopropylamin o]methyl]piperidin-1-yl]carbonyl]piperazin-1-yl]-1H-pyrazolo[3,4-d]pyrimidine;
1-[(6-propylpyridin-2-yl)methyl]-4-[4-[[4-[[N-(2-methoxyethyl)=N-(tert-butyl)ami no] methyl] piperidin-1-yl] carbonyl]piperazin-1-yl]-1 H-pyrazolo [3,4-d]pyrimidine;
1-[(6-propylpyridin-2-yl)methyl]-4-[4-[[4-[(N,N-diisopropylamino)methyl]piperid in-1-yl]carbonyl]piperazin-1-yl]-1H-pyrazolo[3,4-d]pyrimidine;
1-[(6-propylpyridin-2-yl)methyl]-4-[4-[[4-[[N-ethyl-N-(tert-butyl)amino]methyl]pi peridin-1-yl]carbonyl]piperazin-1-yl]-1H-pyrazolo[3,4-d]pyrimidine;
1-[(6-propylpyridin-2-yl)methyl]-4-[4-[[4-[[N-(2-hydroxyethyl)-N-(isopropylamin o)methyl]piperidin-1-yl]carbonyl]piperazin-1-yl]-1H-pyrazolo[3,4-d]pyrimidine; or a pharmaceutically acceptable salt thereof.

23. A pharmaceutical composition which comprises as an active ingredient a fused bicyclic compound of the following formula [I]: 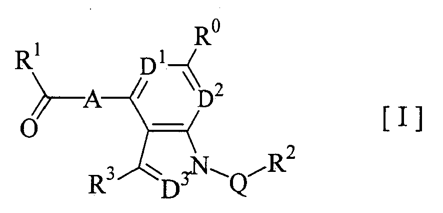 wherein
R⁰ is a hydrogen atom, a halogen atom, a cyano group, a lower alkoxy group, a lower alkyl group, an amino group (said amino group may be substituted by a lower alkyl group(s)) or a heteroaryl group,
R¹ is a group of the following formula: 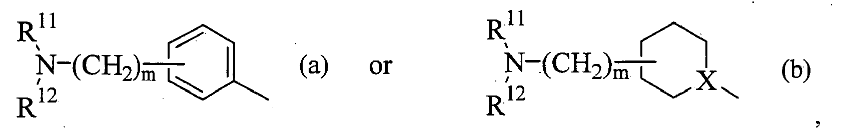 R¹¹ and R¹² are the same or different and a hydrogen atom, a lower alkyl group, a hydroxy-lower alkyl group, a cyclo-lower alkyl group, a lower alkanoyl group, a lower alkenoyl group, a lower alkoxy-lower alkyl group, a cyclo-lower alkyl-carbonyl group, an amino-lower alkyl group (said amino moiety may be substituted by a lower alkyl group(s)), a lower alkoxy-carbonyl group, a nitrogen-containing heterocyclic group-substituted lower alkyl group or a nitrogen-containing heterocyclic group optionally substituted by a lower alkyl group, or both groups combine each other at their termini together with an adjacent nitrogen atom to form a nitrogen-containing heterocyclic group (said heterocyclic group may be substituted by a lower alkyl group), X is N or CH, m is an integer of 0 or 1,
A is a group of the formula: 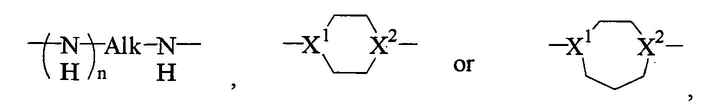 one of X¹ and X² is N or CH and another is N, Alk is a lower alkylene group, n is an integer of 0 or 1,
D¹, D² and D³ are the same or different and each is N or CH,
R² is an aryl (or heteroaryl) group optionally substituted by one or two groups selected from a halogen atom, a lower alkyl group optionally substituted by a halogen atom, a lower alkoxy group optionally substituted by a halogen atom, a lower alkanoyl group and an amino group optionally substituted by one or two lower alkyl groups,
R³ is a hydrogen atom or a lower alkyl group, and
Q is a lower alkylene group,
or a pharmaceutically acceptable salt thereof.

24. The pharmaceutical composition according to Claim 23 in which the active ingredient is a compound claimed in one of Claims 2 to 22.

25. The pharmaceutical composition according to Claim 23 which is a medicine for treatment or prophylaxis of a SK channel-related disease.
